(19) Europäisches Patentamt
European Patent Office
Office européen des brevets

(11) EP 2 906 525 B1

(12) # FASCICULE DE BREVET EUROPEEN

(45) Date de publication et mention de la délivrance du brevet:
**25.03.2020 Bulletin 2020/13**

(21) Numéro de dépôt: **13766593.1**

(22) Date de dépôt: **28.08.2013**

(51) Int Cl.:
***C07C 41/30*** (2006.01) ***C07C 43/23*** (2006.01)
***C08G 8/12*** (2006.01)

(86) Numéro de dépôt international:
**PCT/FR2013/051988**

(87) Numéro de publication internationale:
**WO 2014/033406 (06.03.2014 Gazette 2014/10)**

(54) **PROCEDE DE PREPARATION A HAUTS RENDEMENTS DE P-(R-OXY)CALIX[9-20]ARENES**

VERFAHREN ZUR ERGIEBIGE PRODUKTION VON P-(R-OXY) CALIX [ 9 -20 ] ARENEN

METHOD FOR THE HIGH-YIELD PRODUCTION OF P-(R-OXY)CALIX[9-20] ARENES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.08.2012 FR 1258052**

(43) Date de publication de la demande:
**19.08.2015 Bulletin 2015/34**

(73) Titulaire: **Université Paris Sud-Paris XI**
**97405 Orsay Cedex (FR)**

(72) Inventeurs:
- **HUC, Vincent, Germain**
  **91400 Orsay (FR)**
- **MARTINI, Cyril**
  **91140 Villebon-sur-Yvette (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**CA-A1- 2 251 070 JP-A- 10 260 556**

- **ALESSANDRO CASNATI ET AL: "p -(Benzyloxy)calix[8]arene: One-Pot Synthesis and Functionalization", THE JOURNAL OF ORGANIC CHEMISTRY, vol. 62, no. 18, 1 septembre 1997 (1997-09-01), pages 6236-6239, XP055034562, ISSN: 0022-3263, DOI: 10.1021/jo970620r**
- **VINCENT HUC ET AL: "C 3 v (Trimethyl) p -(Benzyloxy)calix[6]arene: A Versatile Platform for the Synthesis of Functionalized C 3 v Calix[6]arenes", EUROPEAN JOURNAL OF ORGANIC CHEMISTRY, vol. 2010, no. 11, 1 avril 2010 (2010-04-01), pages 2199-2205, XP055057188, ISSN: 1434-193X, DOI: 10.1002/ejoc.200901281**
- **C DAVID GUTSCHE ET AL: "Calixarenes: paradoxes and paradigms in molecular baskets", PURE & APPLIED CHEMISTRY, vol. 62, no. 3, 1 janvier 1990 (1990-01-01), pages 485-491, XP055057932,**
- **OSTASZEWSKI R ET AL: "Influence of Base and Solvent on the Reaction between p-Cresol and Formaldehyde Leading to p-Methalcalix(n)arenes", POLISH JOURNAL OF CHEMISTRY, POLSKIE TOWARZYSTWO CHEMICZNE, PL, vol. 71, no. 8, 1 janvier 1997 (1997-01-01), pages 1053-1059, XP009168220, ISSN: 0137-5083**
- **KERRY J GOODWORTH ET AL: "Synthesis and in vivo biological activity of large-ringed calixarenes against", TETRAHEDRON, ELSEVIER SCIENCE PUBLISHERS, AMSTERDAM, NL, vol. 67, no. 2, 8 novembre 2010 (2010-11-08), pages 373-382, XP028165003, ISSN: 0040-4020, DOI: 10.1016/J.TET.2010.11.034 [extrait le 2010-11-13]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- GUTSCHE C D ET AL: "CALIXARENES. 4. THE SYNTHESIS, CHARACTERIZATION, AND PROPERTIES OF THE CALIXARENES FROM P-TERT-BUTYLPHENOL", JOURNAL OF THE AMERICAN CHEMICAL SOCIETY, ACS PUBLICATIONS, US, vol. 103, no. 13, 1 juillet 1981 (1981-07-01), pages 3782-3792, XP000676352, ISSN: 0002-7863, DOI: 10.1021/JA00403A028
- NAKAYAMA T ET AL: "NEW POSITIVE-TYPE PHOTORESIST BASED ON MONO-SUBSTITUTED HYDROQUINONE CALIX (8) ARENE AND DIAZONAPHTHOQUINONE", JOURNAL OF MATERIALS CHEMISTRY, THE ROYAL SOCIETY OF CHEMISTRY, CAMBRIDGE, GB, vol. 9, no. 3, 1 mars 1999 (1999-03-01), pages 697-702, XP000853243, ISSN: 0959-9428, DOI: 10.1039/A807718E
- DATABASE REGISTRY CHEMICAL ABSTRACTS SERVICE, COLUMBUS, OHIO, US; 26 avril 2004 (2004-04-26), XP002694618, extrait de STN Database accession no. 676613-35-7 -& DATABASE CHEMCATS CHEMICAL ABSTRACTS SERVICE, COLUMBUS, OHIO, US; 21 août 2012 (2012-08-21), XP002694619, extrait de STN Database accession no. 0033740586
- Roger Lamartine ET AL: "Solid State Polycondensation of Precursors of Phenolic Resins", Molecular Crystals and Liquid Crystals, vol. 134, no. 1, 1 April 1986 (1986-04-01) , pages 219-236, XP55058975, ISSN: 0026-8941, DOI: 10.1080/00268948608079586
- JACK M. HARROWFIELD ET AL: "Deprotonation of Large Calixarenes - Cation Binding and Conformations", AUSTRALIAN JOURNAL OF CHEMISTRY: AN INTERNATIONAL JOURNAL FOR CHEMICAL SCIENCE, vol. 69, no. 5, 1 January 2016 (2016-01-01), page 546, XP055560605, AU ISSN: 0004-9425, DOI: 10.1071/CH15761
- Bernd Garska ET AL: "Molecular Reinforcement of Transparent Materials With Acrylamide-Modified Calix[4]arene as a Crosslinker", Macromolecular Materials and Engineering, vol. 297, no. 8, 10 February 2012 (2012-02-10), pages 785-789, XP055086943, ISSN: 1438-7492, DOI: 10.1002/mame.201100354


Remarques:

Le dossier contient des informations techniques présentées postérieurement au dépôt de la demande et ne figurant pas dans le présent fascicule.

## Description

La présente invention concerne un procédé de préparation à hauts rendements de *p*-(R-oxy)calix[9-20]arènes.

Les calixarènes ont été particulièrement étudiés ces dix dernières années en raison des possibilités immenses offertes par ces macrocycles aisément accessibles. Ces macrocycles possèdent une forme de calice dont la cavité est généralement inférieure ou égale à environ 1 nm.

Quelques unes des propriétés les plus étudiées incluent les phénomènes de reconnaissance des ions inorganiques et des molécules organiques, les assemblages supramoléculaires ou bien encore la synthèse des nanoparticules, entre autres.

Dans la plupart des cas, ces études ont été effectuées avec les calixarènes fonctionnalisés par un *p*-(*t*-butyl) ou par des calixarènes obtenus par modification chimique de ces *p*-(*t*-butyl)calixarènes, étant donné que la synthèse des *p*-(*t*-butyl)calixarènes est de loin la plus documentée depuis le travail pionnier de Gutsche et *al.*
D. Gutsche, Calixarenes: An Introduction, The Royal Society of Chemistry, Cambridge, 2008**.** Z. Asfari, V. Böhmer, J. Harrowfield, J. Vicens, Calixarenes 2001, Kluwer, Dordrecht, the Netherlands, 2001**.**

Par conséquent, l'utilisation d'autres monomères de type phénols fonctionnalisés pour la synthèse des calixarènes est en grande partie inexplorée, même si des synthèses en une étape d'autres *p*-(alkyl)calixarènes sont connues (T. Patrick, P. Egan, J. Org. Chem. 1977, 42, 382 ; T. Patrick, P. Egan, J. Org. Chem. 1978, 43, 4280 ; Z. Asfari, J. Vicens, Tetrahedron Lett. 1988, 29, 2659 ; F. Vocanson, M. Perrin, R. Lamartine, J. Inclusion Phenom. Macrocyclic Chem. 2001, 39, 127 ; Jerry L. Atwood et al ; Org. Lett. 1999, 1, 1523).

Les calixarènes *p*-substitués sont habituellement obtenus en mélange de *p*-calix[4, 5, 6, 7, 8]arènes par réaction d'un phénol *p*-substitué avec du paraformaldéhyde en présence d'au moins une base telle que la potasse ou la soude (B. Dahwan et al., Macromolec. Chem. 1987, 188, 921 ; C. D. Gutsche et al., Org. Synth. 1990, 68, 234 ; C. D. Gutsche et al., Org. Synth. 1990, 68, 238).

En général, les *p*-(alkyl)calix[8]arènes sont les calixarènes obtenus le plus facilement, car ils correspondent au produit cinétique de la réaction de polycondensation. Les *p*-(alkyl)calix[4]arènes correspondent, eux, au produit thermodynamique.

Dans la plupart des cas, le retrait ou la substitution de la position alkyle en para de la fonction hydroxyle de ces calixarènes, lorsqu'elle est possible, est au mieux délicate. Dans le cas le plus courant des *p*-(*t*-butyl)calixarènes, il s'agit en général d'un procédé en plusieurs étapes. Dans un premier temps, un réactif de type acide de Lewis est généralement associé à un phénol pour retirer le groupement *t*-butyle, puis dans un second temps, une autre fonction peut être introduite en lieu et place du groupement *t*-butyle en ayant au préalable halogéné la position. La réaction n'étant pas quantitative, la « déterbutylation » devient problématique, notamment lorsque le nombre d'unités du calixarène augmente. La présence de sous-produits limite le rendement, impose une étape de purification et restreint la pureté du produit. Ceci peut limiter le rendement final en produit entièrement déprotégé ainsi que son utilisation.

Les *p*-(benzyloxy)calixarènes représentent une alternative très intéressante au *p*-(alkyl)calixarènes. Les motifs (benzyloxy)phénols sont en effet quantitativement réduits en phénols par hydrogénolyse catalysée par le palladium sur charbon, autorisant une post-fonctionnalisation aisée.

Le *p*-(benzyloxy)calix[6]arène est un calixarène déjà décrit dans la littérature sous forme neutralisée mais obtenu soit en tant que produit très minoritaire de la synthèse du *p*-(benzyloxy)calix[8]arène, après plusieurs étapes de purification avec un rendement de 1 à 2% (A. Casnati et al., J. Am. Chem. Soc 2001, 123, 12182), soit sous forme relativement impure avec une pureté de l'ordre de 80% (V. Huc et al., Eur. J. Org. Chem 2010, 6186).

Le *p*-(benzyloxy)calix[7]arène comporte dans sa structure 7 motifs de répétition. Cette taille de cycle est habituellement considérée comme difficile à obtenir dans le domaine de la chimie des calixarènes. En effet, les études antérieures montrent que les calixarènes comportant un nombre impair de motifs sont difficilement accessibles. Dans le cas précis du *p*-(benzyloxy)calix[7]arène, ce produit est déjà décrit comme un sous produit très minoritaire de la synthèse d'autres calixarènes avec des rendements inférieurs à 10%, utilisant des procédures de purification complexes incluant des purifications par chromatographie sur colonne (V. Huc et al., Eur. J. Org. Chem. 2010, 6186). D'autres calix[7]arènes sont également connus, notamment en série *p*-(*t*-butyle), mais là encore les rendements sont faibles (Pour les *p*-(*t*-butyles) : A. Ninegawa et al. 1982, Macromol. Chem. Rapid. Chem., 3, 65 ; Y. Nakamoto et al. Macromol. Chem. Rapid. Chem. 1982, 3, 705 ; F. Vocanson et al., New. J. Chem. 1995, 19, 825. Pour *les p*-(benzyles) : J. L. Atwood et al. 1999, Org. Lett., 1, 1523).

Le *p*-(benzyloxy)calix[7]arène sous forme monosalifiée n'est pas décrit dans la littérature et son obtention ouvrirait la voie à de nouveaux calix[7]arènes mono-fonctionnalisés, le sel de phénol étant bien plus nucléophile que les phénols neutres adjacents.

Le *p*-(benzyloxy)calix[8]arène est obtenu dans l'art antérieur avec un rendement de 48% par réaction du *p*-(benzyloxy)phénol en présence de formaldéhyde avec une base choisie parmi la soude, la potasse ou l'hydroxyde de lithium, la base étant à une concentration de 0,02 équivalent par rapport au *p*-(benzyloxy)phénol (A Casnati et al, J. Org. Chem. 1997, 62, 6236).

Peu de documents décrivent la synthèse de grands calixarènes, c'est-à-dire comportant de 9 à 20 motifs de répétition.

Ainsi le brevet CA 2,251,070 décrit l'obtention avec de faibles rendements des calixarènes comportant 9 et 11-14 motifs de répétition avec un procédé en deux étapes par réaction d'un *p*-(alkyl) ou *p*-(aralkyl)phénol en milieu aqueux en présence d'une base en quantité inférieure à 0,5 eq puis chauffage dans un solvant organique sans eau.

Ce brevet ne décrit pas l'obtention de *p*-(R-oxy)calix[9-20]arènes.

L'article de Gutsche et al. (J. Am. Chem. Soc. 1999, 121, 4136) décrit l'obtention de *p*-(*t*-butyl)calix[9-20]arènes en milieu acide mais pas l'obtention de *p*-(R-oxy)calixarènes.

Par conséquent, l'obtention de grands calixarènes, qui sont de plus aisément fonctionnalisables sur la couronne haute par une large variété de groupements chimiques dans des conditions douces, est toujours une question ouverte et notamment l'obtention de grands calixarènes tels que les *p*-(R-oxy)calix[9-20]arènes avec de bons rendements.

L'un des objets de l'invention est la fabrication de *p*-(R-oxy)calix[9-20]arènes (aussi appelés grands calixarènes) avec des rendements cumulés supérieurs à 5%, notamment supérieurs à 10%, 20%, 30%, 40% ou 50%, en particulier supérieurs à 50%.

Un autre objet de l'invention est de fournir un procédé de synthèse en une étape permettant :
l'obtention d'un mélange de douze *p*-(R-oxy)calix[9-20]arènes ou de quelques *p*-(R-oxy)calix[9-20]arènes, isolés et purs, ou un mélange de deux, trois, quatre, cinq, six, sept, huit, neuf, dix ou onze parmi les douze, ainsi qu'une procédure de purification en une étape par simple cristallisation du mélange de calixarènes, permettant de les obtenir purs, en particuliers exempts des *p*-(benzyloxy)cali[5-8]arènes.

La présente invention concerne l'utilisation d'au moins une base, choisie parmi l'hydroxyde de rubidium ou l'hydroxyde de césium, avec au moins un phénol substitué en position 4 de formule (I) suivante :

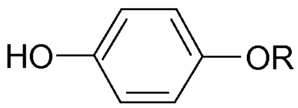

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)ORaOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire,
- un PEG-1 à 10 ramifié ou linéaire, dont le groupe terminal -OH est alkylé par un alkyle $C_1$-$C_{20}$ ramifié ou linéaire,

une source de formaldéhyde et un solvant organique,
le mélange de ladite base, dudit phénol, de ladite source de formaldéhyde et dudit solvant organique constituant un milieu réactionnel,
ledit milieu réactionnel étant chauffé à une température comprise de 50 °C à 250 °C durant un temps compris de 4 à 24h et ladite base étant à une concentration totale comprise de plus de 0,5 équivalent à 1,5 équivalent, avantageusement de plus de 0,5 équivalent à 1,2 équivalent, en particulier égale à 0,6 équivalent, par rapport au susdit phénol substitué en position 4 de formule (I),
pour la mise en œuvre d'une réaction de préparation d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou d'un mélange d'au moins deux desdits *p*-(R-oxy)calixarènes dans lequel lesdits au moins deux *p*-(R-oxy)calixarènes sont présents dans ledit mélange à raison d'au moins 5% molaire chacun.

Le chauffage dépend du solvant organique utilisé et est compris de 50°C à 250°C, en particulier au reflux, c'est-à-dire au point d'ébullition du solvant organique utilisé.

Le terme alkyle en $C_1$-$C_{20}$ utilisé dans toute la description désigne un groupe alkyle linéaire ou ramifié comprenant 1 à 20 atomes de carbone.

Par groupe alkyle linéaire en $C_1$ à $C_{20}$, il faut entendre : un groupe méthyle, un éthyle, un propyle, un butyle, un pentyle, un hexyle, un heptyle, un octyle, un nonyle, un décyle, un undécyle, un dodécyle, le tridécyle, tétradécyle, le pentadécyle, l'hexadécyle, l'heptadécyle, l'octadécyle, le nonadécyle et l'eicosyle, ainsi que tous leurs isomères.

Par groupe alkyle ramifié, il faut comprendre un groupe alkyle tel que défini ci-dessus comprenant des substituants choisis parmi la liste des groupes alkyles linéaires définie ci-dessus, lesdits groupes alkyles linéaires étant également susceptibles d'être ramifiés.

Par groupe cycloalkyle en $C_3$ à $C_{20}$ il faut comprendre un groupe cyclopropyle, cyclobutyle, cyclopentyle, cyclohexyle, cycloheptyle, cyclooctyle, cyclononyle, cyclodécyle, cycloundécyle, cyclododécyle, cyclotridécyle, cyclotétradécyle, cyclopentadécyle, cyclohexadécyle, cycloheptadécyle, cyclooctadécyle, cyclononadécyle et cycloeicosyle.

De tels groupes cycloalkyles peuvent être eux-mêmes substitués par un ou plusieurs groupe(s) alkyle linéaire ou ramifié tel que défini ci-dessus.

L'expression « source de formaldéhyde » signifie que du paraformaldéhyde, du trioxane ou du formaldéhyde en solution aqueuse tel que de la formaline ou du formol peuvent être utilisés.

Le terme solvant organique désigne tout solvant polaire, ou de préférence non polaire, dont le point d'ébullition est supérieur à environ 50°C, en particulier un solvant dont le point d'ébullition est compris de 50 à 250°C, en particulier le xylène, le toluène, l'octane, le diphenyléther, le mésitylène, le chlorobenzène, le dichlorobenzène, le trichlorobenzène.

L'expression « milieu réactionnel » signifie le mélange des différents composants, comprenant la ou les base(s), le ou les phénol(s) substitué(s) en position 4 de formule (I), la source de formaldéhyde et le solvant organique.

Lorsque ledit mélange vient d'être constitué, il est dénommé milieu réactionnel initial, c'est-à-dire un milieu réactionnel dans lequel la réaction de préparation des *p*-(R-oxy)calixarènes n'a pas encore été effectuée, en particulier avant tout chauffage dudit mélange des différents composés.

Lorsque la réaction de préparation des *p*-(R-oxy)calix[9-20]arènes est effectuée, en particulier après chauffage dudit mélange des différents composés, le milieu réactionnel est dénommé milieu réactionnel final.

Les *p*-(R-oxy)calix[9-20]arènes possèdent la structure suivante :

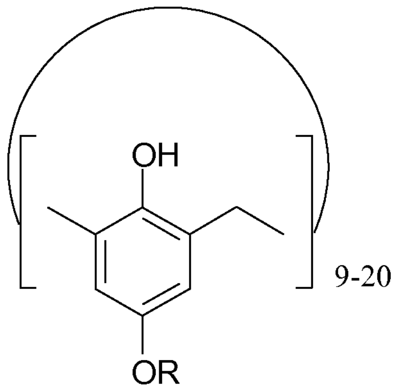

et sont donc constitués :

- d'un mélange de deux, trois, quatre, cinq, six, sept, huit, neuf, dix, onze ou des douze p-(R-oxy)calixarènes choisis parmi le groupe de *p*-(R-oxy)[9-20]calixarènes, quelle que soit la proportion de chaque *p*-(R-oxy)calixarène présent dans le mélange et peuvent également être dénommés dans toute la description « grands *p*-(R-oxy)calixarènes »,
- ou de l'un des douze *p*-(R-oxy)calixarènes, purifié ou pur, choisi parmi le groupe de *p*-(R-oxy)calixarènes de 9 à 20.

En particulier, les *p*-(R-oxy)calix[9-20]arènes possèdent la structure suivante :

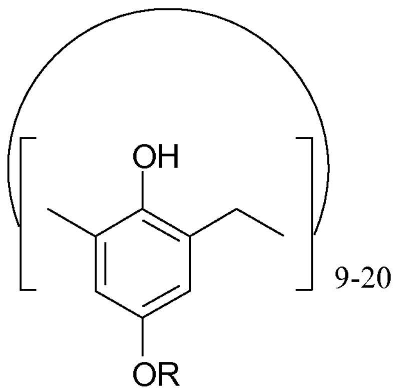

et sont donc constitués :

- d'un mélange de deux, trois, quatre, cinq, six, sept, huit, neuf, dix, onze ou des douze p-(R-oxy)calixarènes choisis parmi le groupe de *p*-(R-oxy)[9-20]calixarènes, quelle que soit la proportion de chaque *p*-(R-oxy)calixarène présent dans le mélange, lesdits *p*-(R-oxy)calixarènes étant présents dans ledit mélange à raison d'au moins 5% molaire chacun, et peuvent également être dénommés dans toute la description « grands *p*-(R-oxy)calixarènes »,
- ou de l'un des douze *p*-(R-oxy)calixarènes, purifié ou pur, choisi parmi le groupe de *p*-(R-oxy)calixarènes de 9 à 20.

Dans toute la description, le terme « purifié » ou « pur » désigne un composé ayant une pureté supérieure à 90%.

Le mélange de *p*-(R-oxy)calix[9-20]arènes ou les *p*-(R-oxy)calixarènes purifiés sont en règle générale obtenus sous forme neutralisée après neutralisation de la base présente dans le milieu réactionnel final.

Par l'expression « sous forme neutralisée », il faut comprendre un *p*-(R-oxy)calixarène purifié dont tous les hydroxyles des groupements phénols ont été neutralisés, c'est-à-dire sont sous forme OH, non salifiée.

Sans neutralisation de la base, le mélange de *p*-(R-oxy)calix[9-20]arènes ou les *p*-(R-oxy)calixarènes purifiés sont obtenus sous forme potentiellement salifiée (selon la concentration en base et le nombre de motifs du calixarène), c'est-à-dire qu'au moins l'un des groupements phénol libres est sous forme salifiée avec le cation métallique provenant de la base, en particulier le mélange de *p*-(R-oxy)calixarènes ou les *p*-(R-oxy)calixarènes purifiés sont obtenus sous forme mono-salifiée, c'est-à-dire qu'un seul des groupements phénol libres est sous forme salifiée avec le cation métallique provenant de la base.

Les inventeurs ont trouvé de façon surprenante que la combinaison d'au moins une base choisie parmi l'hydroxyde de rubidium ou l'hydroxyde de césium avec un *p*-(R-oxy)phénol et d'une concentration en base totale comprise de plus de 0,5 équivalent à 1,5 équivalent, avantageusement de 0,5 équivalent à 1,2 équivalent, en particulier égale à 0,6 équivalent, par rapport audit *p*-(R-oxy)phénol, permettait d'obtenir un mélange de douze *p*-(benzyloxy)calix[9-20]arènes de façon majoritaire ou purifiée, avec de bons rendements.

Ce résultat est d'autant plus inattendu que l'utilisation d'une concentration en base inférieure ou égale à 0,5 équivalent, telle que la potasse, la soude ou l'hydroxyde de césium avec un *p*-(R-oxy)phénol tel que le *p*-(benzyloxy)phénol conduit majoritairement avec de bons rendements à un mélange de *p*-(benzyloxy)calix[6-8]arènes.

Dans un mode de réalisation avantageux, la concentration en base est comprise de plus de 0,5 équivalent à 1,2 équivalent, préférentiellement de plus de 0,5 équivalent à 1,1 équivalent, préférentiellement de plus de 0,5 équivalent à 1 équivalent, préférentiellement de plus de 0,5 équivalent à 0,9 équivalent, préférentiellement de plus de 0,5 équivalent à 0,8 équivalent, préférentiellement de plus de 0,5 équivalent à 0,7 équivalent, préférentiellement de plus de 0,5 équivalent à 0,6 équivalent, en particulier 0,6 équivalent.

Dans un autre mode de réalisation, la concentration en base est comprise de 0,6 équivalent à 1,2 équivalent, préférentiellement de 0,6 équivalent à 1,1 équivalent, préférentiellement de 0,6 équivalent à 1 équivalent, préférentiellement de 0,6 équivalent à 0,9 équivalent, préférentiellement de 0,6 équivalent à 0,8 équivalent, préférentiellement de 0,6 équivalent à 0,7 équivalent, en particulier 0,6 équivalent.

A l'exception de l'hydroxyde de rubidium, une concentration inférieure ou égale à 0,5 équivalent, les *p*-(benzyloxy)calixarènes obtenus correspondent majoritairement aux p-(benzyloxy)calix[6-8] arènes.

Dans un mode de réalisation avantageux, la présente divulgation décrit l'utilisation d'au moins une base, en particulier choisie parmi la potasse, la soude, l'hydroxyde de lithium, l'hydroxyde de rubidium ou l'hydroxyde de césium, avec au moins un phénol substitué en position 4 de formule (I) tel que défini ci-dessus, dans laquelle le phénol substitué en position 4 de formule (I) est choisi parmi le 4-benzyloxyphénol ou le 4-octyloxyphénol.

Dans un mode de réalisation avantageux, la présente divulgation décrit l'utilisation d'au moins une base, en particulier choisie parmi la potasse, la soude, l'hydroxyde de lithium, l'hydroxyde de rubidium ou l'hydroxyde de césium, avec au moins un phénol substitué en position 4 de formule (I) tel que défini ci-dessus, dans laquelle le phénol substitué en position 4 de formule (I) est choisi parmi le 4-benzyloxyphénol, le 4-octyloxyphénol ou le 4-méthoxyphénol.

Dans un mode de réalisation avantageux, la présente divulgation décrit l'utilisation d'au moins une base, en particulier choisie parmi la potasse, la soude, l'hydroxyde de lithium, l'hydroxyde de rubidium ou l'hydroxyde de césium, avec au moins un phénol substitué en position 4 de formule (I), en particulier choisi parmi le 4-benzyloxyphénol ou le 4-octyloxyphénol, telle que définie ci-dessus, dans laquelle le milieu réactionnel initial avant début de la réaction est substantiellement dépourvu d'eau.

Dans un mode de réalisation avantageux, la présente divulgation décrit l'utilisation d'au moins une base, en particulier choisie parmi la potasse, la soude, l'hydroxyde de lithium, l'hydroxyde de rubidium ou l'hydroxyde de césium, avec au moins un phénol substitué en position 4 de formule (I), en particulier choisi parmi le 4-benzyloxyphénol, le 4-octyloxyphénol ou le 4-méthoxyphénol, telle que définie ci-dessus, dans laquelle le milieu réactionnel initial avant début de la réaction est substantiellement dépourvu d'eau.

L'expression « substantiellement dépourvue d'eau » signifie que la proportion d'eau présente dans le milieu réactionnel initial est inférieure ou égale à 5% en poids dans le milieu réactionnel, ou à l'état de traces.

L'eau présente dans le milieu réactionnel initial provient uniquement de la base introduite, éventuellement en solution aqueuse, telle que, par exemple, l'hydroxyde de rubidium, l'hydroxyde de césium, la soude ou la potasse, et de la quantité résiduelle présente dans le solvant et les réactifs constituant le milieu réactionnel tels que le xylène, le phénol substitué ou la source de formaldéhyde.

L'eau présente dans le milieu réactionnel final après complétion de la réaction ne provient que de la réaction elle-même et de la base utilisée, aucune addition d'eau extérieure avant la complétion de la réaction n'étant effectuée.

Dans un mode de réalisation avantageux, la présente invention concerne l'utilisation d'au moins une base, avec au moins un phénol substitué en position 4 de formule (I), en particulier choisi parmi le 4-benzyloxyphénol ou le 4-octyloxyphénol, le milieu réactionnel initial avant début de la réaction étant en particulier substantiellement dépourvu d'eau, telle que définie ci-dessus, dans laquelle la source de formaldéhyde est du para-formaldéhyde et le solvant organique est du xylène.

Dans un mode de réalisation avantageux, la présente invention concerne l'utilisation d'au moins une base, avec au moins un phénol substitué en position 4 de formule (I), en particulier choisi parmi le 4-benzyloxyphénol, le 4-octyloxyphénol ou le 4-méthoxyphénol, le milieu réactionnel initial avant début de la réaction étant en particulier substantiellement dépourvu d'eau, telle que définie ci-dessus, dans laquelle la source de formaldéhyde est du para-formaldéhyde et le solvant organique est du xylène.

Dans ces modes de réalisation, le formaldéhyde et la base utilisés sont non aqueux. L'eau présente dans le milieu réactionnel initial provient uniquement de la quantité résiduelle présente dans les réactifs constituant le milieu réactionnel tels que la base, le solvant ou la source de formaldéhyde.

L'eau présente dans le milieu réactionnel final après complétion de la réaction ne provient que de la réaction elle-même, car la réaction de préparation des *p*-(R-oxy)calix[9-20]arènes conduit à la formation d'eau, aucune addition d'eau extérieure avant la complétion de la réaction n'étant effectuée.

Avantageusement, la base utilisée est non aqueuse et correspond à tBuOK ou tBuONa.

Dans un mode de réalisation avantageux, la présente divulgation décrit l'utilisation d'au moins une base, en particulier choisie parmi la potasse, la soude, l'hydroxyde de lithium, l'hydroxyde de rubidium ou l'hydroxyde de césium, avec au moins un phénol substitué en position 4 de formule (I), en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique du xylène, telle que définie ci-dessus, dans laquelle ladite base est choisie parmi l'hydroxyde de césium ou l'hydroxyde de rubidium, notamment en solution aqueuse.

Dans un mode de réalisation avantageux, la base utilisée est l'hydroxyde de césium ou l'hydroxyde de rubidium, le phénol substitué en position 4 est le 4-benzyloxyphénol, et le mélange de *p*-(benzyloxy)calix[9-20]arènes obtenu est constitué majoritairement de *p*-(benzyloxy)calix[9]arène, *p*-(benzyloxy)calix[10]arène, *p*-(benzyloxy)calix[12]arène, *p*-(benzyloxy)calix[13]arène, *p*-(benzyloxy)calix[16]arène dans lequel le *p*-(benzyloxy)calix[12]arène est lui-même majoritaire et comprend également du *p*-(benzyloxy)calix[5]arène en proportion d'environ 10% molaires (déterminée par RMN).

Dans un mode de réalisation avantageux, la présente divulgation décrit l'utilisation d'au moins une base, en particulier choisie parmi la potasse, la soude, l'hydroxyde de lithium, l'hydroxyde de rubidium ou l'hydroxyde de césium, avec au moins un phénol substitué en position 4 de formule (I), en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique du xylène, telle que définie ci-dessus, dans laquelle ladite base est choisie parmi la soude ou la potasse, notamment en solution aqueuse.

Dans un mode de réalisation avantageux, la présente divulgation décrit l'utilisation d'au moins une base, en particulier choisie parmi la potasse, la soude, l'hydroxyde de lithium, l'hydroxyde de rubidium ou l'hydroxyde de césium, avec au moins un phénol substitué en position 4 de formule (I), en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique du xylène, ladite base étant choisie parmi la soude ou la potasse telle que définie ci-dessus, dans laquelle ledit mélange desdits *p*-(R-oxy)calix[9-20]arènes comprend de plus un *p*-(R-oxy)calix[7]arène sous forme de monosel de sodium ou de potassium, et/ou un *p*-(R-oxy)calix[6]arène en fonction de la base utilisée.

Dans ce mode de réalisation, la réaction conduit minoritairement à un mélange de *p*-(R-oxy)calix[9-20]arènes (entre 5 et 10% molaires) et majoritairement à un *p*-(R-oxy)calix[7]arène sous forme de monosel de sodium ou de potassium (entre 50 et 95% molaires) qui peut être isolé avant neutralisation de la base, et un *p*-(R-oxy)calix[6]arène isolé en faible proportion sous forme acide après neutralisation.

Dans un mode de réalisation plus avantageux, le phénol utilisé est le 4-benzyloxyphénol ou le 4-octyloxyphénol.

Les Inventeurs ont trouvé que lorsqu'au moins une base, choisie parmi la potasse et la soude, ladite base étant à une concentration totale de plus de 0,5 équivalent, notamment comprise de plus de 0,5 équivalent à 1,5 équivalent, avantageusement de plus de 0,5 équivalent à 1,2 équivalent, en particulier égale à 0,6 équivalent, par rapport au susdit

phénol substitué en position 4 de formule (I), est mise à réagir avec :

au moins un phénol substitué en position 4 de formule (I) tel que défini ci-dessus, en particulier le 4-benzyloxyphénol ou le 4-octyloxyphénol,
une source de formaldéhyde et un solvant organique constituant un milieu réactionnel, le milieu réactionnel initial étant substantiellement dépourvu d'eau,
des oligomères sont préalablement formés pour générer ensuite un dimère phénolique substitué en position 4 de formule (II) suivante :

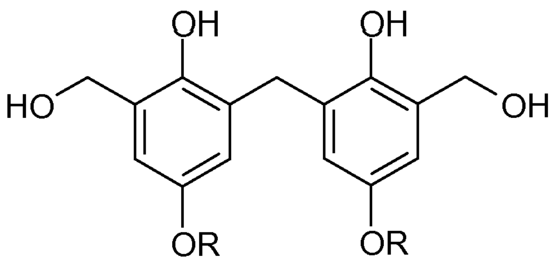

(II)

dans laquelle R est tel que défini dans la formule (I) ci-dessus.

Lorsque ledit dimère est laissé à réagir, il conduit alors à un mélange de *p*-(R-oxy)calix[9-20]arènes obtenu minoritairement, de *p*-(R-oxy)calix[7]arène sous forme de monosel de sodium ou de potassium, de *p*-(R-oxy)calix[6]arène et de *p*-(R-oxy)calix[8]arène, ces trois derniers composés étant obtenus majoritairement.
Selon un autre aspect, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange desdits *p*-(R-oxy)calix[9-20]arènes, comprenant une étape de mise en contact d'une base, choisie parmi l'hydroxyde de rubidium ou l'hydroxyde de césium, avec au moins un phénol substitué en position 4 de formule (I) suivante :

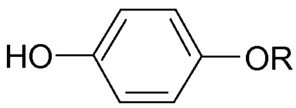

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)ORaOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire,
- un PEG-1 à 10 ramifié ou linéaire, dont le groupe terminal -OH est alkylé par un alkyle $C_1$-$C_{20}$ ramifié ou linéaire,

une source de formaldéhyde et un solvant organique,
ledit milieu réactionnel étant chauffé à une température comprise de 50 °C à 250 °C durant un temps compris de 4 à 24h et le mélange de ladite base, dudit phénol, de ladite source de formaldéhyde et dudit solvant organique constituant un milieu réactionnel, ladite base étant à une concentration totale comprise de plus de 0,5 équivalent à 1,5 équivalent, avantageusement de plus de 0,5 équivalent à 1,2 équivalent, en particulier égale à 0,6 équivalent par rapport audit au moins un phénol substitué en position 4 de formule (I).
Le chauffage dépend du solvant organique utilisé et est compris de 50°C à 250°C, en particulier au reflux, c'est-à-dire au point d'ébullition du solvant organique utilisé.
L'expression « source de formaldéhyde » signifie que du paraformaldéhyde, du trioxane ou du formaldéhyde

en solution aqueuse tel que de la formaline ou du formol peuvent être utilisés.

Le terme solvant organique désigne tout solvant non polaire dont le point d'ébullition est supérieur à environ 50°C, en particulier un solvant dont le point d'ébullition est compris de 50 à 250°C, en particulier le xylène, le toluène, l'octane, le diphenyléther, le mésitylène, le chlorobenzène, le dichlorobenzène, le trichlorobenzène.

Dans ce mode de réalisation, le milieu réactionnel initial, c'est-à-dire après introduction de tous les réactifs dans le solvant avant chauffage, peut contenir également de l'eau ajoutée dans une proportion de 1 à 10% en poids.

La présente invention concerne également un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,

comprenant une étape de mise en contact d'une base, choisie parmi l'hydroxyde de rubidium ou l'hydroxyde de césium, avec au moins un phénol substitué en position 4 de formule (I) suivante :

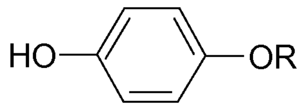

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)ORaOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire,
- un PEG-1 à 10 ramifié ou linéaire, dont le groupe terminal -OH est alkylé par un alkyle $C_1$-$C_{20}$ ramifié ou linéaire,

une source de formaldéhyde et un solvant organique,

ledit milieu réactionnel étant chauffé à une température comprise de 50 °C à 250 °C durant un temps compris de 4 à 24h et le mélange de ladite base, dudit phénol, de ladite source de formaldéhyde et dudit solvant organique constituant un milieu réactionnel,

ladite base étant à une concentration totale comprise de plus de 0,5 équivalent à 1,5 équivalent, avantageusement de plus de 0,5 équivalent à 1,2 équivalent, en particulier égale à 0,6 équivalent par rapport audit au moins un phénol substitué en position 4 de formule (I).

Dans un mode de réalisation avantageux, la concentration en base est comprise de plus de 0,5 équivalent à 1,2 équivalent par phénol, de plus de 0,5 équivalent à 1,1 équivalent par phénol, de plus de 0,5 équivalent à 1 équivalent par phénol, de plus de 0,5 équivalent à 0,9 équivalent par phénol, préférentiellement de plus de 0,5 équivalent à 0,8 équivalent, préférentiellement de plus de 0,5 équivalent à 0,7 équivalent, préférentiellement de plus de 0,5 équivalent à 0,6 équivalent, en particulier 0,6 équivalent.

Dans un autre mode de réalisation, la concentration en base est comprise de 0,6 équivalent à 1 équivalent, préférentiellement de 0,6 équivalent à 0,9 équivalent, préférentiellement de 0,6 équivalent à 0,8 équivalent, préférentiellement 0,6 équivalent à 0,7 équivalent, en particulier 0,6 équivalent.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange desdits *p*-(R-oxy)calix[9-20]arènes,

tel que défini ci-dessus, dans lequel ledit phénol substitué en position 4 de formule (I) est choisi parmi le 4-benzyloxyphénol ou le 4-octyloxyphénol.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,

tel que défini ci-dessus, dans lequel ledit phénol substitué en position 4 de formule (I) est choisi parmi le 4-benzyloxyphénol ou le 4-octyloxyphénol.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène , un *p*-(R-oxy)calix[19]arène, un *p*-(R-oxy)calix[20]arène, ou
- d'un mélange desdits *p*-(R-oxy)calix[9-20]arènes,

dans lequel ledit phénol substitué en position 4 de formule (I) est en particulier choisi parmi le 4-benzyloxyphénol ou le 4-octyloxyphénol, tel que défini ci-dessus,
dans lequel le milieu réactionnel initial avant début de la réaction est substantiellement dépourvu d'eau.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène , un *p*-(R-oxy)calix[19]arène, un *p*-(R-oxy)calix[20]arène, ou
- d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,

dans lequel ledit phénol substitué en position 4 de formule (I) est en particulier choisi parmi le 4-benzyloxyphénol ou le 4-octyloxyphénol, tel que défini ci-dessus,
dans lequel le milieu réactionnel initial avant début de la réaction est substantiellement dépourvu d'eau.

L'expression « substantiellement dépourvue d'eau » signifie que la proportion d'eau présente dans le milieu réactionnel initial est inférieure à 4,5% en poids dans le milieu réactionnel, ou à l'état de traces.

L'eau présente dans le milieu réactionnel initial provient uniquement de la base introduite, éventuellement en solution aqueuse, choisie parmi l'hydroxyde de rubidium, ou l'hydroxyde de césium, et de la quantité résiduelle présente dans le solvant et les réactifs constituant le milieu réactionnel tels que le xylène, le phénol substitué ou la source de formaldéhyde.

Dans ce mode de réalisation, le formaldéhyde est non aqueux. L'eau présente dans le milieu réactionnel initial provient uniquement de la base utilisée en solution aqueuse et de la quantité résiduelle présente dans le solvant et les réactifs constituant le milieu réactionnel tels que le xylène, le phénol substitué ou la source de formaldéhyde.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange desdits *p*-(R-oxy)calix[9-20]arènes,

dans lequel ledit phénol substitué en position 4 de formule (I) est en particulier choisi parmi le 4-benzyloxyphénol ou le 4-octyloxyphénol, le milieu réactionnel initial avant début de la réaction étant en particulier substantiellement dépourvu d'eau, tel que défini ci-dessus, dans lequel la source de formaldéhyde est du para-formaldéhyde et le solvant organique est du xylène.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15] arène, un *p*-(R-oxy)calix[16]arène, un *p*-(R-oxy)calix[17]arène, un *p*-(R-oxy)calix[18]arène, un *p*-(R-oxy)calix[19] arène, un *p*-(R-oxy)calix[20]arène, ou
- d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,

dans lequel ledit phénol substitué en position 4 de formule (I) est en particulier choisi parmi le 4-benzyloxyphénol ou le 4-octyloxyphénol, le milieu réactionnel initial avant début de la réaction étant en particulier substantiellement dépourvu d'eau, tel que défini ci-dessus, dans lequel la source de formaldéhyde est du para-formaldéhyde et le solvant organique est du xylène.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11] arène, un *p*-(R-oxy)calix[12]arène, un *p*-(R-oxy)calix[13]arène, un *p*-(R-oxy)calix[14]arène, un *p*-(R-oxy)calix[15] arène, un *p*-(R-oxy)calix[16]arène, un *p*-(R-oxy)calix[17]arène, un *p*-(R-oxy)calix[18]arène, un *p*-(R-oxy)calix[19] arène, un *p*-(R-oxy)calix[20]arène, ou
- d'un mélange desdits *p*-(R-oxy)calix[9-20]arènes,

dans lequel ledit phénol substitué en position 4 de formule (I) est en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique étant en particulier du xylène, tel que défini ci-dessus, dans lequel de l'eau est formée dans le milieu réactionnel durant ladite préparation dudit composé, ladite eau étant optionnellement substantiellement éliminée du milieu réactionnel durant la réaction.

Dans ce mode de réalisation, l'eau est ou non éliminée du milieu réactionnel :

1) L'eau formée peut être substantiellement éliminée durant la réaction au fur et à mesure de sa formation par des méthodes bien connues de l'homme du métier telles qu'un appareil de Dean-Stark ou un balayage du milieu réactionnel avec un gaz neutre, en particulier de l'azote ou de l'argon.
Dans ce cas, la proportion d'eau présente après complétion de la réaction est de moins de 1% en poids.
2) L'eau formée peut être conservée durant la réaction. L'eau présente dans le milieu réactionnel final après complétion de la réaction ne provient que de la réaction elle-même dont le mécanisme réactionnel conduit à la formation d'eau et aucune addition d'eau extérieure avant la complétion de la réaction n'est effectuée.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11] arène, un *p*-(R-oxy)calix[12]arène, un *p*-(R-oxy)calix[13]arène, un *p*-(R-oxy)calix[14]arène, un *p*-(R-oxy)calix[15] arène, un *p*-(R-oxy)calix[16]arène, un *p*-(R-oxy)calix[17]arène, un *p*-(R-oxy)calix[18]arène, un *p*-(R-oxy)calix[19] arène, un *p*-(R-oxy)calix[20]arène, ou
- d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,

dans lequel ledit phénol substitué en position 4 de formule (I) est en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique étant en particulier du xylène, tel que défini ci-dessus, dans lequel de l'eau est formée dans le milieu réactionnel durant ladite préparation dudit composé, ladite eau étant optionnellement substantiellement éliminée du milieu réactionnel durant la réaction.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11] arène, un *p*-(R-oxy)calix[12]arène, un *p*-(R-oxy)calix[13]arène, un *p*-(R-oxy)calix[14]arène, un *p*-(R-oxy)calix[15] arène, un *p*-(R-oxy)calix[16]arène, un *p*-(R-oxy)calix[17]arène, un *p*-(R-oxy)calix[18]arène, un *p*-(R-oxy)calix[19] arène, un *p*-(R-oxy)calix[20]arène, ou
- d'un mélange desdits *p*-(R-oxy)calix[9-20]arènes,

dans lequel ledit phénol substitué en position 4 de formule (I) est en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique étant en particulier

du xylène, l'eau formée dans le milieu réactionnel durant ladite préparation dudit composé étant optionnellement substantiellement éliminée dudit milieu réactionnel, tel que défini ci-dessus, dans lequel ladite base est choisie parmi l'hydroxyde de césium et l'hydroxyde de rubidium, notamment en solution aqueuse.

Dans ce mode de réalisation l'hydroxyde de rubidium ou de césium sont utilisés dans un milieu réactionnel initial substantiellement dépourvu d'eau ou non et l'eau formée durant la réaction est substantiellement éliminée ou conservée dans le milieu réactionnel.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,

dans lequel ledit phénol substitué en position 4 de formule (I) est en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique étant en particulier du xylène, l'eau formée dans le milieu réactionnel durant ladite préparation dudit composé étant optionnellement substantiellement éliminée dudit milieu réactionnel, tel que défini ci-dessus, dans lequel ladite base est choisie parmi l'hydroxyde de césium et l'hydroxyde de rubidium, notamment en solution aqueuse.

Dans un mode de réalisation avantageux, la présente invention concerne un des procédés de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange desdits *p*-(R-oxy)calix[9-20]arènes,
  tel que défini ci-dessus, comprenant les étapes suivantes :

  a. mise en contact de l'hydroxyde de césium et/ou de rubidium, notamment en solution aqueuse, avec au moins un phénol substitué en position 4 de formule (I), ladite base étant à une concentration totale comprise de plus de 0,5 équivalent à 1,5 équivalent, avantageusement de plus de 0,5 équivalent à 1,2 équivalent, en particulier égale à 0,6 équivalent par rapport audit au moins un phénol substitué en position 4 de formule (I), une source de formaldéhyde, en particulier du paraformaldéhyde dans un solvant organique puis chauffage en éliminant optionnellement ladite eau formée du milieu réactionnel,
  pour obtenir :

  un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou, un mélange desdits *p*-(R-oxy)calix[9-20]arènes, sous forme salifiée dans le milieu réactionnel final,

  b. neutralisation de ladite forme salifiée dudit *p*-(R-oxy)calixarène ou dudit mélange pour obtenir ledit *p*-(R-oxy)calixarène ou ledit mélange sous forme neutralisée.

Dans un mode de réalisation avantageux, la présente invention concerne un des procédés de préparation :

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange desdits *p*-(R-oxy)calix[9-20]arènes,

tel que défini ci-dessus, comprenant les étapes suivantes :

a. mise en contact de l'hydroxyde de césium et/ou de rubidium, notamment en solution aqueuse, avec au moins

un phénol substitué en position 4 de formule (I), ladite base étant à une concentration totale comprise de plus de 0,5 équivalent à 1,5 équivalent, avantageusement de plus de 0,5 équivalent à 1,2 équivalent, en particulier égale à 0,6 équivalent par rapport audit au moins un phénol substitué en position 4 de formule (I), une source de formaldéhyde, en particulier du paraformaldéhyde dans un solvant organique puis chauffage en éliminant optionnellement ladite eau formée du milieu réactionnel,
pour obtenir :

un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, unp-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou, un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,
sous forme salifiée dans le milieu réactionnel final,

b. neutralisation de ladite forme salifiée dudit *p*-(R-oxy)calixarène ou dudit mélange pour obtenir ledit *p*-(R-oxy)calixarène ou ledit mélange sous forme neutralisée.

Dans l'étape a., l'hydroxyde de rubidium et/ou de césium utilisé peut être en solution aqueuse ou solide. Lorsque l'hydroxyde de rubidium et/ou de césium est en solution aqueuse, la concentration de la base en solution aqueuse utilisée est avantageusement d'environ 50% en poids. La source de formaldéhyde peut être aqueuse ou non, en particulier sous forme non aqueuse et notamment elle correspond à du paraformaldéhyde.

Le milieu réactionnel initial peut donc être substantiellement dépourvu d'eau ou non.

Le solvant organique désigne tout solvant non polaire dont le point d'ébullition est supérieur à environ 50°C, en particulier un solvant dont le point d'ébullition est compris de 50 à 250°C, en particulier le xylène, le toluène, l'octane, le diphenyléther, le mésitylène, le chlorobenzène, le dichlorobenzène, le trichlorobenzène, en particulier le solvant organique est le xylène.
le chauffage est compris de 50°C à 250°C, en particulier il est effectué au point d'ébullition du solvant organique utilisé, durant un temps compris de 4 à 24h pour obtenir ledit *p*-(R-oxy)calix[n]arène dans lequel n est compris de 9 à 20 ou ledit mélange sous forme salifiée dans le milieu réactionnel final.

L'eau formée dans le milieu réactionnel durant la réaction peut être éliminée par des techniques bien connues de l'homme du métier, par exemple au moyen d'un appareil de Dean-Stark Stark ou un balayage du milieu réactionnel avec un gaz neutre, en particulier de l'azote ou de l'argon.

Dans l'étape b., la neutralisation est effectuée avec un acide, en particulier en solution aqueuse, notamment HCl ou $H_2SO_4$. De préférence HCl, qui peut être facilement évaporé.

Avantageusement, le formaldéhyde utilisé est du paraformaldéhyde, l'hydroxyde de césium et/ou de rubidium sont en solution aqueuse, le solvant utilisé est le xylène et le temps de réaction est compris de 5 à 9h, en particulier 7h. le temps de réaction de l'étape a. ci-dessus défini est compris de 30 min à 1h pour obtenir dans un premier temps des oligomères linéaires d'unités phénoliques puis, dans un second temps, le dimère de formule (II) défini ci-dessus, sous forme salifiée, de façon quasi quantitative, qui après ladite neutralisation de ladite étape b. , est obtenu sous forme neutralisée.

Lorsque le chauffage est poursuivi avec le dimère salifié, il y a alors formation des calixarènes de l'invention.

Ledit mélange de *p*-(R-oxy)calixarène est constitué d'au moins 50% de *p*-(R-oxy)calix[9-20]arènes, en particulier 50% d'un mélange comprenant le *p*-(R-oxy)calix[9]arène, le *p*-(R-oxy)calix[10]arène, le *p*-(R-oxy)calix[12]arène, le *p*-(R-oxy)calix[13]arène, le *p*-(R-oxy)calix[16]arène, ainsi que du *p*-(R-oxy)cali[5]arène, en particulier dans une proportion d'environ 10% molaires.

Dans un mode de réalisation avantageux, le procédé défini ci-dessus comprend une étape additionnelle de purification dudit *p*-(R-oxy)calixarène ou dudit mélange sous forme neutralisée, notamment par cristallisation dans un mélange de solvants à base de DMSO pour obtenir dans le solide ledit *p*-(R-oxy)calixarène ou ledit mélange sous forme neutralisée purifié, suivi éventuellement d'une chromatographie.

L'étape additionnelle de purification est effectuée après l'étape b. définie ci-dessus.

Le mélange de solvant à base d'un solvant aprotique polaire, en particulier à base de DMSO ou de DMF, et notamment un mélange DMSO/toluène ou DMSO/acétone, le DMSO étant dans une proportion de 5% à 50% en volume dans le mélange de solvant, en particulier de 10%.

Le deuxième solvant peut également être un mélange de solvants toluène/acétone quelle que soit la proportion de chacun des deux solvants, soit : acétone de 0,1 à 99,9% en poids et toluène : de 99,9 à 0,1% en poids.

Dans un mode de réalisation avantageux, le mélange constitué d'au moins 50% de *p*-(R-oxy)calix[9-20]arène et du *p*-(R-oxy)calix[5]arène, purifié ou non, est un mélange constitué d'au moins 50% de *p*-(benzyloxy)calix[9-20]arène

et de *p*-(benzyloxy)calix[5]arène ou constitué d'au moins 50% de *p*-(octyloxy)calix[9-20]arène et de *p*-(octyloxy)calix[5] arène.

Dans un mode de réalisation avantageux, la présente invention concerne un procédé de préparation :

- d'un *p*-(R-oxy)calixarène choisi parmi un *p*-(R-oxy)calix[9]arène, un *p*-(R-oxy)calix[10]arène, un *p*-(R-oxy)calix[11] arène, un *p*-(R-oxy)calix[12]arène, un *p*-(R-oxy)calix[13]arène, un *p*-(R-oxy)calix[14]arène, un *p*-(R-oxy)calix[15] arène, un *p*-(R-oxy)calix[16]arène, un *p*-(R-oxy)calix[17]arène, un *p*-(R-oxy)calix[18]arène, un *p*-(R-oxy)calix[19] arène, un *p*-(R-oxy)calix[20]arène, ou
- d'un mélange desdits *p*-(R-oxy)calix[9-20]arènes,

dans lequel ledit phénol substitué en position 4 de formule (I) est en particulier choisi parmi le 4-benzyloxyphénol ou le 4-octyloxyphénol, le milieu réactionnel initial avant début de la réaction étant en particulier substantiellement dépourvu d'eau, la source de formaldéhyde étant en particulier du para-formaldéhyde et le solvant organique étant en particulier du xylène, l'eau formée dans le milieu réactionnel durant ladite préparation dudit composé étant optionnellement substantiellement éliminée dudit milieu réactionnel, dans lequel ladite base est choisie parmi la soude ou la potasse tel que défini ci-dessus, dans lequel ledit *p*-(R-oxy)calixarène ou ledit mélange desdits *p*-(R-oxy)calix[9-20]arènes comprend de plus un *p*-(R-oxy)calix[7]arène sous forme de monosel de sodium ou de potassium en fonction de la base utilisée.

Dans ce mode de réalisation, le mélange de *p*-(R-oxy)calix[9-20]arènes obtenu est minoritaire (entre 5 et 10% molaires) et la réaction conduit majoritairement au *p*-(R-oxy)calix[7]arène sous forme de monosel de sodium ou de potassium qui peut être isolé sous cette forme entre 50 et 95% molaires.

La présente divulgation décrit un dimère phénolique substitué en position 4 de formule (II) suivante :

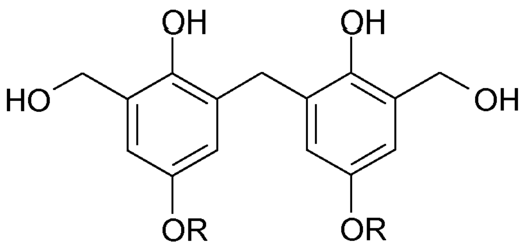

(II)

dans laquelle R est tel que défini dans la formule (I) ci-dessus.

Dans un mode de réalisation avantageux, la présente divulgation décrit le *p*-(*R*-oxy)calix[7]arène sous forme de monosel de sodium ou de potassium, en particulier le *p*-(octyloxy)calix[7]arène sous forme de monosel de sodium ou de potassium ou le *p*-(benzyloxy)calix[7]arène sous forme de monosel de sodium ou de potassium.

Ces deux composés sous forme de sels de sodium ou de potassium sont des produits nouveaux.

Dans un mode de réalisation avantageux, la présente divulgation décrit un dimère phénolique, dans lequel R est choisi parmi le groupe benzyle ou le groupe octyle.

La présente divulgation décrit un *p*-(R-oxy)calixarène ou un mélange de *p*-(R-oxy)calix[9-20]arènes de formule (III) suivante :

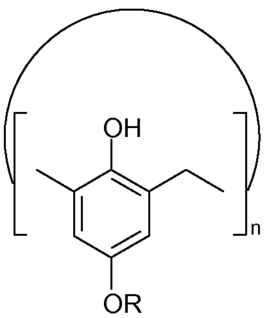

(III)

dans lequel n est un entier compris de 9 à 20, R étant tel que défini dans la formule (I) ci-dessus.

Le *p*-(R-oxy)calix[n]arène dans lequel n est un entier compris de 9 à 20, correspond donc à un composé choisi parmi un *p*-(R-oxy)calix[9]arène, un *p*-(R-oxy)calix[10]arène, un *p*-(R-oxy)calix[ll]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou un mélange de ceux-ci comprenant au moins deux *p*-(R-oxy)calix[9-20]arènes parmi les douze définis ci-dessus.

Chaque *p*-(R-oxy)calix[n]arène présent dans ledit mélange, dans lequel n est un entier compris de 9 à 20, peut être dans une gamme de proportion comprise de 0 à 100% à condition qu'au moins deux *p*-(R-oxy)calix[n]arènes dans lequel n est un entier compris de 9 à 20 soient présents en proportion supérieure à 0.

La présente divulgation décrit également un *p*-(R-oxy)calixarène ou un mélange d'au moins deux *p*-(R-oxy)calix[9-20]arènes dans lequel lesdits au moins deux *p*-(R-oxy)calixarènes sont présents dans ledit mélange à raison d'au moins 5% molaire chacun de formule (III) suivante :

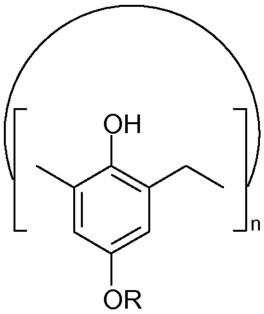

(III)

dans lequel n est un entier compris de 9 à 20, R étant tel que défini dans la formule (I) ci-dessus.

En particulier, le *p*-(R-oxy)calix[n]arène dans lequel n est un entier compris de 9 à 20, correspond donc à un composé choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16] arène, un *p*-(R-oxy)calix[17]arène, un *p*-(R-oxy)calix[18]arène, un *p*-(R-oxy)calix[19]arène, un *p*-(R-oxy)calix[20]arène, ou un mélange de ceux-ci comprenant au moins deux *p*-(R-oxy)calix[9-20]arènes parmi les douze définis ci-dessus, dans lequel lesdits au moins deux *p*-(R-oxy)calixarènes sont présents dans ledit mélange à raison d'au moins 5% molaire chacun.

Ainsi, les au moins deux *p*-(R-oxy)calix[n]arènes présents dans ledit mélange, dans lequel n est un entier compris de 9 à 20, peuvent être dans une gamme de proportion comprise de 5 à 95% chacun.

Dans un autre mode de réalisation avantageux, la présente divulgation décrit un *p*-(R-oxy)calix[n]arène dans lequel n est un entier compris de 9 à 20 ou un mélange de *p*-(R-oxy)calix[9-20]arènes, tel que défini ci-dessus, dans lequel R est choisi parmi le groupe benzyle ou le groupe octyle.

Dans un autre mode de réalisation avantageux, le mélange de *p*-(R-oxy)calix[9-20]arènes obtenu est constitué majoritairement de *p*-(R-oxy)calix[9]arène, *p*-(R-oxy)calix[10]arène, *p*-(R-oxy)calix[12]arène, *p*-(R-oxy)calix[13]arène, *p*-(R-oxy)calix[16]arène, en particulier le mélange correspond à un mélange de *p*-(benzyloxy)calix[9-20]arènes constitué majoritairement de *p*-(benzyloxy)calix[9] arène, *p*-(benzyloxy)calix[10]arène, *p*-(benzyloxy)calix[12]arène, *p*-(benzyloxy)calix[13]arène, *p*-(benzyloxy)calix[16]arène ou de *p*-(octyloxy)calix[9-20]arènes constitué majoritairement de *p*-(octyloxy)calix[9]arène, *p-(*octyloxy)calix[10]arène, *p*-( octyloxy)calix[12]arène, *p*-(octyloxy)calix[13]arène, *p*-(octyloxy)calix[16]arène.

Dans un autre mode de réalisation avantageux, le mélange de *p*-(benzyloxy)calix[9-20]arènes obtenu est constitué majoritairement de *p*-(benzyloxy)calix[9]arène, *p*-(benzyloxy)calix[10]arène, *p*-(benzyloxy)calix[12]arène, *p*-(benzyloxy)calix[13]arène, *p*-(benzyloxy)calix[16]arène ou de *p*-(octyloxy)calix[9-20]arènes constitué majoritairement de *p*-( octyloxy)calix[9]arène, *p*-(octyloxy)calix[10]arène, *p*-(octyloxy)calix[12]arène, *p*-(octyloxy)calix[13]arène, *p*-(octyloxy)calix[16]arène et comprend également du *p*-(R-oxy)calix[5]arène, en particulier du *p*-(benzyloxy)calix[5]arène ou du *p*-(octyloxy)calix[5]arène en proportion d'environ 10% en poids.

Selon un autre aspect, la présente divulgation décrit un *p*-(R-oxy)calix[5]arène, en particulier le *p*-(benzyloxy)calix[5]arène ou le *p*-(octyloxy)calix[5]arène.

Le *p*-(benzyloxy)calix[5]arène ou le *p*-(octyloxy)calix[5]arène peuvent être obtenus aisément par purification du mélange le contenant ci-dessus défini.

Dans un mode de réalisation avantageux, la présente divulgation décrit un *p*-(R-oxy)calix[n]arène dans lequel n est un entier compris de 9 à 20 ou un mélange de *p*-(R-oxy)calix[9-20]arènes, tel que défini ci-dessus, R étant en particulier choisi parmi le groupe benzyle ou le groupe octyle, comprenant de plus un *p*-(R-oxy)calix[7]arène, en particulier *le p*-(benzyloxy)calix[7]arène ou le *p*-(octyloxy)calix[7]arène.

La proportion de *p*-(R-oxy)calix[7]arène présente avec le *p*-(R-oxy)calix[n]arène dans lequel n est un entier compris de 9 à 20 ou avec un mélange de *p*-(R-oxy)calix[9-20]arène est variable et est comprise de plus de 0% à 99,1% en poids.

Dans ce mode de réalisation, le *p*-(R-oxy)calix[7]arène peut être sous forme salifiée ou non.

Dans un autre mode de réalisation avantageux, la présente divulgation décrit un p-(R-oxy)calix[n]arène dans lequel n est un entier compris de 9 à 20 ou un mélange de *p*-(R-oxy)calix[9-20]arènes, R étant en particulier choisi parmi le groupe benzyle ou le groupe octyle, tel que défini ci-dessus, comprenant de plus un *p*-(R-oxy)calix[7]arène sous forme de monosel de sodium ou potassium.

La proportion de *p*-(R-oxy)calix[7]arène sous forme salifiée présente avec le *p*-(R-oxy)calix[n]arène dans lequel n est un entier compris de 9 à 20 ou avec un mélange de *p*-(R-oxy)calix[9-20]arène est variable et est comprise de plus de 0% à 99,1% en poids.

Dans un autre mode de réalisation avantageux, la présente divulgation décrit le *p*-(R-oxy)calix[7]arène sous forme de monosel de sodium ou potassium, en particulier le *p*-(benzyloxy)calix[7]arène ou le *p*-(octyloxy)calix[7]arène sous forme de monosel de sodium ou potassium.

Le *p*-(R-oxy)calix[7]arène, en particulier le *p*-(benzyloxy)calix[7]arène ou le *p*-(octyloxy)calix[7] arène, sous forme de monosel de sodium ou potassium, peuvent être obtenus aisément par filtration du milieu réactionnel final du mélange le contenant défini ci-dessus.

Selon un autre aspect, la présente divulgation décrit l'utilisation d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un p-(R-oxy)calix[15]arène, un *p*-(R-oxy)calix[16]arène, un *p*-(R-oxy)calix[17] arène, un *p*-(R-oxy)calix[18]arène, un *p*-(R-oxy)calix[19]arène, un *p*-(R-oxy)calix[20]arène, ou d'un mélange desdits *p*-(R-oxy)calix[9-20]arènes, pour la constitution d'un matériau ou dans le cadre du renfort mécanique des matériaux.

La présente divulgation décrit également l'utilisation d'un *p*-(R-oxy)calixarène choisi parmi un *p*-(R-oxy)calix[9] arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un p-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou d'un mélange d'au moins deux desdits *p*-(R-oxy)calix[9-20]arènes, pour la constitution d'un matériau ou dans le cadre du renfort mécanique des matériaux dans lequel lesdits au moins deux *p*-(R-oxy)calixarènes sont présents dans ledit mélange à raison d'au moins 5% molaire chacun.

Le *p*-(R-oxy)calix[n]arène dans lequel n est compris de 9 à 20 ou le mélange desdits *p*-(R-oxy)calix[9-20]arènes peut également être utilisé pour le piégeage des particules, la synthèse de particules métalliques avec des dimensions contrôlées et une faible dispersion, la nanofiltration, la filtration de gaz, la complexation d'ions, la vectorisation et l'encapsulation de molécules. La cavité importante présente au cœur des grands calixarènes permet également d'envisager la formation de matériaux poreux, réticulés ou pas, ayant des applications dans les gels ou les mousses.

La présente divulgation décrit également l'utilisation d'un *p*-(R-oxy)calixarène choisi parmi un *p*-(R-oxy)calix[9] arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un p-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou d'un mélange desdits *p*-(R-oxy)calix[9-20] arènes, pour la constitution d'un matériau, par exemple un matériau poreux, réticulé ou non réticulé, ledit matériau pouvant être utilisé dans la mise en œuvre d'un procédé d'obtention de gels ou de mousses, pour la constitution d'un matériau dans le cadre du renfort mécanique des matériaux, pour la synthèse de particules métalliques avec des dimensions contrôlées et une faible dispersion, pour la nanofiltration, pour la filtration de gaz, pour la complexation d'ions, pour la vectorisation ou pour l'encapsulation de molécules.

La présente divulgation décrit également l'utilisation d'un *p*-(R-oxy)calixarène choisi parmi un *p*-(R-oxy)calix[9] arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un p-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun, pour la constitution d'un matériau, par exemple un matériau poreux, réticulé ou non réticulé, ledit matériau pouvant être utilisé dans la mise en œuvre d'un procédé d'obtention de gels ou de mousses, pour la constitution d'un matériau dans le cadre du renfort mécanique des matériaux, pour la synthèse de particules métalliques avec des dimensions contrôlées et une faible dispersion, pour la nanofiltration, pour la filtration de gaz, pour la complexation d'ions, pour la vectorisation ou pour l'encapsulation de molécules.

Dans un mode de réalisation, la présente divulgation décrit l'utilisation d'un *p*-(R-oxy)calixarène choisi parmi un *p*-(R-oxy)calix[9]arène, un *p*-(R-oxy)calix[10]arène, un *p*-(R-oxy)calix[ll]arène, un *p*-(R-oxy)calix[12]arène, un *p*-(R-oxy)calix[13]arène, un *p*-(R-oxy)calix[14]arène, un p-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou d'un mélange desdits *p*-(R-oxy)calix[9-20]arènes, telle que définie ci-dessus dans laquelle ledit *p*-(R-oxy)calixarène ou ledit mélange comprend de plus un *p*-(R-oxy)calix[7] arène.

Dans un mode de réalisation, la présente divulgation décrit l'utilisation d'un *p*-(R-oxy)calixarène choisi parmi un *p*-(R-oxy)calix[9]arène, un *p*-(R-oxy)calix[10]arène, un *p*-(R-oxy)calix[ll]arène, un *p*-(R-oxy)calix[12]arène, un *p*-(R-oxy)calix[13]arène, un *p*-(R-oxy)calix[14]arène, un p-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou d'un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun, telle que définie ci-dessus dans laquelle ledit *p*-(R-oxy)calixarène ou ledit mélange comprend de plus un *p*-(R-oxy)calix[7]arène.

Le *p*-(R-oxy)calix[7]arène peut être sous forme de sel de sodium ou de potassium ou neutre.

Dans un mode de réalisation avantageux, le groupe R dudit *p*-(R-oxy)calix[n]arène, dans lequel n est compris de 9 à 20 ou dudit mélange desdits *p*-(R-oxy)calix[9-20]arènes utilisés avec ou sans *p*-(R-oxy)calix[7]arène, est un benzyle ou un octyle.

Selon encore un autre aspect, la présente divulgation décrit l'utilisation d'un *p*-(R-oxy)calixarène ou d'un mélange de *p*-(R-oxy)calixarènes tel que défini ci-dessus, susceptible d'être obtenu par l'un des procédés défini ci-dessus, pour la constitution d'un matériau ou dans le cadre du renfort mécanique des matériaux.

Selon encore un autre aspect, le *p*-(R-oxy)calixarène ou le mélange de *p*-(R-oxy)calixarènes tel que défini ci-dessus, susceptible d'être obtenu par l'un des procédés défini ci-dessus, peut également être utilisé pour le piégeage des particules, la synthèse de particules métalliques avec des dimensions contrôlées et une faible dispersion, la nanofiltration, la filtration de gaz, la complexation d'ions, la vectorisation et l'encapsulation de molécules. La cavité importante présente au cœur des grands calixarènes permet également d'envisager la formation de matériaux poreux, réticulés ou pas, ayant des applications dans les gels ou les mousses.

Selon encore un autre aspect, la présente divulgation décrit l'utilisation d'un *p*-(R-oxy)calixarène ou d'un mélange de *p*-(R-oxy)calixarènes tel que défini ci-dessus, susceptible d'être obtenu par l'un des procédés défini ci-dessus, pour la constitution d'un matériau, par exemple un matériau poreux, réticulé ou non réticulé, ledit matériau pouvant être utilisé dans la mise en œuvre d'un procédé d'obtention de gels ou de mousses, pour la constitution d'un matériau dans le cadre du renfort mécanique des matériaux, pour la synthèse de particules métalliques avec des dimensions contrôlées et une faible dispersion, pour la nanofiltration, pour la filtration de gaz, pour la complexation d'ions, pour la vectorisation ou pour l'encapsulation de molécules.

La présente divulgation décrit également l'utilisation d'au moins une base, ladite base étant l'hydroxyde de rubidium, avec un phénol de formule (I), ladite base étant à une concentration totale supérieure ou égale à 0,3 équivalent et inférieure ou égale à 0,5 équivalent par rapport audit phénol de formule (I), pour la préparation d'un mélange comprenant du *p*-(R-oxy)calix[6]arène, du *p*-(R-oxy)calix[7]arène et du *p*-(R-oxy)calix[8]arène et un mélange *de p*-(R-oxy)calix[9-20]arènes.

La présente divulgation décrit également l'utilisation d'au moins une base, ladite base étant l'hydroxyde de rubidium, avec le p-(benzyloxy)phénol, ladite base étant à une concentration totale supérieure ou égale à 0,3 équivalent et inférieure ou égale à 0,5 équivalent par rapport audit p-(benzyloxy)phénol, pour la préparation d'un mélange comprenant du *p*-(benzyloxy)calix[6]arène, du *p*-(benzyloxy)calix[7]arène et du *p*-(benzyloxy)calix[8]arène et un mélange de *p*-(benzyloxy)calix[9-20]arènes.

Les Inventeurs ont trouvé de manière surprenante que la combinaison d'au moins une base, ladite base étant l'hydroxyde de rubidium, et d'une concentration totale supérieure ou égale à 0,3 équivalent et inférieure ou égale à 0,5 équivalent par rapport audit phénol de formule (I) permettait d'obtenir un mélange de *p*-(R-oxy)calix[9-20]arènes, à 50% ou plus, alors que le mélange de *p*-(R-oxy)calix[6]arène et de *p*-(R-oxy)calix[7]arène également obtenu est minoritaire ou égal à 50% au plus

Les Inventeurs ont trouvé de manière surprenante que la combinaison d'au moins une base, ladite base étant l'hydroxyde de rubidium, et d'une concentration totale supérieure ou égale à 0,3 équivalent et inférieure ou égale à 0,5 équivalent par rapport au *p*-(benzyloxy)phénol permettait d'obtenir un mélange de *p*-(benzyloxy)calix[9-20]arènes, à 50% ou plus, alors que le mélange de *p*-(benzyloxy)calix[6]arène et de *p*-(benzyloxy)calix[7]arène, également obtenu est minoritaire ou égal à 50% au plus.

Selon un autre aspect, la présente divulgation décrit un procédé de préparation d'un composé consistant en un mélange comprenant un mélange de *p*-(R-oxy)calix[9-20]arènes, *du p*-(R-oxy)calix[6]arène, du *p*-(R-oxy)calix[7]arène et du *p*-(R-oxy)calix[8]arène, comprenant une étape de mise en contact d'hydroxyde de rubidium avec un phénol de formule (I) et du paraformaldéhyde, ladite une base étant à une concentration totale supérieure ou égale à 0,3 équivalent et inférieure ou égale à 0,5 équivalent par rapport audit phénol de formule (I).

Selon un autre aspect, la présente divulgation décrit un procédé de préparation d'un composé consistant en un mélange comprenant un mélange de *p*-(benzyloxy)calix[9-20]arènes, du *p*-(benzyloxy)calix[6] arène, du *p*-(benzyloxy)calix[7]arène et du *p*-(benzyloxy)calix[8]arène, comprenant une étape de mise en contact d'hydroxyde de rubidium avec du *p*-(benzyloxy)phénol et du paraformaldéhyde, ladite une base étant à une concentration totale supérieure ou égale à 0,3 équivalent et inférieure ou égale à 0,5 équivalent par rapport audit *p*-(benzyloxy)phénol.

Selon un autre aspect, la présente divulgation décrit un procédé de préparation d'un composé consistant en un mélange comprenant un mélange de *p*-(R-oxy)calix[9-20]arènes, *du p*-(R-oxy)calix[6]arène, du *p*-(R-oxy)calix[7]arène et du *p*-(R-oxy)calix[8]arène, comprenant une étape de mise en contact d'hydroxyde de rubidium avec un phénol de

formule (I) et du paraformaldéhyde, dans lequel ladite base est à une concentration totale supérieure ou égale à 0,3 équivalent et inférieure ou égale à 0,5 équivalent, pour obtenir un mélange comprenant un mélange de *p*-(R-oxy)calix[9-20]arènes, du *p*-(R-oxy)calix[6]arène, du *p*-(R-oxy)calix[7] arène et du *p*-(R-oxy)calix[8] arène sous forme salifiée. De façon tout à fait inattendue, les Inventeurs ont trouvé que l'utilisation d'hydroxyde de rubidium à une concentration totale supérieure ou égale à 0,3 équivalent et inférieure ou égale à 0,5 équivalent permettait l'obtention d'un mélange comprenant majoritairement, c'est-à-dire en proportion de 50% ou plus en poids, un mélange de *p*-(R-oxy)calix[9-20] arènes sous forme salifiée, ainsi que du *p*-(R-oxy)calix[6]arène, du *p*-(R-oxy)calix[7]arène et du *p*-(R-oxy)calix[8]arène sous forme salifiée de manière minoritaire.

Selon un autre aspect, la présente divulgation décrit un procédé de préparation d'un composé consistant en un mélange comprenant un mélange de *p*-(R-oxy)calix[9-20]arènes, *du p*-(R-oxy)calix[6]arène, du *p*-(R-oxy)calix[7]arène et du *p*-(R-oxy)calix[8] arène, en particulier un mélange de *p*-(benzyloxy)calix[9-20]arènes, du *p*-(benzyloxy)calix[6] arène, du *p*-(benzyloxy)calix[7]arène et du *p*-(benzyloxy)calix[8]arène, comprenant une étape de mise en contact d'hydroxyde de rubidium avec du *p*-(benzyloxy)phénol et du paraformaldéhyde, dans lequel ladite base est à une concentration totale supérieure ou égale à 0,3 équivalent et inférieure ou égale à 0,5 équivalent, pour obtenir un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8]arène sous forme salifiée. De façon tout à fait inattendue, les Inventeurs ont trouvé que l'utilisation d'hydroxyde de rubidium à une concentration totale supérieure ou égale à 0,3 équivalent et inférieure ou égale à 0,5 équivalent permettait l'obtention d'un mélange comprenant majoritairement, c'est-à-dire en proportion de 50% ou plus en poids un mélange de *p*-(benzyloxy)calix[9-20]arènes sous forme salifiée, ainsi que du *p*-(benzyloxy)calix[6]arène, du *p*-(benzyloxy)calix[7]arène et du *p*-(benzyloxy)calix[8]arène sous forme salifiée de manière minoritaire.

Dans un mode de réalisation avantageux, la concentration totale d'hydroxyde de rubidium est de 0,30 équivalent, ou de 0,31 équivalent, ou de 0,32 équivalent, ou de 0,33 équivalent, ou de 0,34 équivalent, ou de 0,35 équivalent, ou de 0,36 équivalent, ou de 0,37 équivalent, ou de 0,38 équivalent, ou de 0,39 équivalent, ou de 0,40 équivalent, ou de 0,41 équivalent, ou de 0,42 équivalent, ou de 0,43 équivalent, ou de 0,44 équivalent, ou de 0,45 équivalent, ou de 0,46 équivalent, ou de 0,47 équivalent, ou de 0,48 équivalent, ou de 0,49 équivalent, ou de 0,50 équivalent.

Dans un mode de réalisation avantageux, la présente divulgation décrit un procédé de préparation d'un composé consistant en un mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, comprenant une étape de mise en contact d'hydroxyde de rubidium avec un phénol de formule (I) et du paraformaldéhyde, dans lequel ladite base est à une concentration totale supérieure ou égale à 0,3 équivalent et inférieure ou égale à 0,5 équivalent, pour obtenir un mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, sous forme salifiée, tel que défini ci-dessus,

comprenant une étape additionnelle de neutralisation du mélange comprenant un mélange de *p*-(R-oxy)calix[9-20] arènes, du *p*-(R-oxy)calix[6]arène, du *p*-(R-oxy)calix[7]arène et du *p*-(R-oxy)calix[8]arène, sous forme neutralisée.

Dans un mode de réalisation avantageux, la présente divulgation décrit un procédé de préparation d'un composé consistant en un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8]arène, comprenant une étape de mise en contact d'hydroxyde de rubidium avec du *p*-(benzyloxy)phénol et du paraformaldéhyde, dans lequel ladite base est à une concentration totale supérieure ou égale à 0,3 équivalent et inférieure ou égale à 0,5 équivalent, pour obtenir un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8]arène, sous forme salifiée, tel que défini ci-dessus,

comprenant une étape additionnelle de neutralisation du mélange comprenant un mélange de *p*-(benzyloxy)calix[9-20] arènes, du *p*-(benzyloxy)calix[6]arène, du *p*-(benzyloxy)calix[7]arène et du *p*-(benzyloxy)calix[8]arène, sous forme neutralisée.

Dans un mode de réalisation avantageux, la présente divulgation décrit un procédé de préparation d'un composé consistant en un mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, comprenant une étape de mise en contact d'hydroxyde de rubidium avec un phénol de formule (I) et du paraformaldéhyde, dans lequel ladite base est à une concentration totale supérieure ou égale à 0,3 équivalent et inférieure ou égale à 0,5 équivalent, pour obtenir un mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, sous forme salifiée, comprenant une étape additionnelle de neutralisation du mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, obtenu sous forme salifiée pour obtenir un mélange comprenant un mélange de *p*-(R-oxy)calix[9-20]arènes, du *p*-(R-oxy)calix[6] arène, du *p*-(R-oxy)calix[7]arène et du *p*-(R-oxy)calix[8] arène, sous forme neutralisée, tel que défini ci-dessus,

comprenant une étape additionnelle de cristallisation du mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8] arène, sous forme neutralisée, dans un mélange de solvants à base de DMSO pour obtenir le *p*-(R-oxy)calix[6]arène sous forme neutralisée.

Dans un mode de réalisation avantageux, la présente divulgation décrit un procédé de préparation d'un composé

consistant en un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8]arène, comprenant une étape de mise en contact d'hydroxyde de rubidium avec du *p*-(benzyloxy)phénol et du paraformaldéhyde, dans lequel ladite base est à une concentration totale supérieure ou égale à 0,3 équivalent et inférieure ou égale à 0,5 équivalent, pour obtenir un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8] arène, sous forme salifiée, comprenant une étape additionnelle de neutralisation du mélange comprenant un mélange de *p*-(benzyloxy)calix[9-20]arènes, du *p*-(benzyloxy)calix[6] arène, du *p*-(benzyloxy)calix[7]arène et du *p*-(benzyloxy)calix[8]arène, obtenu sous forme salifiée, pour obtenir un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8] arène, sous forme neutralisée, tel que défini ci-dessus,
comprenant une étape additionnelle de cristallisation du mélange comprenant un mélange de *p*-(benzyloxy)calix[9-20] arènes, du *p*-(benzyloxy)calix[6]arène, du *p*-(benzyloxy)calix[7]arène et du *p*-(benzyloxy)calix[8] arène, sous forme neutralisée, dans un mélange de solvants à base de DMSO pour obtenir le *p*-(benzyloxy)calix[6]arène sous forme neutralisée.

Les Inventeurs ont également trouvé que le traitement du mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8] arène, sous forme neutralisée dans un mélange de solvants à base de DMSO, permettait d'isoler le mélange de *p*-(R-oxy)calix[9-20]arènes, avec un excellent rendement, supérieur ou égal à 50%, contrairement à l'art antérieur.

Les Inventeurs ont également trouvé que le traitement du mélange comprenant un mélange de *p*-(benzyloxy)calix[9-20]arènes, du *p*-(benzyloxy)calix[6] arène, du *p*-(benzyloxy)calix[7]arène et du *p*-(benzyloxy)calix[8] arène, sous forme neutralisée, dans un mélange de solvants à base de DMSO permettait d'isoler le mélange de *p*-(benzyloxy)calix[9-20]arènes, avec un excellent rendement, supérieur ou égal à 50%, contrairement à l'art antérieur.

Par l'expression « solvant à base de DMSO», il faut comprendre un mélange de solvant comprenant au moins 10% en volume de DMSO.

Le DMSO permet de solubiliser le colloïde formé par le mélange comprenant du *p*-(R-oxy)calix[6]arène, du *p*-(R-oxy)calix[7]arène et du *p*-(R-oxy)calix[8]arène, en particulier le mélange comprenant du *p*-(benzyloxy)calix[6]arène, du *p*-(benzyloxy)calix[7]arène et du *p*-(benzyloxy)calix[8]arène sous forme neutralisée.

Dans un mode de réalisation avantageux, la présente divulgation décrit un procédé de préparation d'un composé consistant en un mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, tel que défini ci-dessus, comprenant les étapes suivantes :

a. mise en contact de l'hydroxyde de rubidium à une concentration totale supérieure ou égale à 0,3 équivalent et inférieure ou égale à 0,5 équivalent, avec un phénol de formule (I) et du formaldéhyde, dans un solvant, puis chauffage pour obtenir un mélange comprenant un mélange de *p*-(R-oxy)calix[9-20]arènes, du *p*-(R-oxy)calix[6] arène, du *p*-(R-oxy)calix[7]arène et du *p*-(R-oxy)calix[8]arène sous forme salifiée,
b. neutralisation dudit mélange comprenant un mélange de *p*-(R-oxy)calix[9-20]arènes, du *p*-(R-oxy)calix[6] arène, du *p*-(R-oxy)calix[7]arène et du *p*-(R-oxy)calix[8]arène sous forme neutralisée,

optionnellement, cristallisation du mélange comprenant du *p*-(R-oxy)calix[6] arène et *du p*-(R-oxy)calix[7]arène sous forme neutralisée dans un mélange de solvants à base de DMSO pour obtenir le mélange de *p*-(R-oxy)calix[9-20]arènes sous forme neutralisée.

Le solvant de l'étape a. est un solvant possédant un point d'ébullition élevé compris d'environ 100 à environ 200°C, en particulier environ 140°C, par exemple le xylène, mais sans être limité à celui-ci.

Le chauffage dépend donc en partie de la nature du solvant et peut être effectué de 30°C à 200°C.

L'étape a. conduit à un mélange comprenant un mélange de *p*-(R-oxy)calix[9-20]arènes, du *p*-(R-oxy)calix[6] arène, du *p*-(R-oxy)calix[7]arène et du *p*-(R-oxy)calix[8]arène, sous forme salifiée.

La neutralisation du mélange est effectuée par un acide, par exemple de l'acide chlorhydrique aqueux ou de l'acide sulfurique aqueux.

Dans un mode de réalisation avantageux, la présente divulgation décrit un procédé de préparation d'un composé consistant en un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8] arène, tel que défini ci-dessus, comprenant les étapes suivantes : mise en contact de l'hydroxyde de rubidium à une concentration totale supérieure ou égale à 0,3 équivalent et inférieure ou égale à 0,5 équivalent, avec du *p*-(benzyloxy)phénol et du formaldéhyde, dans un solvant, puis chauffage pour obtenir un mélange comprenant un mélange de *p*-(benzyloxy)calix[9-20]arènes, du *p*-(benzyloxy)calix[6] arène, du *p*-(benzyloxy)calix[7]arène et du *p*-(benzyloxy)calix[8]arène sous forme salifiée,

a. neutralisation dudit mélange comprenant un mélange de *p*-(benzyloxy)calix[9-20]arènes, du *p*-(benzyloxy)calix[6] arène, du *p*-(benzyloxy)calix[7] arène et du *p*-(benzyloxy)calix[8]arène sous forme neutralisée,
b. optionnellement, cristallisation du mélange comprenant du *p*-(benzyloxy)calix[6] arène et du *p*-(benzyloxy)calix[7]

arène, sous forme neutralisée, dans un mélange de solvants à base de DMSO, pour obtenir le mélange de *p*-(benzyloxy)calix[9-20]arènes sous forme neutralisée.

L'étape a. conduit à un mélange comprenant un mélange de *p*-(benzyloxy)calix[9-20]arènes, du *p*-(benzyloxy)calix[6] arène, du *p*-(benzyloxy)calix[7]arène et du *p*-(benzyloxy)calix[8]arène, sous forme salifiée.
Dans un mode de réalisation avantageux, la présente divulgation décrit un procédé de préparation d'un composé consistant en un mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, tel que défini ci-dessus, comprenant les étapes suivantes :

a. mise en contact de l'hydroxyde de rubidium à une concentration totale supérieure ou égale à 0,3 équivalent et inférieure ou égale à 0,5 équivalent, avec un phénol de formule (I) et du formaldéhyde, dans un solvant, puis chauffage pour obtenir un mélange comprenant un mélange de *p*-(R-oxy)calix[9-20]arènes, du *p*-(R-oxy)calix[6] arène, du *p*-(R-oxy)calix[7]arène et du *p*-(R-oxy)calix[8]arène, sous forme salifiée,
b. neutralisation dudit mélange comprenant un mélange de *p*-(R-oxy)calix[9-20]arènes, du *p*-(R-oxy)calix[6] arène, du *p*-(R-oxy)calix[7]arène et du *p*-(R-oxy)calix[8]arène, sous forme neutralisée,

cristallisation dudit mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, sous forme neutralisée, dans un mélange de solvants à base de DMSO pour obtenir le mélange de *p*-(R-oxy)calix[9-20]arènes sous forme neutralisée.
Dans un mode de réalisation avantageux, la présente divulgation décrit un procédé de préparation d'un composé consistant en un mélange comprenant un mélange de *p*-(benzyloxy)calix[9-20]arènes, du *p*-(benzyloxy)calix[6]arène, du *p*-(benzyloxy)calix[7]arène et du *p*-(benzyloxy)calix[8] arène, tel que défini ci-dessus, comprenant les étapes suivantes :

c. mise en contact de l'hydroxyde de rubidium à une concentration totale supérieure ou égale à 0,3 équivalent et inférieure ou égale à 0,5 équivalent, avec du *p*-(benzyloxy)phénol et du formaldéhyde, dans un solvant, puis chauffage pour obtenir un mélange comprenant un mélange de *p*-(benzyloxy)calix[9-20]arènes, du *p*-(benzyloxy)calix[6] arène, du *p*-(benzyloxy)calix[7]arène et du *p*-(benzyloxy)calix[8]arène, sous forme salifiée,
d. neutralisation dudit mélange comprenant un mélange de *p*-(benzyloxy)calix[9-20]arènes, du *p*-(benzyloxy)calix[6] arène, du *p*-(benzyloxy)calix[7] arène et du *p*-(benzyloxy)calix[8]arène, sous forme neutralisée,
e. cristallisation dudit mélange comprenant un mélange de *p*-(benzyloxy)calix[9-20]arènes, du *p*-(benzyloxy)calix[6] arène, du *p*-(benzyloxy)calix[7] arène et du *p*-(benzyloxy)calix[8] arène, sous forme neutralisée, dans un mélange de solvants à base de DMSO pour obtenir le mélange de *p*-(benzyloxy)calix[9-20]arènes sous forme neutralisée.

Dans un mode de réalisation avantageux, la présente divulgation décrit un procédé de préparation d'un composé consistant en un mélange comprenant un mélange de *p*-(R-oxy)calix[9-20]arènes, du *p*-(R-oxy)calix[6]arène, du *p*-(R-oxy)calix[7]arène et du *p*-(R-oxy)calix[8]arène, comprenant la mise en contact d'hydroxyde de rubidium à une concentration totale supérieure ou égale à 0,3 équivalent et inférieure ou égale à 0,5 équivalent, avec un phénol de formule (I) et du formaldéhyde, tel que défini ci-dessus, dans lequel le chauffage est effectué au reflux du solvant notamment du xylène.
Dans un mode de réalisation avantageux, la présente divulgation décrit un procédé de préparation d'un composé consistant en un mélange comprenant : 1) un mélange de *p*-(benzyloxy)calix[9-20]arènes, 2) du *p*-(benzyloxy)calix[6] arène, 3) du *p*-(benzyloxy)calix[7]arène et du *p*-(benzyloxy)calix[8]arène, comprenant la mise en contact d'hydroxyde de rubidium à une concentration totale supérieure ou égale à 0,3 équivalent et inférieure ou égale à 0,5 équivalent, avec du *p*-(benzyloxy)phénol et du formaldéhyde, tel que défini ci-dessus,
dans lequel le chauffage est effectué au reflux du solvant notamment du xylène.
Dans un mode de réalisation avantageux, la présente divulgation décrit un procédé de préparation d'un composé consistant en un mélange comprenant du *p*-(R-oxy)calix[6]arène et du *p*-(R-oxy)calix[7]arène, ou d'un composé consistant du *p*-(R-oxy)calix[6]arène ou du *p*-(R-oxy)calix[7]arène comprenant la mise en contact avec l'hydroxyde de rubidium à une concentration totale supérieure ou égale à 0,3 équivalent et inférieure ou égale à 0,5 équivalent, avec un phénol de formule (I) et du formaldéhyde, tel que défini ci-dessus,
dans lequel le mélange de solvants de l'étape de cristallisation comprend du DMSO et un solvant polaire tel que l'acétone.
Par solvant polaire, il faut comprendre un solvant possédant un moment dipolaire.
Le DMSO sont mélangés à un solvant polaire tel que l'acétone dans une proportion de 10 % à 90 % en volume. Le précipité obtenu correspond au mélange de *p*-(R-oxy)calix[9-20]arènes purifié.
Dans un mode de réalisation avantageux, la présente divulgation décrit un procédé de préparation d'un composé consistant en un mélange comprenant du *p*-(benzyloxy)calix[6]arène et du *p*-(benzyloxy)calix[7]arène, ou d'un composé consistant du *p*-(benzyloxy)calix[6]arène ou du *p*-(benzyloxy)calix[7]arène comprenant la mise en contact de l'hydroxyde

de rubidium, à une concentration totale supérieure ou égale à 0,3 équivalent et inférieure ou égale à 0,5 équivalent, avec du *p*-(benzyloxy)phénol et du formaldéhyde, tel que défini ci-dessus,
dans lequel le mélange de solvants de l'étape de cristallisation comprend du DMSO et un solvant polaire tel que l'acétone.
Le précipité obtenu correspond au mélange de *p*-(benzyloxy)calix[9-20]arènes purifié.

**Description des figures**

La figure 1 présente la courbe d'étalonnage obtenue avec les échantillons purs de *p*-(benzyloxy)calix[4-16]arènes en RMN diffusionnelle PGSE (Pulse Gradient Spin Echo). Elle permet de valider la masse molaire des *p*-(benzyloxy)calix[4-16]arènes purs déterminée par chromatographie d'exclusion stérique.
Axe des abscisses : ln(M) où M représente le poids moléculaire des *p*-(benzyloxy)calix[4-16]arènes.
Axe des ordonnées : ln(D) où D représente le coefficient d'autodiffusion des molécules qui dépend entre autre de leur volume hydrodynamique déterminé par RMN diffusionnelle PGSE.
Cette courbe permet de relier le coefficient d'autodiffusion à la masse molaire moyenne des calixarènes purs.
La figure 2 présente la chromatographie d'exclusion stérique des échantillons purs *de p*-(benzyloxy)calix[5] arène, *p*-(benzyloxy)calix[7]arène, *p*-(benzyloxy)calix[8]arène, *p*-(benzyloxy)calix[10]arène, *p*-(benzyloxy)calix[12]arène et *p*-(benzyloxy)calix[16]arène.
Cette technique permet de séparer les molécules en fonction de leur volume hydrodynamique et d'estimer la masse molaire d'échantillons purs ou en mélange à partir d'une gamme d'étalons purs. La masse molaire représentative d'un calixarène pur ou d'un mélange de calixarènes est traduite par une « masse molaire au pic » (Mp) et des masses molaires moyennes (Mz).
En abscisse : Volume d'élution : Ve (ml)
En ordonnée : Signal RI (indice de réfraction), correspondant à l'indice de réfraction d'échantillons ou d'étalons en solution sortant de la colonne de chromatographie.
La figure 3 présente le schéma de purification d'un mélange de grands calixarènes obtenu après l'étape de cristallisation dans le mélange acétone-DMSO (10% en volume de DMSO). Les abréviations PX et FX correspondent respectivement au précipité de la fraction X et au filtrat de la fraction X. Les fractions en gras correspondent à des calixarènes purs isolés.
Chaque lettre en italique correspond à une étape de purification, réalisée par ordre chronologique dans les conditions suivantes :

*A* : Le produit brut contenant le mélange de calixarènes est mis sous agitation dans l'acétate d'éthyle puis filtré pour obtenir les fractions P0 et F0.
*B* : Le précipité P0 est chauffé à reflux lh sous argon et sous agitation dans 450 mL de chlorobenzène puis filtré pour obtenir les fractions P1 et F1.
C : Le précipité P1 est placé vigoureusement sous agitation dans 40 mL de THF pendant une nuit, filtré et abondamment rincé au THF pour obtenir les fractions P8 (640 mg, Mp correspond à 20 motifs) et F8.
*D* : F1 est chauffé à reflux sous argon et sous agitation dans 75 mL de chlorobenzène puis refroidit et agité 1h à température ambiante. La solution est placée une nuit à -20°C et filtrée pour obtenir les fractions P2 et F2.
*E* : P2 est placé une nuit sous agitation dans 20mL de dichlorométhane à température ambiante pour obtenir les fractions F3et P3 (40 mg, mélange de calixarènes dont les Mp correspondent à 22, 11 et 8 motifs).
*F* : F3 est dissout dans 40 mL de toluène à reflux puis refroidit à température ambiante. Après 4h sous agitation à température ambiante, la suspension est filtrée pour obtenir F5 (428 mg, mélange de calixarène dont les Mp correspondent à 11 et 17 motifs) et P5 (15 mg).
*G* : F2 est dissout à chaud dans du toluène et déposé sur une colonne de silice. Le mélange de calixarène est élué dans un premier temps dans du dichlorométhane pour obtenir les fractions F2a et F2b, puis dans du tetrahydrofurane pour obtenir la fraction Fc.
*H* : On ajoute 15 mL de DCM sur la fraction F2a et un précipité blanc apparait immédiatement. On laisse reposer 1h sans agitation et on filtre la suspension pour obtenir P4 (40 mg, calix[16]arène pur) et F4.
*I* : F4 est chauffé brièvement à reflux du toluène (20 mL) jusqu'à dissolution totale puis placé 24h à température ambiante sans agitation. On obtient une suspension qui est filtrée pour obtenir les fractions F6 et P6 (30 mg, mélange de grands calixarènes).
*J* : F6 est dissout dans le toluène puis déposé sur une colonne de silice. La chromatographie est effectuée avec un gradient d'éluant 70/30 à 100/0 de dichlorométhane/toluène. On isole, par ordre d'élution, du calix[7]arène (30 mg), du calix[9]arène et un mélange de calix[10+12]arènes (fraction F6c).
*K* : F6c est chauffé brièvement à reflux du toluène (6 mL) jusqu'à dissolution totale puis placée 48h à température

ambiante sans agitation. On obtient une suspension que l'on dilue dans 20 mL de toluène. La suspension est filtrée pour obtenir P7 (44 mg, calix[10]arène pur) et F7.

*L* : F2b est dissoute dans 10 mL de toluène. Un précipité blanc apparait rapidement et la suspension est stockée 5 jours sans agitation. On rajoute 25 mL de toluène et, après 2 semaines sans agitation, la suspension est filtrée pour obtenir P9 (88 mg, calix[10]arène pur) et F9.

*M* : F7 et F9 sont rassemblés, dispersés dans 150 mL d'acétone puis chauffé 1h à reflux du solvant. La suspension est filtrée pour obtenir P10 (688 mg, calix[12]arène pur) et F5 (75 mg, mélange de calix[10]arène et d'autres grands calixarènes).

N : F2c est placé 24h sous agitation dans l'acétone à température ambiante puis filtré pour obtenir un précipité. Ce précipité est soumis à trois cycles de lavages/filtrations dans l'acétone. On obtient P11 (512 mg) et, après assemblage des filtrats, F11 (1.43g).

*O* : P11 est placé sous agitation dans 25 mL de dichlorométhane pendant un weekend à température ambiante. On obtient P12 (50 mg, mélange de calixarènes dont Mp correspond à 21 motifs) et F12 (547 mg, deux populations dont les Mp correspondent à 19 et 30 motifs).

*P* : F8 et P13 sont assemblés et placés 24h sous agitation dans l'acétone pour obtenir P14 (790 mg, deux populations dont les Mp correspondent à 19 et 12 motifs) et F14 (92 mg, deux populations dont les Mp correspondent à 17 et 11 motifs).

*Q* : F11 est mélangé à 50 mL d'acétonitrile. Après chauffage pendant 15 min à reflux du solvant, une sorte d'huile noire visqueuse se dépose sur les parois du ballon et le surnageant est immédiatement séparé. Cette opération est répétée et on obtient P15 (696.5 mg, mélange de calixarènes dont Mp correspond à 34 motifs) et, après assemblage des filtrats, F15 (425,6 mg).

*R* : F15 est placé sous agitation pendant 24h dans 50 mL d'acétonitrile à température ambiante. On obtient F16 (30 mg, calix[13]arène pur) et P16 (401 mg, deux populations dont les Mp correspondent à 18 et 12 motifs).

La figure 4 présente l'ensemble des chromatographies d'exclusion stérique réalisées sur les mélanges de grands calixarènes issus du schéma de purification présenté en figure 3. Chaque chromatogramme inclue le calix[16]arène en référence.

La figure 5 présente les spectres de masse Maldi-Tof des échantillons purs de *p*-(benzyloxy)calix[9] arène, *p*-(benzyloxy)calix[10]arène, *p*-(benzyloxy)calix[12]arène et *p*-(benzyloxy)calix[16]arène. Cette technique permet de déterminer avec précision la masse molaire d'un composé afin de remonter à sa composition chimique.

La figure 6 présente les spectres RMN des échantillons purs de *p*-(benzyloxy)calix[9] arène, *p*-(benzyloxy)calix[10] arène, *p*-(benzyloxy)calix[12]arène, *p*-(benzyloxy)calix[13]arène et *p*-(benzyloxy)calix[16]arène.

La figure 7 présente une analyse RMN du brut réactionnel relatif à l'exemple 2.

La figure 8.1 présente une analyse RMN du brut réactionnel relatif à l'exemple 3.

La figure 8.2 présente une analyse RMN du monosel de sodium purifié relatif à l'exemple 3.

La figure 9 présente une analyse RMN du brut réactionnel relatif à l'exemple 3.1.

La figure 10 présente une analyse RMN du brut réactionnel relatif à l'exemple A.1.

La figure 11 présente une analyse RMN de la solution orange vif décrite à l'exemple A.2.

La figure 12 présente le chromatogramme (SEC) du macroamorceur obtenu à partir du calix[12]arène (exemple C).

La figure 13 présente le chromatogramme (SEC) des polymères en étoile obtenus à partir du calix[12]arène, lesdits polymères différant par la durée du procédé ATRP

La figure 14 présente la caractérisation des structures des polymères en étoile, en exprimant log Rh où Rh correspond au rayon hydrodynamique du polymère étudié, en fonction de log Mw où Mw est la masse molaire du polymère.

**EXEMPLES :**

**Exemple 1 : Préparation et purification d'un mélange de *p*-(benzyloxy)calix[9-20]arènes avec RbOH (0,6 eq)**

Dans un ballon de 2L muni d'un agitateur magnétique et d'un Dean-Stark, on prépare une suspension de 50g de 4-(benzyloxy)phénol (0,25 équivalent), 15g de paraformaldehyde (0,5 équivalent) dans 670ml de xylène (mélange d'isomères).

Cette suspension est mise en chauffe. A 90°C, on ajoute rapidement 18 ml (0,6 équivalent par rapport au phénol) d'une solution de RbOH à 50% en poids dans l'eau.

La solution est portée à reflux pendant 6h. On laisse revenir à température ambiante.

Une analyse RMN proton montre que le milieu réactionnel est constitué à 60% de grands calixarènes, et contient une proportion de 15% de p-(benzyloxy)calix[5]arène.

Les différents constituants de ce mélange peuvent ensuite être purifiés selon la séquence décrite à la figure 3.

**Exemple 2 : Préparation d'un mélange de *p*-(benzyloxy)calix[9-20]arènes avec CsOH (0,6 equivalent par rapport au phénol)**

Dans un ballon de 21 muni d'un agitateur magnétique et d'un dean-Stark, on prépare une suspension de 50g de 4-(benzyloxy)phénol (0,25 équivalent) et 15g de paraformaldehyde (0,5 équivalent) dans 670ml de xylène (mélange d'isomères).
Cette suspension est mise en chauffe. A 90°C, on ajoute rapidement 27 ml (0,6 équivalent par rapport au phénol) d'une solution de CsOH à 50% en poids dans l'eau.
La solution est portée à reflux pendant 6h. On laisse revenir à température ambiante.
Une analyse RMN (figure 7) du brut réactionnel montre qu'il est constitué à 60% de grands calixarènes.
La suspension est neutralisée avec 500ml de THF contenant 30ml d'une solution d'HCl à 37% sous forte agitation.
La suspension fluide résultante est filtrée, et le filtrat évaporé à sec. Le solide obtenu est lavé avec 500ml d'acétonitile et filtré (pour éliminer les espèces les plus polaires ou les plus solubles, telles que les restes d'oligomères linéaires, les calix[5]arènes et l'homooxacalixarène).
Après filtration et séchage sous pompe à vide, on récupère 32 g d'un solide brun, qui est dissout à chaud dans un mélange de 75ml de DMSO et 750ml d'acétone.
La solution limpide orange foncé résultante est laissée au réfrigérateur pendant deux jours, durée pendant laquelle se forme un précipité microcristallin (20g).
Une étude RMN montre que ce précipité est constitué exclusivement de grands calixarènes.

**Exemple 3 (exemple de référence) : Préparation d'un mélange de *p*-(benzyloxy)calix[9-20]arènes et de *p*-benzyloxycalix[7]arène sous forme de sel de sodium ou potassium avec la soude ou la potasse (0,6 eq)**

Dans un ballon de 21 muni d'un agitateur magnétique et d'un dean-Stark, on prépare une suspension de 50g de 4-(benzyloxy)phénol (0,25 équivalent) et 15g de paraformaldehyde (0,5 équivalent) dans 670ml de xylène (mélange d'isomères).
Cette suspension est mise en chauffe. A 90°C, on ajoute rapidement 6,5g de NaOH dans 10 ml d'eau (0,6 équivalent par rapport au phénol). La solution est portée à reflux pendant 8h. On laisse revenir à température ambiante.
Une anlyse RMN (figure 8.1) du brut réactionnel montre qu'il est constitué d'un mélange *de p*-(benzyloxy)calix[7] et [8] arènes ainsi que du dimère non réagi, accompagnés d'une proportion beaucoup plus faible de grands calixarènes.
Une filtration de ce produit conduit à l'obtention du *p*-(benzyloxy)calix[7]arène sous forme de monosel de sodium (figure 8.2).

**Exemple 3.1 : Obtention du dimère**

Dans un ballon de 21 muni d'un agitateur magnétique et d'un dean-Stark, on prépare une suspension de 50g de 4-(benzyloxy)phénol (0,25 équivalent) et 15g de paraformaldehyde (0,5 équivalent) dans 670ml de xylène (mélange d'isomères).
Cette suspension est mise en chauffe. A 90°C, on ajoute rapidement 6,5g de NaOH dans 10 ml d'eau (0,6 équivalent par rapport au phénol). La solution est portée à reflux pendant lh. On laisse revenir à température ambiante.
Une anlyse RMN (figure 9) du brut réactionnel montre qu'il est constitué quasi exclusivement du dimère :
Ce produit est récupéré par simple filtration. Rendement : 80%.

**Exemple 4 : Préparation d'un mélange de *p*-(méthoxy)calix[9-20]arènes avec CsOH (0,6 eq)**

Dans un ballon de 2L muni d'un agitateur magnétique et d'un Dean-Stark, on prépare une suspension de 31g de 4-(methoxy)phénol (0,25 équivalent), 15g de paraformaldehyde (0,5 équivalent) dans 670ml de xylène (mélange d'isomères).
Cette suspension est mise en chauffe. A 90°C, on ajoute rapidement 15 ml (0,6 équivalent par rapport au phénol) d'une solution de CsOH à 50% en poids dans l'eau.
La solution est portée à reflux pendant 6h. On laisse revenir à température ambiante.
Une analyse RMN proton montre que le milieu réactionnel est constitué à 50% de grands calixarènes.

**Exemple** 5 **: Préparation d'un mélange de *p*-(octyloxy)calix[9-20]arènes avec CsOH (0,6 eq)**

Dans un ballon de 2L muni d'un agitateur magnétique et d'un Dean-Stark, on prépare une suspension de 56g de 4-(octyloxy)phénol (0,25 équivalent), 15g de paraformaldehyde (0,5 équivalent) dans 670ml de xylène (mélange d'isomères).

Cette suspension est mise en chauffe. A 90°C, on ajoute rapidement 26,5 ml (0,6 équivalent par rapport au phénol) d'une solution de CsOH à 50% en poids dans l'eau.
La solution est portée à reflux pendant 6h. On laisse revenir à température ambiante.
Une analyse RMN proton montre que le milieu réactionnel est constitué à 70% de grands calixarènes.

**Exemple 6 : Utilisation de grands calixarènes pour la synthèse de nanomatériaux : polymères en étoile**

La procédure utilisée est en deux étapes. Au cours de la première, les groupements phénoliques du calixarène sont acylés par le bromure de bromopropionyle. Ces groupements constituent des amorceurs de polymérisation, utilisés au cours d'une deuxième étape pour la croissance de chaînes polystyrène.

Synthèse de l'amorceur pour le calix[12]arène.

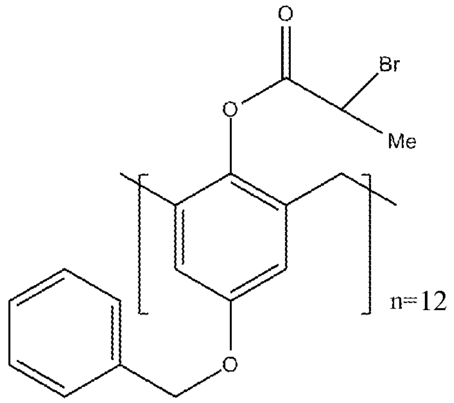

Dans un tube de Schlenk, on introduit le grand calixarène (500mg, 0,0002mol). Sous argon, on ajoute alors 5 mL de DMF et 1,5 mL de $Et_3N$. Le système est plongé dans un bain de glace. On introduit ensuite 300 $\mu$L de bromure de bromopropionyle. La solution est agitée pendant lh. Une précipitation est réalisée dans 50 mL MeOH. On filtre sous vide. L'amorceur est solubilisé dans quelques millilitres de THF. Puis on réalise une deuxième précipitation avec 50mL de MeOH. On filtre sous vide. Le produit est séché au dessiccateur.
Rendement = 60%.
RMN $^1H$ (350MHz, $CDCl_3$) $\delta_{ppm}$: 7,1-7,6 (m, 60H, -$OCH_2$-*Ar*), 6,8-6,4 (m, 24H, Ar), 5,1-4,7 (m, 24H, -O$CH_2$-Ar-), 4-3,5 (m, 24H, -Ar(phenol)-$CH_2$-), 3-1,5 (m, 24H, $CH_3$-*CH*(COR)-Br), 2-1,5 (m, 36H, $CH_3$-CH(COR)-Br).
Ce macroamorceur est également caractérisé par chromatographie d'exclusion stérique (figure 12).

**Synthèse du polymère en étoile par polymérisation radicalaire par transfert d'atomes (ATRP)**

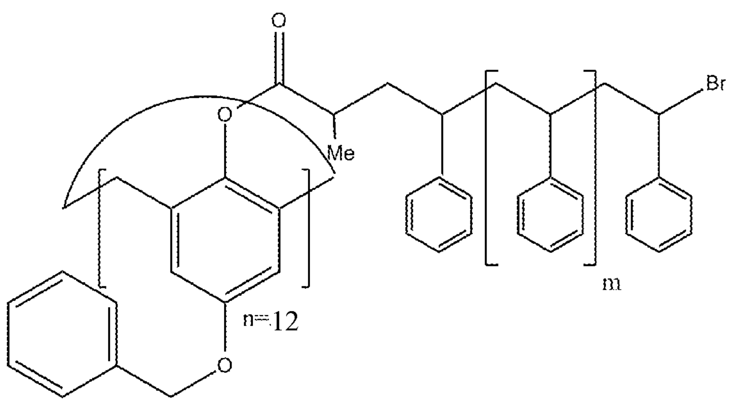

Dans un shlenk sous argon, on introduit CuBr(25mg, 0,00017mol), la bipyridine (80mg, 0,00052mol), l'amorceur

précédemment synthétisé (60mg, 0,00017mol) et 2mL de styrène. Le milieu réactionnel est dégazé par 3 cycles de congélation-décongélation. Le tube de schlenk est plongé dans un bain d'huile chauffé à 100°C pendant un temps donné. Le mélange est solubilisé dans du THF. Le mélange est filtré sur une colonne d'$Al_2O_3$ basique. Le polymère est précipité dans du MeOH (avec un rapport $=\frac{THF}{MeOH}=10$ ), puis filtré sous vide et rincé avec du MeOH. Le produit est séché au dessiccateur.
Rendement = 50%.
RMN $^1H$ (360MHz, $CDCl_3$) $\delta_{ppm}$: 7,1-7,6 (m, 5H, -$OCH_2$-*Ar*),7,2-6,9 (m, 5H, $Ar_{styrène}$), 7,2-6,9 (m, 1H, -$CH_2$-*CH*(Ar)-$CH_2$-), 6,8-6,4 (m, 2H, Ar), 2,1-1,6 (m, 2H, -CH-$CH_2$-CH-), 2,1-1,6 (m, 1H, $HO_2C$-*CH*($CH_3$)-$CH_2$-), 1,6-1,3 (m, 3H, $CH_3$-CH-).

Les polymères en étoile obtenus, qui diffèrent par la durée de l'ATRP, sont également caractérisés par chromatographie d'exclusion stérique (figure 13).

**Etude de la structure des polymères en solution.**

Pour étudier le comportement desdits polymères en étoile en solution on peut utiliser une loi d'échelle :

$$Rh = KM^{\alpha}$$

où Rh correspond au rayon hydrodynamique, M correspond à la masse molaire ; K et $\alpha$ sont des coefficients. Les termes K et $\alpha$ sont des coefficients avec le polymère.

$$\log Rh = \log K + \alpha \log M$$

Le terme $\alpha$ nous donne des informations sur le comportement des polymères en solution:

Si $\alpha$ tend vers 1 alors le polymère se comporte comme un polymère linéaire.
Si $\alpha$ tend vers 0,5 alors le polymère se comporte comme une pelote statistique.
Si $\alpha$ tend vers 0 alors le polymère se comporte comme une sphère dure.

Le tracé du rayon hydrodynamique (mesuré par diffusion de la lumière) en fonction de la masse molaire (obtenue en mesurant la masse molaire des branches de polystyrène (après « décrochage » du cœur calixarène central par saponification) permet donc d'apporter des informations sur la structure de ces nanoobjets en solution (figure 14).

Dans le cas des polymères en étoile dérivé du calix[12]arène, le comportement est celui d'un oligomère linéaire, ils forment donc en solution (THF) des nanoobjets bien définis, potentiellement intéressants pour l'encapsulation et la vectorisation.

**EXEMPLES COMPARATIFS** : **Utilisation d'une base en proportion inférieure ou égale à 0,5 eq avec le *p*-benzyloxyphénol**

**Exemple A : Obtention d'un composé consistant en du *p*-(benzyloxy)calix[6]arène ou dup -(benzyloxy)calix[7] arène, sous forme de sel de césium ou neutralisée, ou d'un mélange comprenant du *p* -(benzyloxy)calix[6]arène et du *p* -(benzyloxy)calix[7]arène avec de l'hydroxyde de césium.**

**Exemple A.1 : concentration en hydroxyde de césium de 0,15 équivalent**

Une suspension de 50,6g de *p*-(benzyloxy)phénol (0,254 mol), 20g (0,667 mol) de paraformaldéhyde (point de fusion : 135°C) dans 700 ml de xylène est mise sous argon dans un ballon tricol de 21 équipé d'un agitateur mécanique et d'un récupérateur d'eau type « Dean-Stark ».

La suspension est mise à chauffer sous agitation. Lorsque la température atteint 90°C, on ajoute rapidement à l'aide d'une seringue (et sous balayage d'argon) 7,4 ml (0,0425 mol) d'une solution de CsOH à 50% (en poids) dans l'eau.

La suspension est laissée à reflux pendant 6h, période au cours de laquelle on observe la formation d'un volumineux précipité blanc.
Une analyse de ce brut réactionnel (figure 10) montre qu'il est constitué très majoritairement du sel de césium du

*p*-(benzyloxy)calix[7]arène.

Ce précipité est filtré, lavé au xylène, puis au pentane. m=35g, rendement en *p*-(benzyloxy)calix[7]arène (sous forme de monosel de césium) : 58%.

Les caractéristiques spectroscopiques de ce précipité correspondent à celles d'un monoanion du *p*-(benzyloxy)calix[7] arène.

RMN $^1H$ (DMSO-$d_6$) : (déplacements chimiques, ppm) 7,60-7,20 (multiplet, aromatiques) ; 6,72 (singulet fin, hydroquinone) ; 4,93 (singulet fin, benzyliques) ; 3,71 (singulet fin, méthylènes intracycliques).

Spectrométrie de masse (MALDI, matrice DHB) : m/z=1617,41 $(M+Cs)^+$.

Un échantillon de 1g de ce précipité est mis en suspension dans 5ml de dichlorométhane, puis neutralisé avec une solution aqueuse concentrée d'HCl, sous forte agitation pendant 24h.

La phase organique est récupérée, puis évaporée à sec au rotovapor.

On récupère 0,91g d'un solide blanc, dont les caractéristiques spectroscopiques sont en parfait accord avec le *p*-(benzyloxy)calix[7]arène neutre.

RMN $^1H$ (DMSO-$d_6$) : (déplacements chimiques, ppm) 7,30 (multiplet large, aromatiques) ; 6,62 (singulet fin, hydroquinone) ; 4,84 (singulet fin, benzyliques) ; 3,74 (singulet fin, méthylènes intracycliques).

Spectrométrie de masse (MALDI, matrice DHB) : m/z=1507,65 $(M+Na)^+$.

La perte de masse observée (90mg) est tout à fait en accord avec celle attendue pour la neutralisation d'un monosel de césium : *p*-(benzyloxy)calix[7]arène)-$Cs^+$ → *p*-(benzyloxy)calix[7] arène.

### Exemple A.2 : concentration en hydroxyde de césium de 0,3 équivalent.

Une suspension de 50,6g de *p*-(benzyloxy)phénol (0,254 mol), 20g (0,667 mol) de paraformaldehyde (point de fusion : 135°C) dans 700 ml de xylène est mise sous argon dans un ballon tricol de 21 équipé d'un agitateur mécanique et d'un récupérateur d'eau type « Dean-Stark ».

La suspension est mise à chauffer sous agitation. Lorsque la température atteint 90°C, on ajoute rapidement à l'aide d'une seringue (et sous balayage d'argon) 14,8 ml (0,085 mol) d'une solution de CsOH à 50% (en poids) dans l'eau.

La suspension est laissée à reflux pendant 5h30. On obtient alors une solution parfaitement limpide orange vif. Une analyse RMN (figure 11) de cette solution montre qu'elle est comporte en grande majorité du *p*-(benzyloxy)calix[6] arène, accompagné d'un faible pourcentage de grands calixarènes.

On ajoute alors 100ml d'une solution de HCl à 37% dans l'eau, et l'on observe la formation d'un précipité. La suspension est laissée sous forte agitation pendant deux jours, puis évaporée à sec à l'évaporateur rotatif.

Après lavage avec 500ml d'eau (élimination des sels et de l'excès d'HCl), le solide est dissout à chaud (130°C) dans 200ml de DMSO. On obtient alors une solution limpide noire, à laquelle sont ajoutés à chaud 21 d'acétone.

Après retour à température ambiante, cette solution limpide est abandonnée une semaine, pendant laquelle un précipité cristallin de *p*-(benzyloxy)calix[6]arène (18 g) se dépose sur les parois du ballon.

Le filtrat est évaporé au rotovapor, puis au pistolet chauffant jusqu'à obtention d'un solide noir. Ce solide est lavé à l'acétone, ce qui conduit à la récupération d'un deuxième lot de *p*-(benzyloxy)calix[6]arène (10 g). Total : 28g, rendement 51%.

RMN $^1H$ (DMSO-$d_6$) : (déplacements chimiques, ppm) 7,30 (multiplet large, aromatiques) ; 6,62 (singulet fin, hydroquinone) ; 4,79 (singulet fin, benzyliques) ; 3,72 (singulet fin, méthylènes intracycliques).

Spectrométrie de masse (MALDI, matrice DHB) : m/z=1295,49 $(M+Na)^+$.

### Exemple B : Obtention d'un composé consistant en du *p*-(benzyloxy)calix[6] arène ou du *p*-(benzyloxy)calix[7] arène sous forme neutralisée ou du *p*-(benzyloxy)calix[8]arène ou d'un mélange comprenant du *p*-(benzyloxy)calix[6]arène, du *p*-(benzyloxy)calix[7]arène et du *p*-(benzyloxy)calix[8]arène avec de l'hydroxyde de sodium ou de potassium.

### Exemple B.1 : concentration en hydroxyde de sodium ou de potassium de 0,15 équivalent.

Une suspension de 34,5g de *p*-(benzyloxy)phénol (0,173 mol), 20g (0,667 mol) de paraformaldehyde (point de fusion : 135°C) dans 450ml de xylène est mise sous argon dans un ballon tricol de 11 équipé d'un agitateur mécanique et d'un récupérateur d'eau type « Dean-Stark ».

La suspension est mise à chauffer sous agitation. Lorsque la température atteint 95°C, on ajoute rapidement à l'aide d'une seringue (et sous balayage d'argon) une solution de 1,43g (0,03 mol) de KOH dans 6ml d'eau Millipore. On observe l'apparition immédiate d'une coloration jaune. Le milieu réactionnel est porté au reflux pendant 3h30, période pendant laquelle on observe la formation d'un abondant précipité blanc et d'une solution orange vif.

Après retour à température ambiante, le précipité est récupéré par filtration, lavé avec 100ml de xylène et 300ml de pentane.

L'analyse de ce précipité indique qu'il est constitué de *p*-(benzyloxy)calix[7]arène pur. M = 20g, rendement 54%.

Caractérisations :

RMN $^1H$ (DMSO-$d_6$) : (déplacements chimiques, ppm) 7,30 (multiplet large, aromatiques) ; 6,62 (singulet fin, hydroquinone) ; 4,84 (singulet fin, benzyliques) ; 3,74 (singulet fin, méthylènes intracycliques).
Spectrométrie de masse (MALDI, matrice DHB) : m/z=1524,62 $(M+K)^+$.

**Exemple B.2 : concentration en hydroxyde de sodium ou de potassium de 0,3 équivalent.**

Une suspension de 51,5g de *p*-(benzyloxy)phénol (0,258 mol), 20g (0,667 mol) de paraformaldehyde (point de fusion : 135°C) dans 700 ml de xylène est mise sous argon dans un ballon tricol de 21 équipé d'un agitateur mécanique et d'un récupérateur d'eau type « Dean-Stark ».
La suspension est mise à chauffer sous agitation. Lorsque la température atteint 90°C, on ajoute rapidement à l'aide d'une seringue (et sous balayage d'argon) 3,056g de NaOH (0,0764 mol) dans 10 ml d'eau.
La suspension est laissée à reflux pendant 4h30, période au bout de laquelle le milieu réactionnel prend en masse.
Après retour à température ambiante, le milieu réactionnel est neutralisé par 500ml d'une solution d'HCl 2M, sous très forte agitation.
L'émulsion résultante est évaporée à sec et lavée avec 500ml d'eau (élimination des sels de sodium).
Le solide orangé résultant est lavé avec 500ml de THF et le précipité blanc résultant est filtré, ce qui conduit à la récupération de 20g de *p*-(benzyloxy)calix[8] arène pur. Rendement : 36,6%.
RMN $^1H$ (DMSO-$d_6$) : (déplacements chimiques, ppm) 7,30 (multiplet large, aromatiques) ; 6,58 (singulet fin, hydroquinone) ; 4,80 (singulet fin, benzyliques) ; 3,77 (singulet fin, méthylènes intracycliques).
Spectrométrie de masse (MALDI, matrice DHB) : m/z=1719,62 $(M+Na)^+$.
Le filtrat correspondant est évaporé à sec et dissout à chaud dans 45 ml de DMSO, ce qui conduit à la formation d'une solution homogène noire. 11 de toluène y est ajouté, et la solution limpide orange foncée est placée au congélateur (-23°C) pendant 1 semaine, ce qui conduit à la formation d'un précipité microcristallin. Ce précipité est filtré, et son analyse par RMN $^1H$ montre qu'il s'agit de*p*-(benzyloxy)calix[6] arène pur ; m=6,3g, 11%.
RMN $^1H$ (DMSO-$d_6$) : (déplacements chimiques, ppm) 7,30 (multiplet large, aromatiques) ; 6,62 (singulet fin, hydroquinone) ; 4,79 (singulet fin, benzyliques) ; 3,72 (singulet fin, méthylènes intracycliques).
Spectrométrie de masse (MALDI, matrice DHB) : m/z=1295,49 $(M+Na)^+$.
Le filtrat DMSO/toluène correspondant est évaporé jusqu'à obtention d'un liquide orangé. Après ajout de 11 de méthanol et filtration, on récupère 28,5g de *p*-(benzyloxy)calix[7] arène pur. Rendement : 52%.
RMN $^1H$ (DMSO D6): (déplacements chimiques, ppm) 7,30 (multiplet large, aromatiques) ; 6,62 (singulet fin, hydroquinone) ; 4,84 (singulet fin, benzyliques) ; 3,74 (singulet fin, méthylènes intracycliques).
Spectrométrie de masse (MALDI, matrice DHB) : m/z=1524,62 $(M+K)^+$.

## Revendications

**1.** Utilisation d'au moins une base, choisie parmi l'hydroxyde de rubidium ou l'hydroxyde de césium, avec au moins un phénol substitué en position 4 de formule (I) suivante :

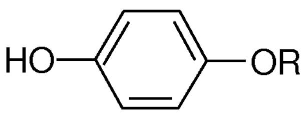

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire,
- un PEG-1 à 10 ramifié ou linéaire, dont le groupe terminal -OH est alkylé par un alkyle $C_1$-$C_{20}$ ramifié ou linéaire,

une source de formaldéhyde et un solvant organique,
le mélange de ladite base, dudit phénol, de ladite source de formaldéhyde et dudit solvant organique constituant un milieu réactionnel,
ledit milieu réactionnel étant chauffé à une température comprise de 50 °C à 250 °C durant un temps compris de 4 à 24h et ladite base étant à une concentration totale comprise de plus de 0,5 équivalent à 1,5 équivalent, par rapport audit au moins un phénol substitué en position 4 de formule (I),
pour la mise en œuvre d'une réaction de préparation d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou d'un mélange d'au moins deux desdits *p*-(R-oxy)calixarènes dans lequel lesdits au moins deux *p*-(R-oxy)calixarènes sont présents dans ledit mélange à raison d'au moins 5% molaire chacun.

**2.** Utilisation selon la revendication 1, dans laquelle ladite base est à une concentration totale comprise de plus de 0,5 équivalent à 1,2 équivalent, ou est égale à 0,6 équivalent, par rapport audit au moins un phénol substitué en position 4 de formule (I).

**3.** Procédé de préparation

- d'un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13]arène, un *p*-(R-oxy)calix[14]arène, un *p*-(R-oxy)calix[15]arène, un *p*-(R-oxy)calix[16]arène, un *p*-(R-oxy)calix[17]arène, un *p*-(R-oxy)calix[18]arène, un *p*-(R-oxy)calix[19]arène, un *p*-(R-oxy)calix[20]arène, ou
- d'un mélange d'au moins deux desdits *p*-(R-oxy)calixarènes dans lequel lesdits au moins deux *p*-(R-oxy)calixarènes sont présents dans ledit mélange à raison d'au moins 5% molaire chacun,

comprenant une étape de mise en contact d'une base, choisie parmi l'hydroxyde de rubidium ou l'hydroxyde de césium, avec au moins un phénol substitué en position 4 de formule (I) suivante :

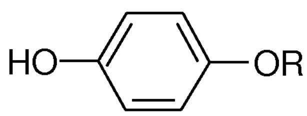

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire,
- un PEG-1 à 10 ramifié ou linéaire, dont le groupe terminal -OH est alkylé par un alkyle $C_1$-$C_{20}$ ramifié ou linéaire,

une source de formaldéhyde et un solvant organique,
le mélange de ladite base, dudit phénol, de ladite source de formaldéhyde et dudit solvant organique constituant un milieu réactionnel,
ledit milieu réactionnel étant chauffé à une température comprise de 50 °C à 250 °C durant un temps compris de 4 à 24h et ladite base étant à une concentration totale comprise de plus de 0,5 équivalent à 1,5 équivalent, par rapport audit au moins un phénol substitué en position 4 de formule (I).

**4.** Procédé selon la revendication 3, dans lequel ladite base est à une concentration totale comprise de plus de 0,5 équivalent à 1,2 équivalent, ou est égale à 0,6 équivalent, par rapport audit au moins un phénol substitué en position 4 de formule (I).

**5.** Procédé selon la revendication 3 ou 4, dans lequel ledit phénol substitué en position 4 de formule (I) est choisi parmi le 4-benzyloxyphénol ou le 4-octyloxyphénol.

6. Procédé selon l'une des revendications 3 à 5, dans lequel la source de formaldéhyde est du paraformaldéhyde et le solvant organique est du xylène.

7. Procédé selon l'une des revendications 3 à 6, comprenant les étapes suivantes :

a. mise en contact de l'hydroxyde de césium et/ou de rubidium, avec au moins un phénol substitué en position 4 de formule (I), ladite base étant à une concentration totale comprise de plus de 0,5 équivalent à 1,5 équivalent, par rapport audit au moins un phénol substitué en position 4 de formule (I), une source de formaldéhyde, dans un solvant organique puis chauffage en éliminant optionnellement ladite eau formée du milieu réactionnel, pour obtenir :

un *p*-(R-oxy)calixarène choisi parmi un *p*-(R-oxy)calix[9]arène, un *p*-(R-oxy)calix[10]arène, un *p*-(R-oxy)calix[11]arène, un *p*-(R-oxy)calix[12]arène, un *p*-(R-oxy)calix[13] arène, un *p*-(R-oxy)calix[14]arène, un *p*-(R-oxy)calix[15]arène, un *p*-(R-oxy)calix[16] arène, un *p*-(R-oxy)calix[17] arène, un *p*-(R-oxy)calix[18]arène, un *p*-(R-oxy)calix[19]arène, un *p*-(R-oxy)calix[20]arène, ou, un mélange d'au moins deux desdits *p*-(R-oxy)calix[9-20]arènes dans lequel lesdits au moins deux *p*-(R-oxy)calixarènes sont présents dans ledit mélange à raison d'au moins 5% molaire chacun,
sous forme salifiée dans le milieu réactionnel final,

b. neutralisation de ladite forme salifiée dudit *p*-(R-oxy)calixarène ou dudit mélange pour obtenir ledit *p*-(R-oxy)calixarène ou ledit mélange sous forme neutralisée,

et comprenant optionnellement une étape additionnelle de purification dudit *p*-(R-oxy)calixarène ou dudit mélange sous forme neutralisée, pour obtenir ledit *p*-(R-oxy)calixarène ou ledit mélange sous forme neutralisée purifié.

8. Procédé selon la revendication 7, dans lequel ladite purification se fait par par cristallisation dans un mélange de solvants à base de DMSO.

## Patentansprüche

1. Verwendung mindestens einer Base, welche aus Rubidiumhydroxid oder Cesiumhydroxid ausgewählt ist, mit mindestens einem Phenol, das substituiert ist in Stellung 4 der folgenden Formel (I):

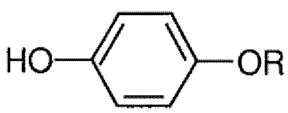
(I)

wobei R ausgewählt ist aus:

- einer Benzylgruppe, die gegebenenfalls in ortho und/oder para und/oder meta durch einen oder mehrere Substituenten substituiert ist, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkylgruppe,
- einem verzweigten oder linearen PEG-1 bis 10, dessen Endgruppe -OH durch ein verzweigtes oder lineares $C_1$-$C_{20}$-Alkyl alkyliert ist,

einer Quelle von Formaldehyd und einem organischen Lösungsmittel,
wobei die Mischung der genannten Base, des genannten Phenols, der genannten Quelle von Formaldehyd und des genannten organischen Lösungsmittels ein Reaktionsmedium darstellt,
wobei das genannte Reaktionsmedium auf eine Temperatur von 50 °C bis 250 °C während einer Zeit von 4 bis 24 h erhitzt wird, und die genannte Base eine Gesamtkonzentration von mehr als 0,5 Äquivalenten bis 1,5 Äquivalenten aufweist, bezogen auf das genannte mindestens eine Phenol, das in Stellung 4 der Formel (I) substituiert ist,
zur Durchführung einer Reaktion zur Herstellung eines *p*-(R-Oxy)-calixarens, ausgewählt aus einem *p*-(R-Oxy)-calix[9]aren, einem *p*-(R-Oxy)-calix[10]aren, einem *p*-(R-Oxy)-calix[11]aren, einem *p*-(R-Oxy)-calix[12]aren, einem

*p*-(R-Oxy)-calix[13]aren, einem *p*-(R-Oxy)-calix[14]aren, einem *p*-(R-Oxy)-calix[15]aren, einem *p*-(R-Oxy)-calix[16]aren, einem *p*-(R-Oxy)-calix[17]aren, einem *p*-(R-Oxy)-calix[18]aren, einem *p*-(R-Oxy)-calix[19]aren, einem *p*-(R-Oxy)-calix[20]aren oder einer Mischung von mindestens zwei der genannten *p*-(R-Oxy)-calixarene, wobei die genannten mindestens zwei p-(R-Oxy)-calixarene in der genannten Mischung in einer Menge von jeweils mindestens 5 Mol-% vorhanden sind.

**2.** Verwendung nach Anspruch 1, wobei die genannte Base eine Gesamtkonzentration von mehr als 0,5 Äquivalenten bis 1,2 Äquivalenten oder gleich 0,6 Äquivalenten aufweist, bezogen auf das genannte mindestens eine Phenol, das in Stellung 4 der Formel (I) substituiert ist.

**3.** Verfahren zur Herstellung von:

- einem *p*-(R-Oxy)-calixaren, ausgewählt aus einem *p*-(R-Oxy)-calix[9]aren, einem *p*-(R-Oxy)-calix[10]aren, einem *p*-(R-Oxy)-calix[11]aren, einem *p*-(R-Oxy)-calix[12]aren, einem p-(R-Oxy)-calix[13]aren, einem *p*-(R-Oxy)-calix[14]aren, einem *p*-(R-Oxy)-calix[15]aren, einem *p*-(R-Oxy)-calix[16]aren, einem *p*-(R-Oxy)-calix[17]aren, einem *p*-(R-Oxy)-calix[18]aren, einem *p*-(R-Oxy)-calix[19]aren, einem *p*-(R-Oxy)-calix[20]aren oder
- einer Mischung von mindestens zwei der genannten *p*-(R-Oxy)-calixarene, wobei die mindestens zwei genannten *p*-(R-Oxy)-calixarene in der genannten Mischung in einer Menge von jeweils mindestens 5 Mol-% vorhanden sind,

umfassend einen Schritt des Inberührungbringens einer Base, ausgewählt aus Rubidiumhydroxid oder Cesiumhydroxid, mit mindestens einem Phenol, das substituiert ist in Stellung 4 der folgenden Formel (I) :

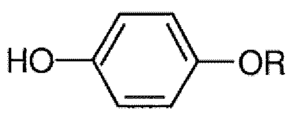

(I)

wobei R ausgewählt ist aus:

- einer Benzylgruppe, die gegebenenfalls in ortho und/oder para und/oder meta durch einen oder mehrere Substituenten substituiert ist, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkylgruppe,
- einem verzweigten oder linearen PEG-1 bis 10, dessen Endgruppe -OH durch ein verzweigtes oder lineares $C_1$-$C_{20}$-Alkyl alkyliert ist,

einer Quelle von Formaldehyd und eines organischen Lösungsmittels,
wobei die Mischung der genannten Base, des genannten Phenols, der genannten Quelle von Formaldehyd und des genannten organischen Lösungsmittels ein Reaktionsmedium darstellt,
wobei das genannte Reaktionsmedium auf eine Temperatur von 50 °C bis 250 °C während einer Zeit von 4 bis 24 h erhitzt wird, und die genannte Base eine Gesamtkonzentration von mehr als 0,5 Äquivalenten bis 1,5 Äquivalenten aufweist, bezogen auf das genannte mindestens eine Phenol, das in Stellung 4 der Formel (I) substituiert ist.

**4.** Verfahren nach Anspruch 3, wobei die genannte Base eine Gesamtkonzentration von mehr als 0,5 Äquivalenten bis 1,2 Äquivalenten oder gleich 0,6 Äquivalenten aufweist, bezogen auf das genannte mindestens eine Phenol, das in Stellung 4 der Formel (I) substituiert ist.

**5.** Verfahren nach Anspruch 3 oder 4, wobei das genannte Phenol, das in Stellung 4 der Formel (I) substituiert ist, aus 4-Benzyloxyphenol oder 4-Octyloxyphenol ausgewählt wird.

**6.** Verfahren nach einem der Ansprüche 3 bis 5, wobei die Quelle von Formaldehyd Paraformaldehyd ist, und das organische Lösungsmittel Xylol ist.

**7.** Verfahren nach einem der Ansprüche 3 bis 6, umfassend die folgenden Schritte:

a. Inberührungbringen von Cesium- und/oder Rubidiumhydroxid mit mindestens einem Phenol, das in Stellung

4 der Formel (I) substituiert ist, wobei die genannte Base eine Gesamtkonzentration von mehr als 0,5 Äquivalenten bis 1,5 Äquivalenten aufweist, bezogen auf das genannte mindestens eine Phenol, das in Stellung 4 der Formel (I) substituiert ist, einer Quelle von Formaldehyd, in einem organischen Lösungsmittel, dann Erhitzen, indem gegebenenfalls das genannte gebildete Wasser des Reaktionsmediums eliminiert wird,
um zu erhalten:

ein *p*-(R-Oxy)-calixaren, ausgewählt aus einem *p*-(R-Oxy)-calix[9]aren, einem *p*-(R-Oxy)-calix[10]aren, einem *p*-(R-Oxy)-calix[11]aren, einem *p*-(R-Oxy)-calix[12]aren, einem *p*-(R-Oxy)-calix[13]aren, einem *p*-(R-Oxy)-calix[14]aren, einem *p*-(R-Oxy)-calix[15]aren, einem *p*-(R-Oxy)-calix[16]aren, einem *p*-(R-Oxy)-calix[17]aren, einem *p*-(R-Oxy)-calix[18]aren, einem *p*-(R-Oxy)-calix[19]aren, einem *p*-(R-Oxy)-calix[20]aren oder einer Mischung von mindestens zwei der genannten *p*-(R-Oxy)-calix[9-20]arene, wobei die genannten mindestens zwei *p*-(R-Oxy)-calixarene in der genannten Mischung in einer Menge von jeweils mindestens 5 Mol-% vorhanden sind,
in versalzter Form in dem End-Reaktionsmedium,

b. Neutralisieren der genannten versalzten Form des genannten *p*-(R-Oxy)-calixarens oder der genannten Mischung, um das genannte *p*-(R-Oxy)-calixaren oder die genannte Mischung in neutralisierter Form zu erhalten, und gegebenenfalls umfassend einen zusätzlichen Schritt des Reinigens des genannten *p*-(R-Oxy)-calixarens oder der genannten Mischung in neutralisierter Form, um das genannte *p*-(R-Oxy)-calixaren oder die genannte Mischung in gereinigter neutralisierter Form zu erhalten.

**8.** Verfahren nach Anspruch 7, wobei die genannte Reinigung durch Kristallisation in einer Mischung von Lösungsmitteln auf der Basis von DMSO erfolgt.

## Claims

**1.** Use of at least one base, selected from rubidium hydroxide or caesium hydroxide, with at least one phenol substituted in position 4 of the following formula (I):

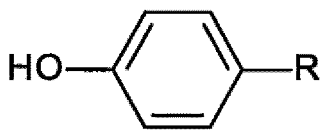

(I)

in which R is selected from:

- a benzyl group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyl group,
- a linear or branched PEG-1 to 10, of which the-OH end group is alkylated with a linear or branched $C_1$-$C_{20}$ alkyl,

a source of formaldehyde, and an organic solvent,
the mixture of said base, said phenol, said source of formaldehyde and said organic solvent constituting a reaction medium,
said reaction medium being heated to a temperature between 50 °C and 250 °C for a time of from 4 to 24h, and said base being at a total concentration comprised from more than 0.5 equivalent to 1.5 equivalent, with respect to said at least one phenol substituted in position 4 of formula (I),
for carrying out a reaction for preparing a *p*-(R-oxy)calixarene selected from a *p*-(R-oxy)calix[9]arene, a *p*-(R-oxy)calix[10] arene, a *p*-(R-oxy)calix[11]arene, a *p*-(R-oxy)calix[12]arene, a *p*-(R-oxy)calix[13]arene, a *p*-(R-oxy)calix[14]arene, a *p*-(R-oxy)calix[15]arene, a *p*-(R-oxy)calix[16]arene, a *p*-(R-oxy)calix[17]arene, a *p*-(R-oxy)calix[18]arene, a *p*-(R-oxy)calix[19]arene, a *p*-(R-oxy)calix[20]arene, or a mixture of at least two of said p-(R-oxy)calixarenes in which said at least two p-(R-oxy)calixarenes are present in said mixture each at a level of at least 5 mol. %.

2. The use according to claim 1, wherein said base is at a total concentration comprised from more than 0.5 equivalent to 1.2 equivalent, or is equal to 0.6 equivalent, with respect to said at least one phenol substituted in position 4 of formula (I).

3. A process for preparing

- a *p*-(R-oxy)calixarene selected from a *p*-(R-oxy)calix[9]arene, a *p*-(R-oxy)calix[10]arene, a *p*-(R-oxy)calix[11]arene, a *p*-(R-oxy)calix[12]arene, a *p*-(R-oxy)calix[13]arene, a *p*-(R-oxy)calix[14]arene, a *p*-(R-oxy)calix[15]arene, a *p*-(R-oxy)calix[16]arene, a *p*-(R-oxy)calix[17]arene, a *p*-(R-oxy)calix[18]arene, a *p*-(R-oxy)calix[19]arene, a *p*-(R-oxy)calix[20]arene, or
- a mixture of at least two of said *p*-(R-oxy)calixarenes in which said at least two *p*-(R-oxy)calixarenes are present in said mixture each at a level of at least 5 mol. %,

comprising a step of bringing at least one base, in particular selected from rubidium hydroxide or caesium hydroxide, into contact with at least one phenol substituted in position 4 of the following formula (I) :

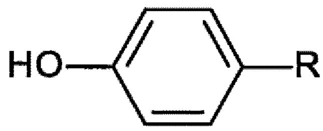

(I)

in which R is selected from:

- a benzyl group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyl group,
- a linear or branched PEG-1 to 10, of which the-OH end group is alkylated with a linear or branched $C_1$-$C_{20}$ alkyl,

a source of formaldehyde, and an organic solvent,
the mixture of said base, said phenol, said source of formaldehyde and said organic solvent constituting a reaction medium,
said reaction medium being heated to a temperature between 50 °C and 250 °C for a time of from 4 to 24h, and said base being at a total concentration comprised from more than 0.5 equivalent to 1.5 equivalent, with respect to said at least one phenol substituted in position 4 of formula (I).

4. The process according to claim 3, wherein said base is at a total concentration comprised from more than 0.5 equivalent to 1.2 equivalent, or is equal to 0.6 equivalent, with respect to said at least one phenol substituted in position 4 of formula (I).

5. The process according to either claim 3 or 4, wherein said phenol substituted in position 4 of formula (I) is selected from 4-benzyloxyphenol or 4-octyloxyphenol.

6. The process according to any one of claims 3 to 5, wherein the source of formaldehyde is paraformaldehyde, and the organic solvent is xylene.

7. The process according to any one of claims 3 to 6, comprising the following steps:

a. bringing caesium and/or rubidium hydroxide into contact with at least one phenol substituted in position 4 of formula (I), said base being at a total concentration comprised from more than 0.5 equivalent to 1.5 equivalent, with respect to said at least one phenol substituted in position 4 of formula (I), and a source of formaldehyde, in an organic solvent, then heating while optionally removing said water formed from the reaction medium
to obtain:

a *p*-(R-oxy)calixarene selected from a *p*-(R-oxy)calix[9]arene, a *p*-(R-oxy)calix[10]arene, a *p*-(R-oxy)calix[11]arene, a *p*-(R-oxy)calix[12]arene, a *p*-(R-oxy)calix[13]arene, a *p*-(R-oxy)calix[14]arene, a *p*-(R-oxy)calix[15]arene, a *p*-(R-oxy)calix[16]arene, a *p*-(R-oxy)calix[17]arene, a *p*-(R-oxy)calix[18]arene, a *p*-(R-oxy)calix[19]arene, a *p*-(R-oxy)calix[20]arene or a mixture of at least two of said *p*-(R-oxy)calix[9-20]arenes in which said at least two *p*-(R-oxy)calixarenes are present in said mixture each at a level of at least 5 mol. %, in salified form in the final reaction medium,

b. neutralising said salified form of said *p*-(R-oxy)calixarene or of said mixture in order to obtain said *p*-(R-oxy)calixarene or said mixture in the neutralised form,

and comprising optionally an additional step of purification of said *p*-(R-oxy)calixarene or of said mixture in its neutralised form, in order to obtain said *p*-(R-oxy)calixarene or said mixture in the purified neutralised form.

**8.** The process according to claim 7, in which said purification is achieved by crystallisation in a mixture of solvents based on DMSO.

**FIGURE 1**

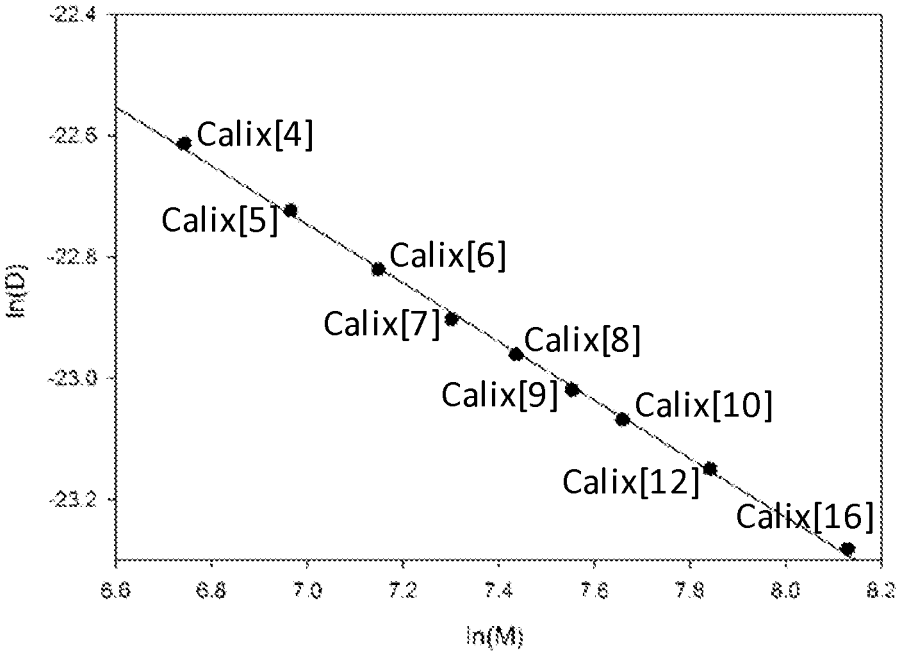


**FIGURE 2**

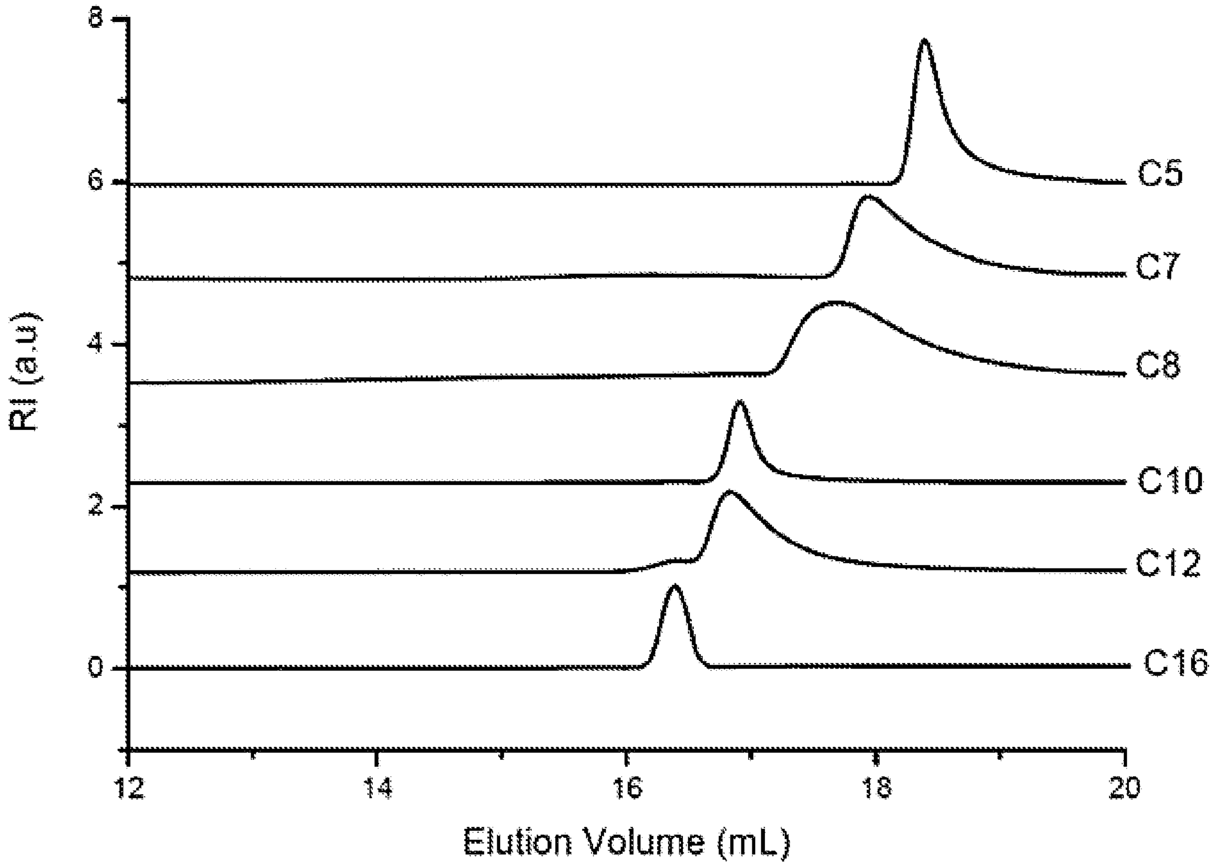

# FIGURE 3

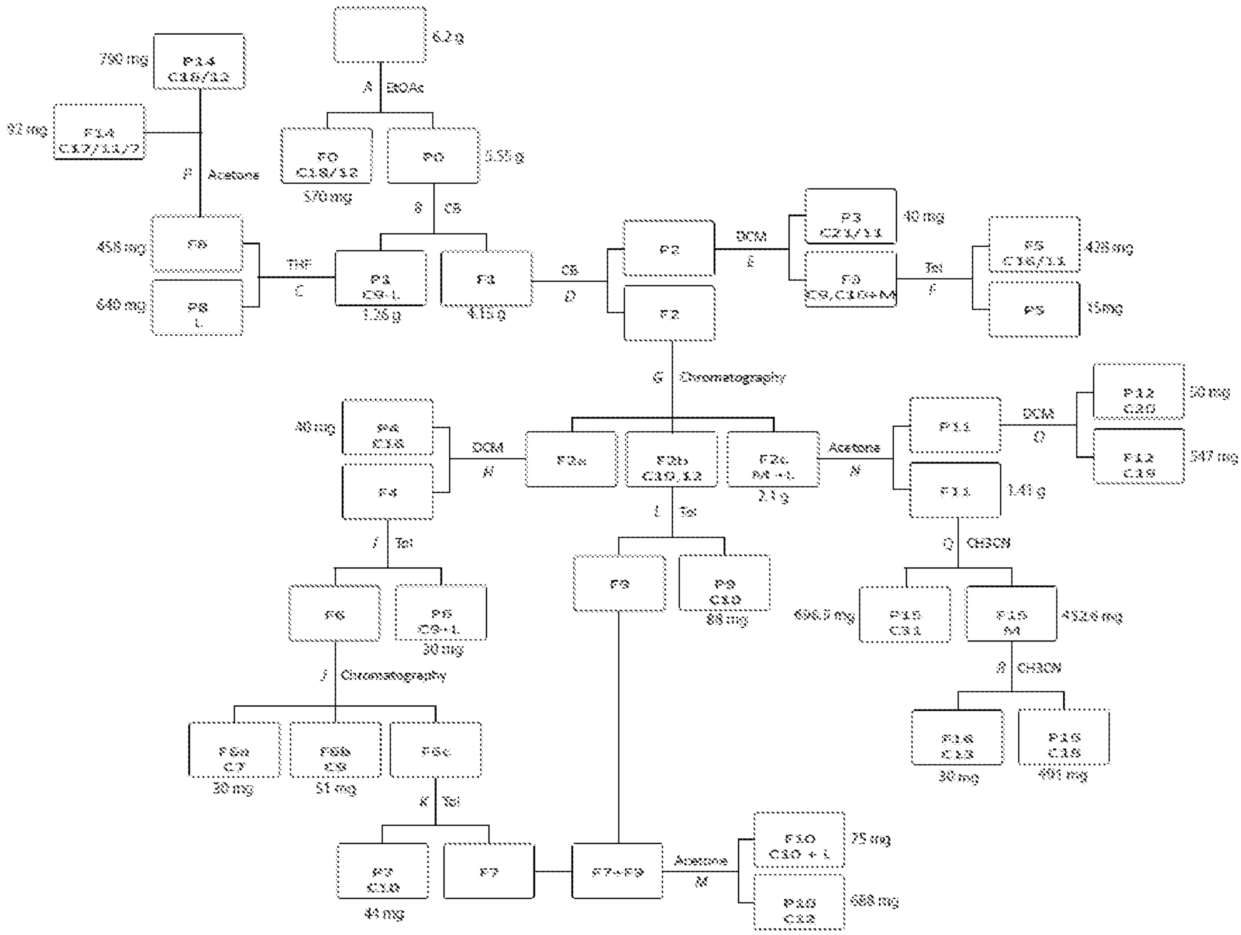
6.2 g
A EtOAc
5.55 g
570 mg
B CB
790 mg
Acetone
458 mg
640 mg
THF
C
1.26 g
CB
D
DCM
E
40 mg
Tol
F
15mg
G Chromatography
DCM
H
Acetone
N
2.1 g
1.41 g
DCM
O
50 mg
547 mg
I Tol
L Tol
Q CH3CN
R CH3CN
30 mg
88 mg
J Chromatography
30 mg
51 mg
K Tol
44 mg
Acetone
M
75 mg
30 mg

**FIGURE 4**

**Figure 4.1 : Fraction F0**

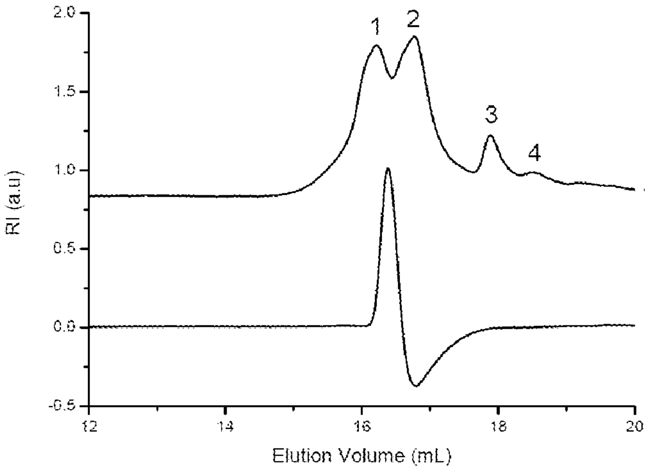


**Figure 4.2 : Fraction P3**

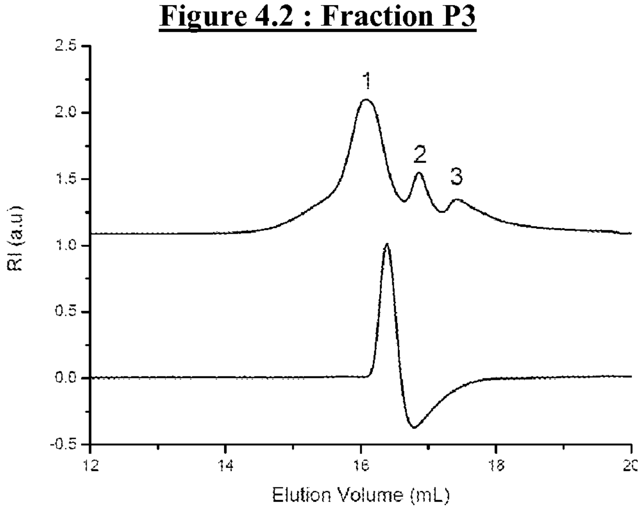


**Figure 4.3 : Fraction F5**

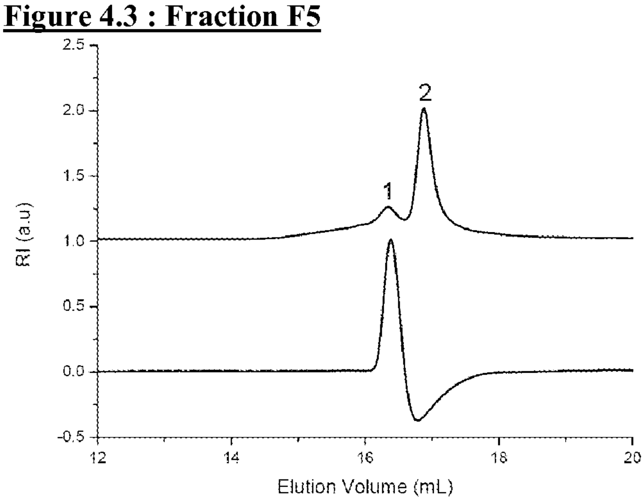

**Figure 4.4 : Fraction P12**

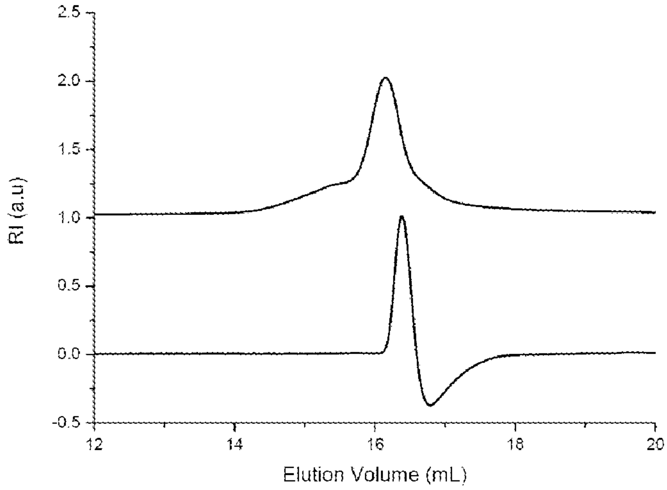


**Figure 4.5 : Fraction F12**

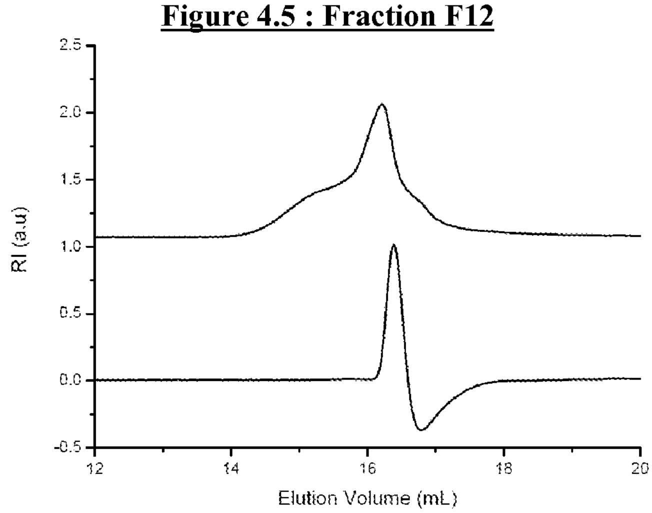


**Figure 4.6 : Fraction P14**

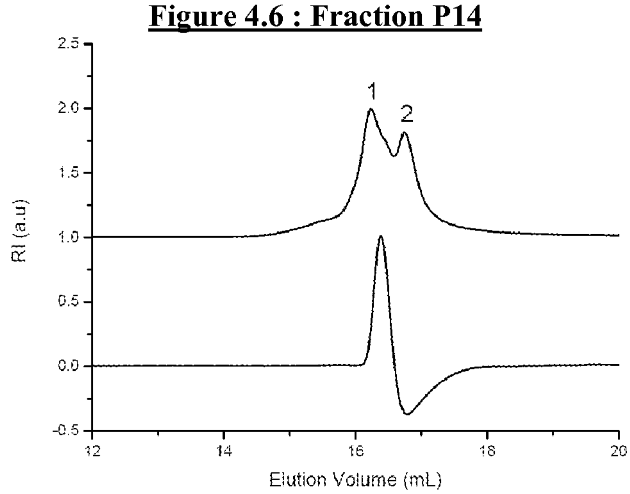

**Figure 4.7 : Fraction F14**

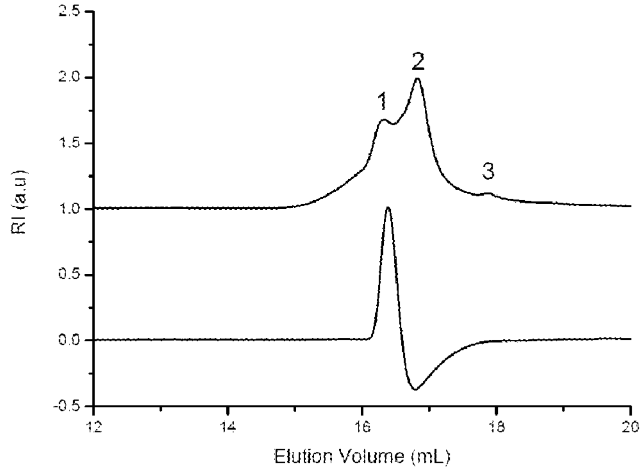


**Figure 4.8 : Fraction P15**

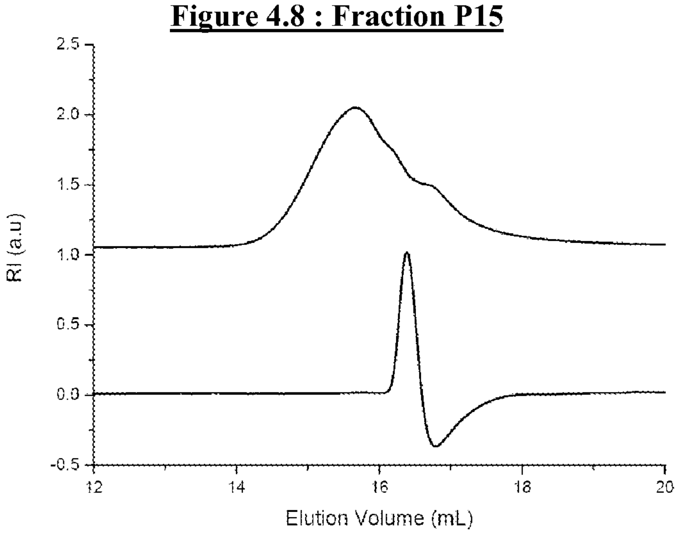


**Figure 4.9 : Fraction P16**

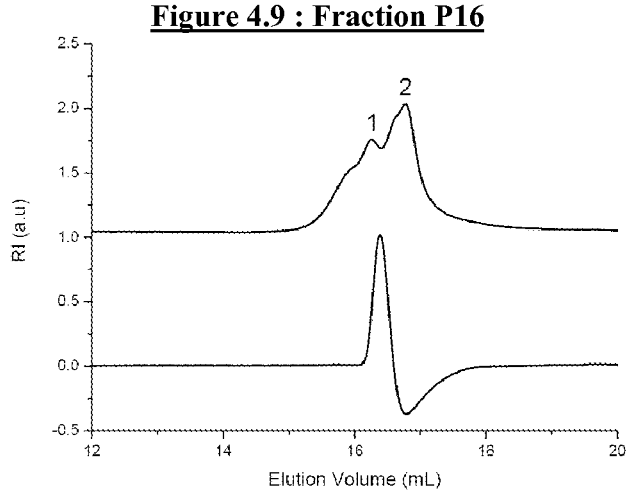

# FIGURE 5

**Figure 5.1 : Fraction F6b (Calix[9]arene)**

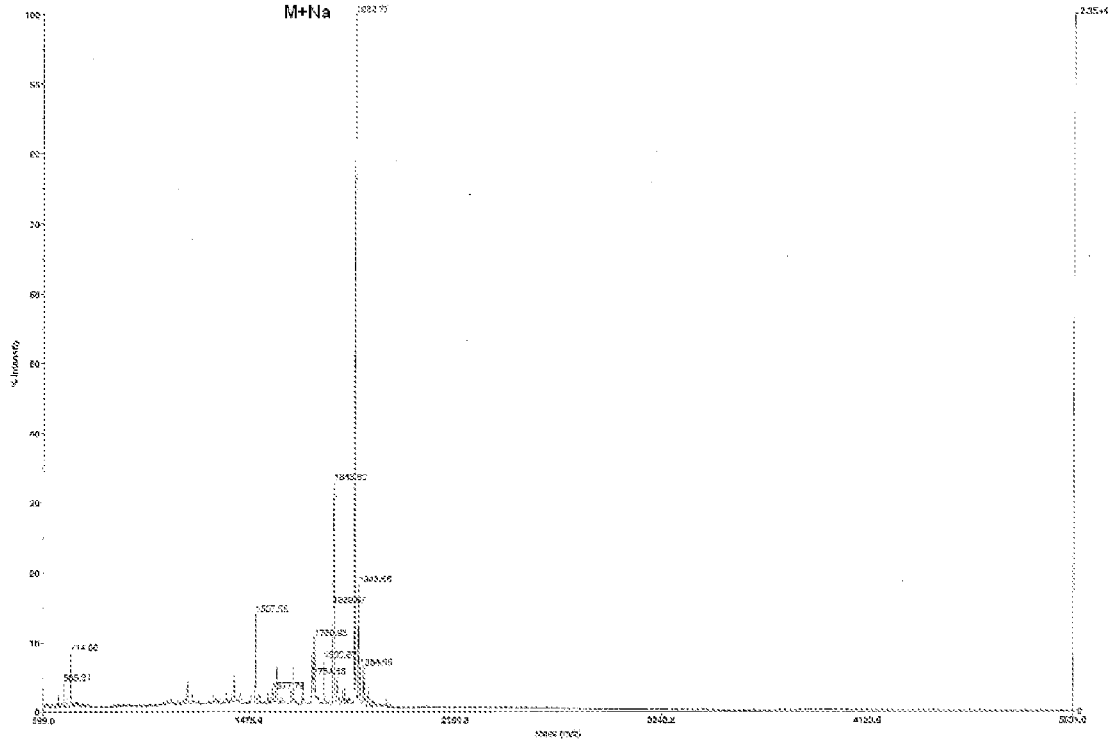


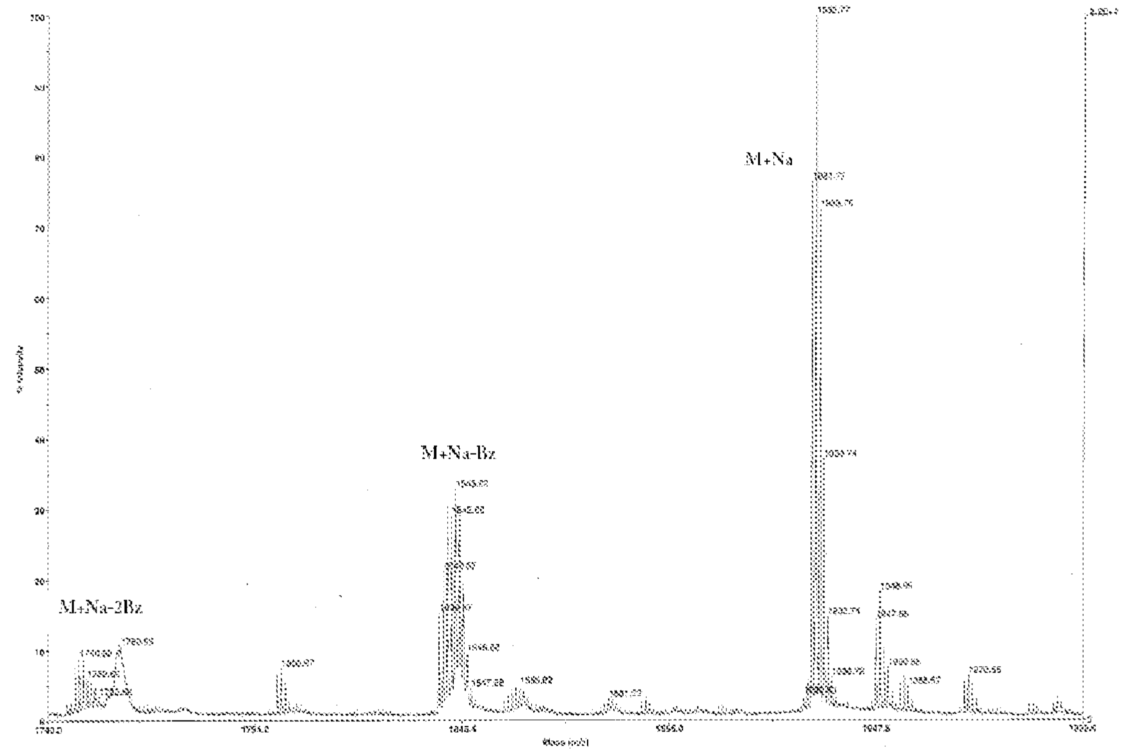

**Figure 5.2 : Fraction P7 (Calix[10]arene)**

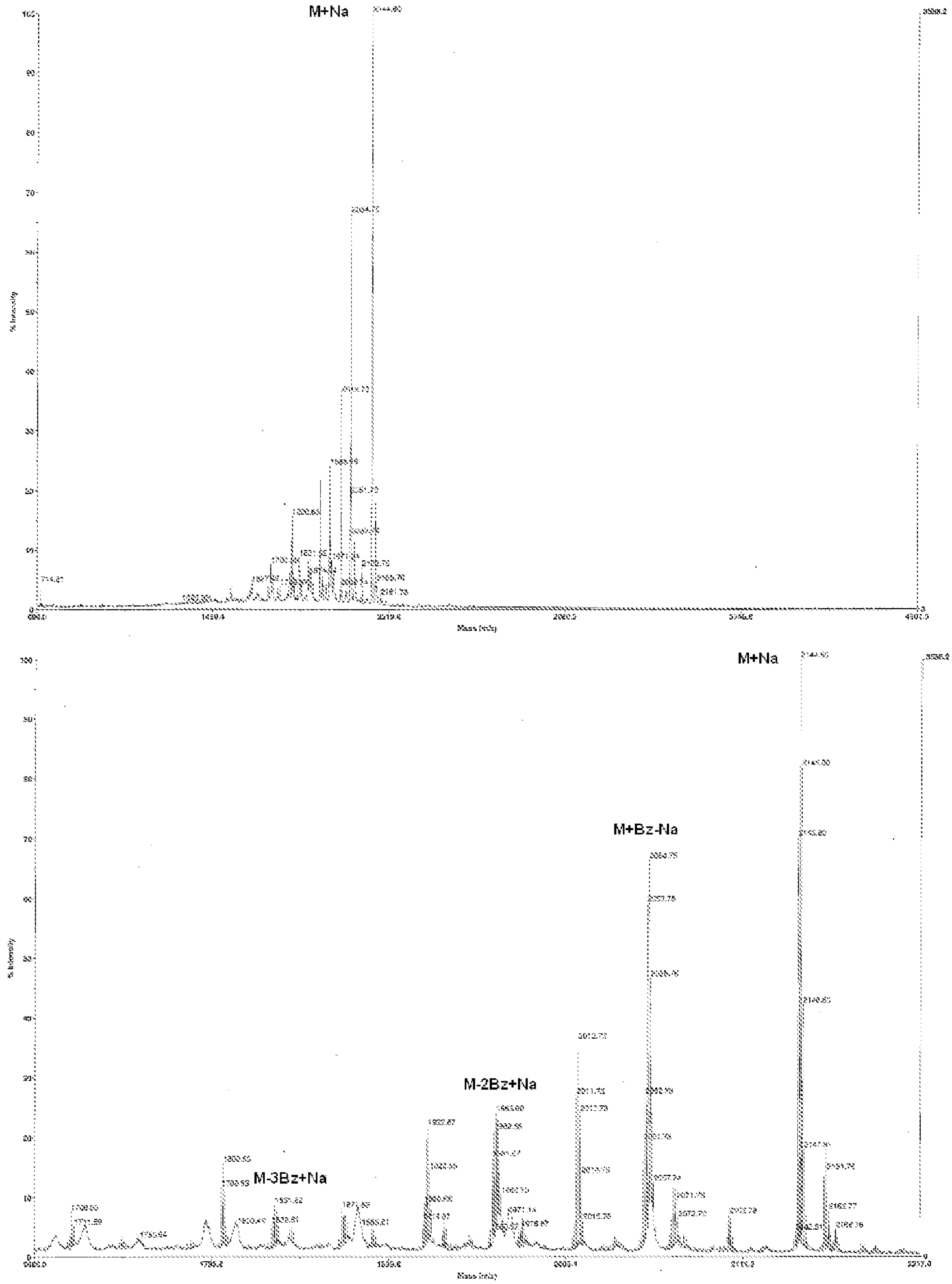

**Figure 5.3 : Fraction P10 (Calix[12]arene)**

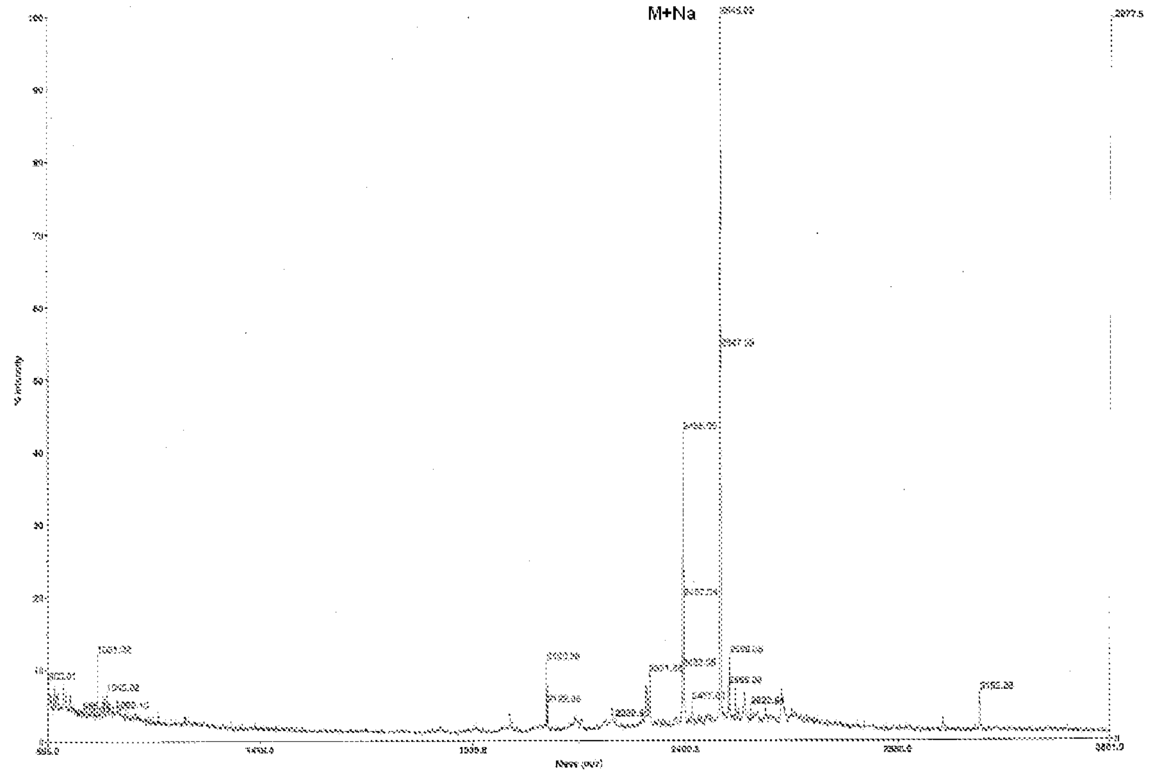


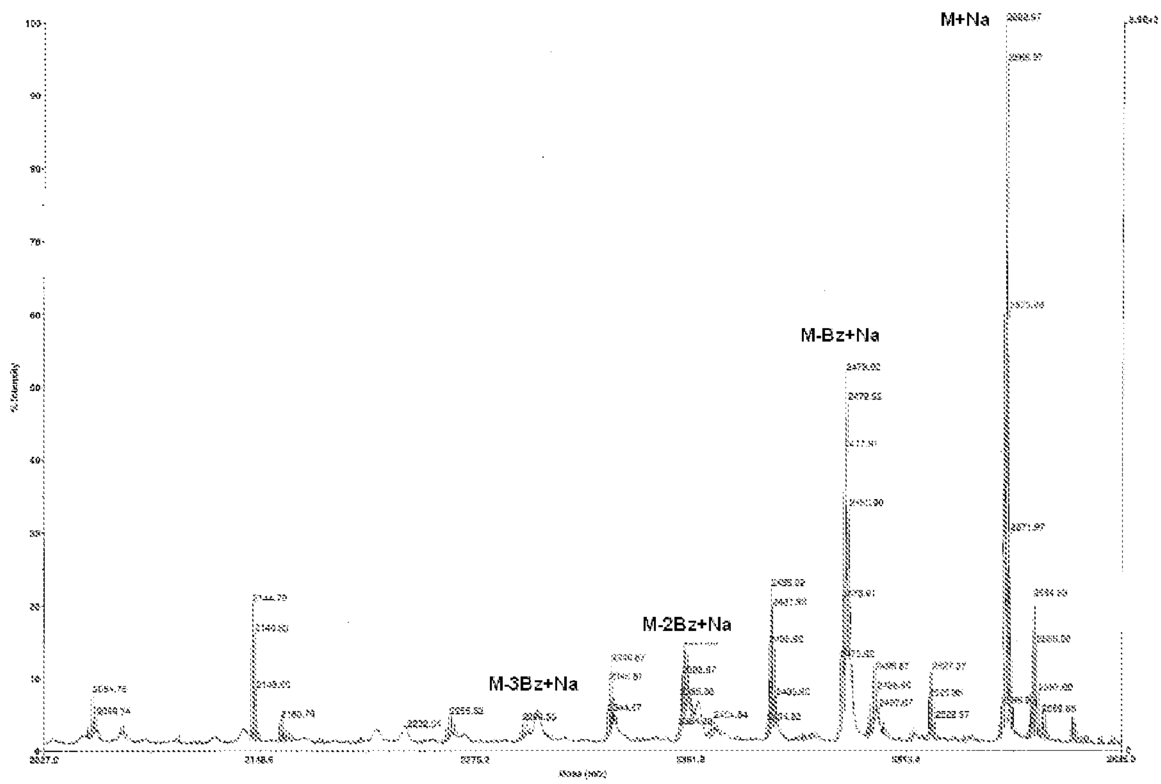

**Figure 5.4 : Fraction P4 (calix[16]arene)**

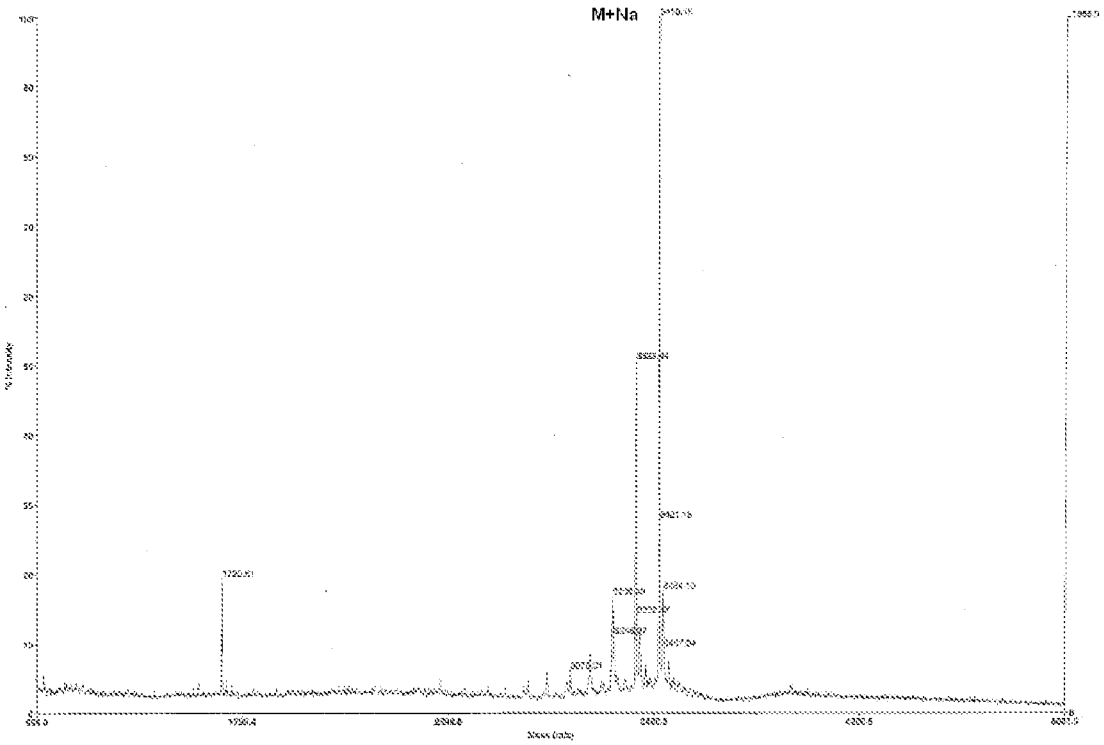


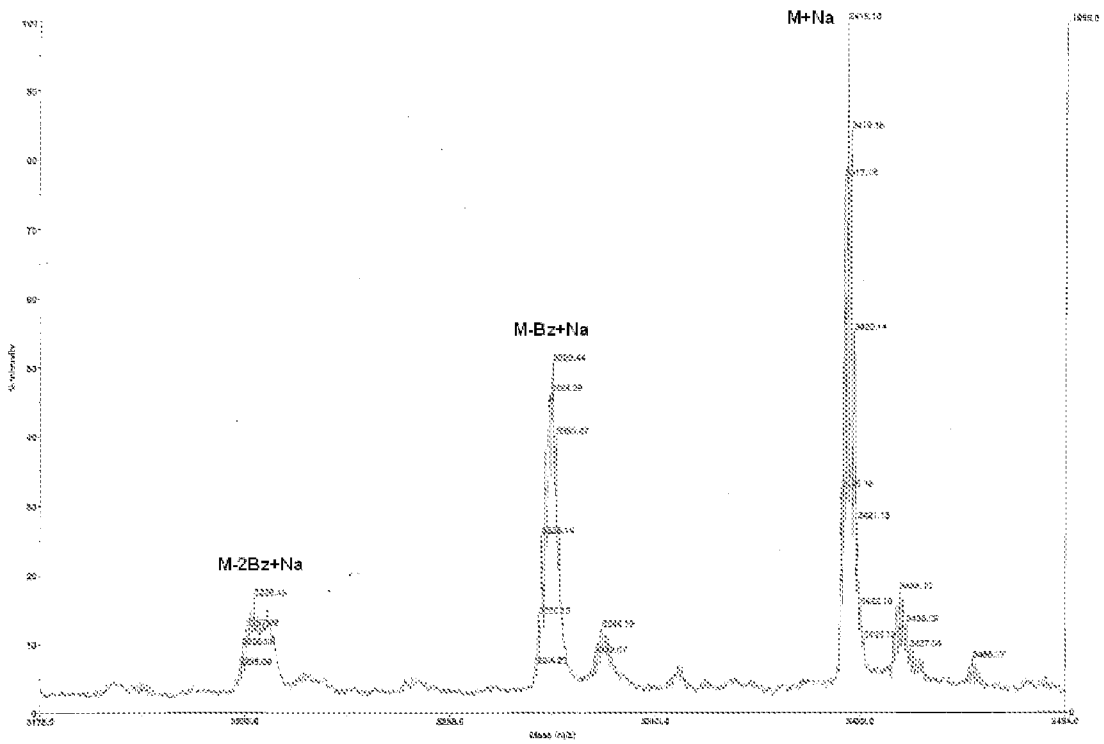

# FIGURE 6

## Figure 6.1 : Fraction F6b (Calix[9]arene)

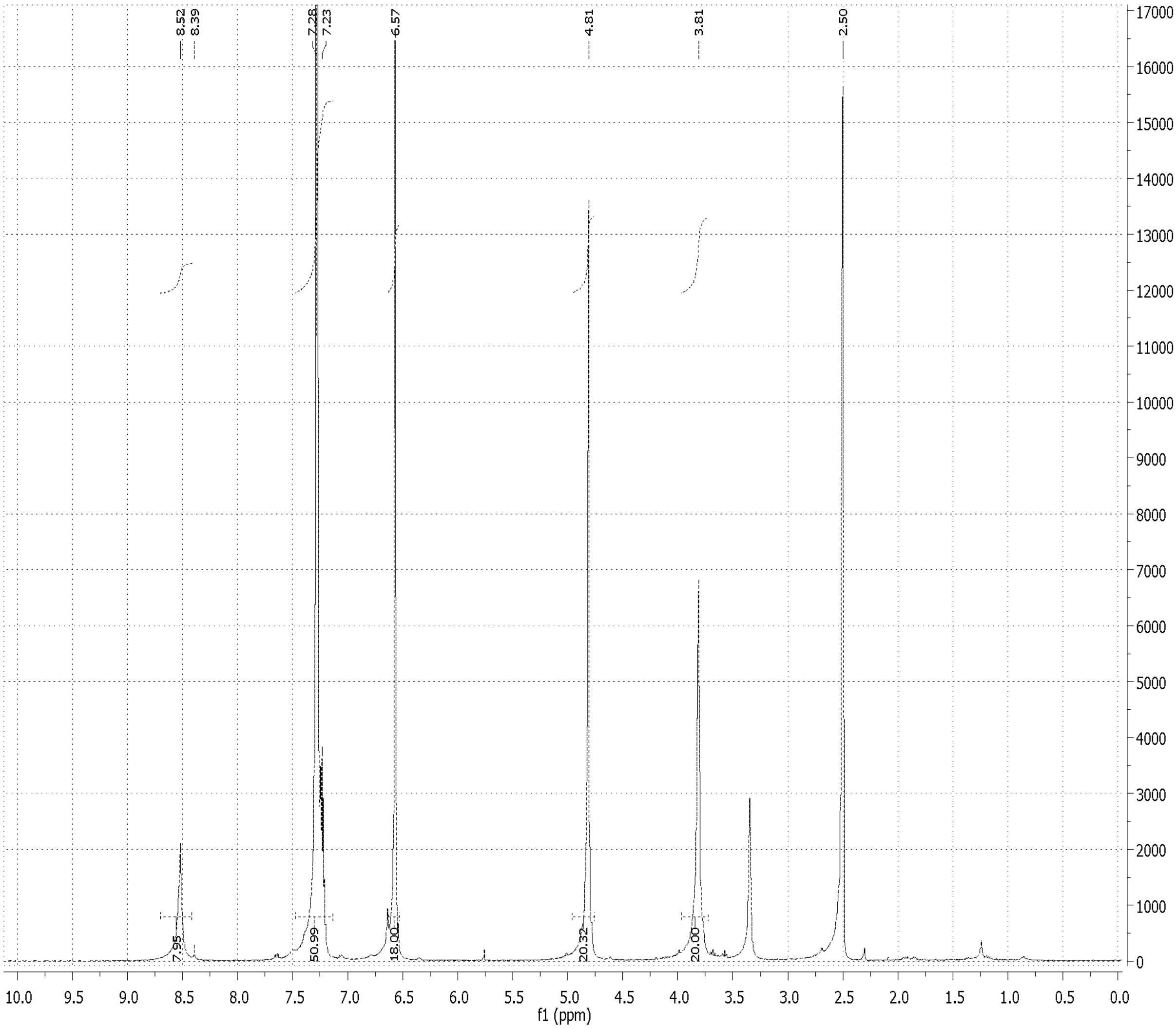

Figure 6.2 : Fraction P7 (Calix[10]arene)

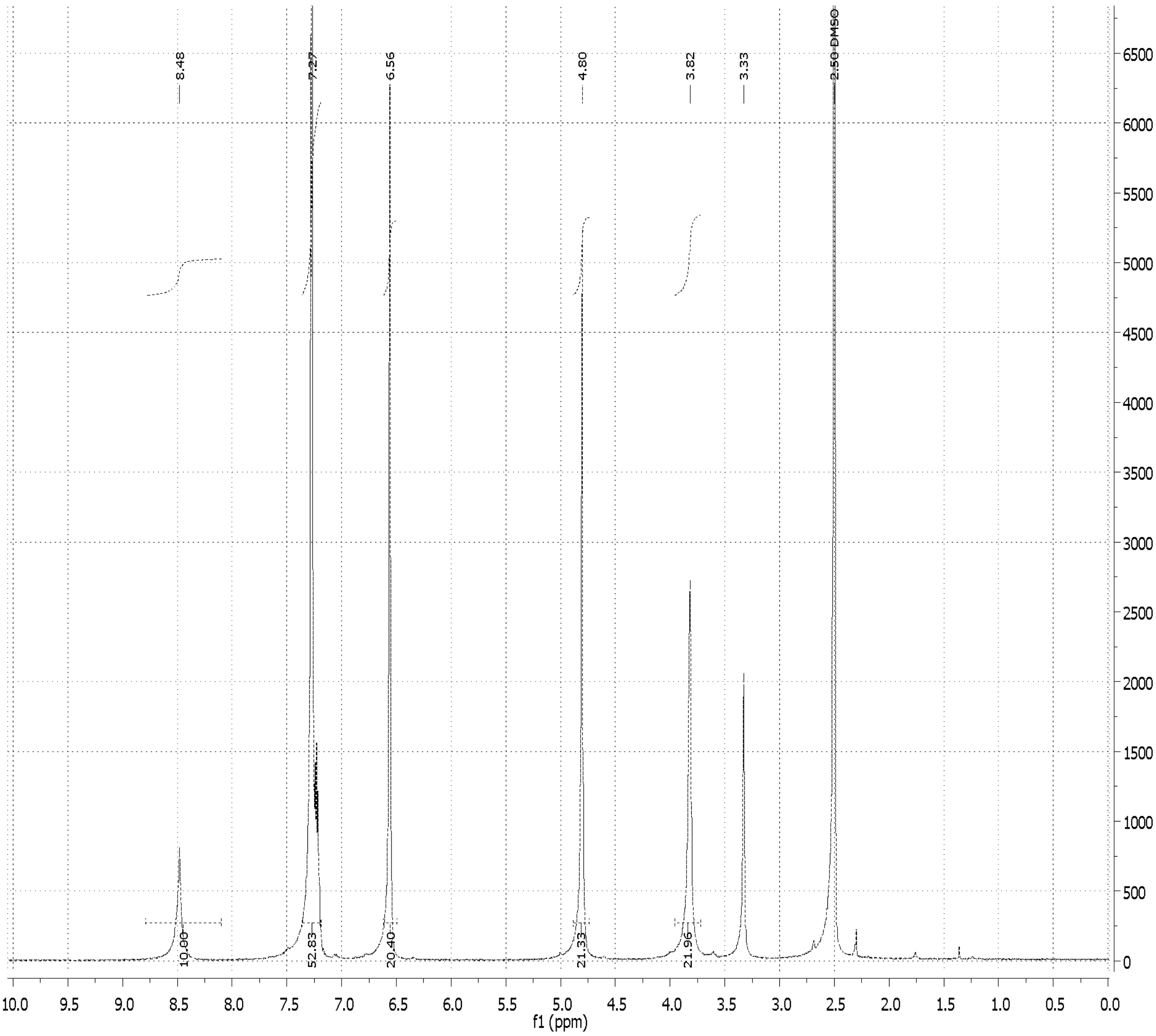

**Figure 6.3 : Fraction P10 (Calix[12]arene)**

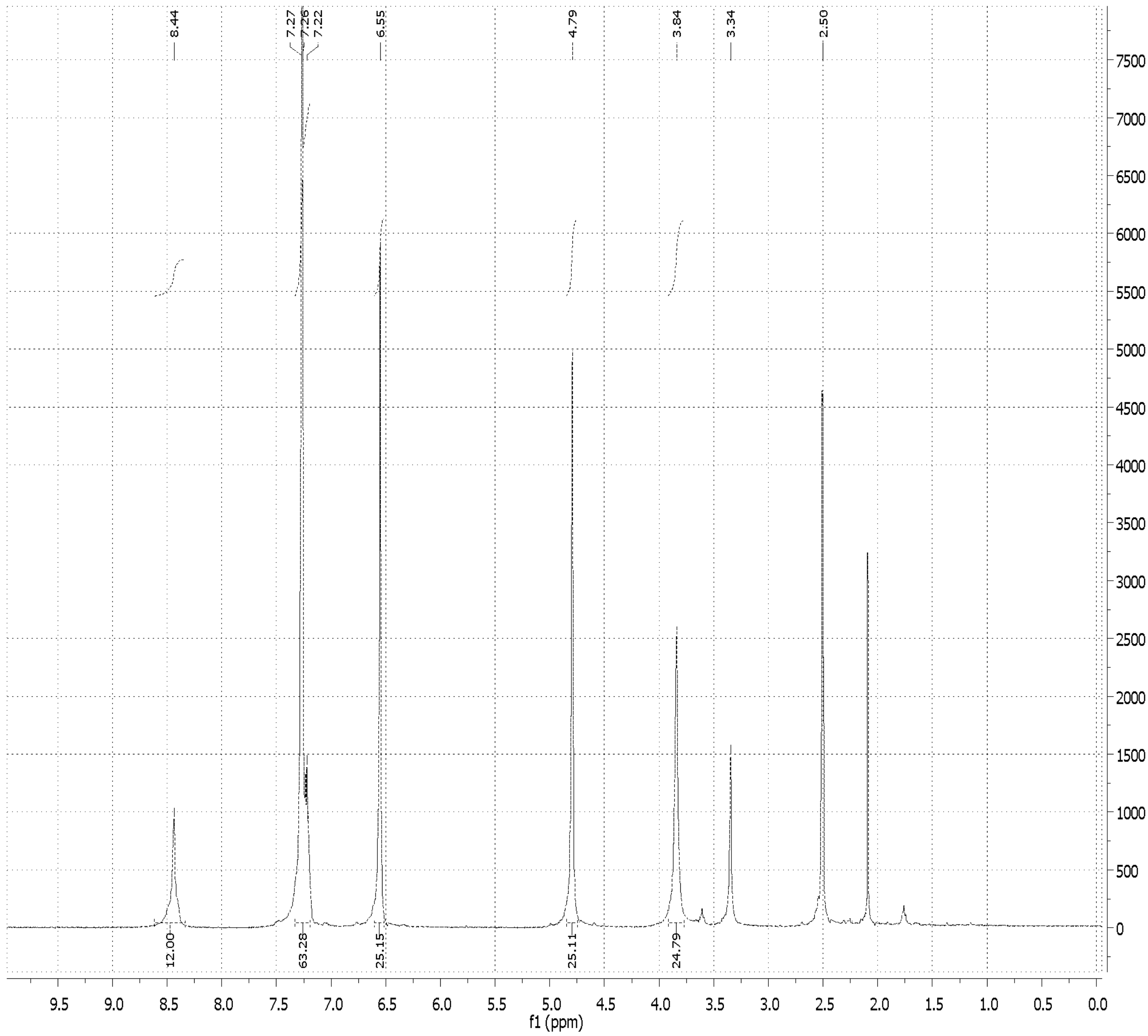

**Figure 6.4 : Fraction F16 (calix[13]arene)**

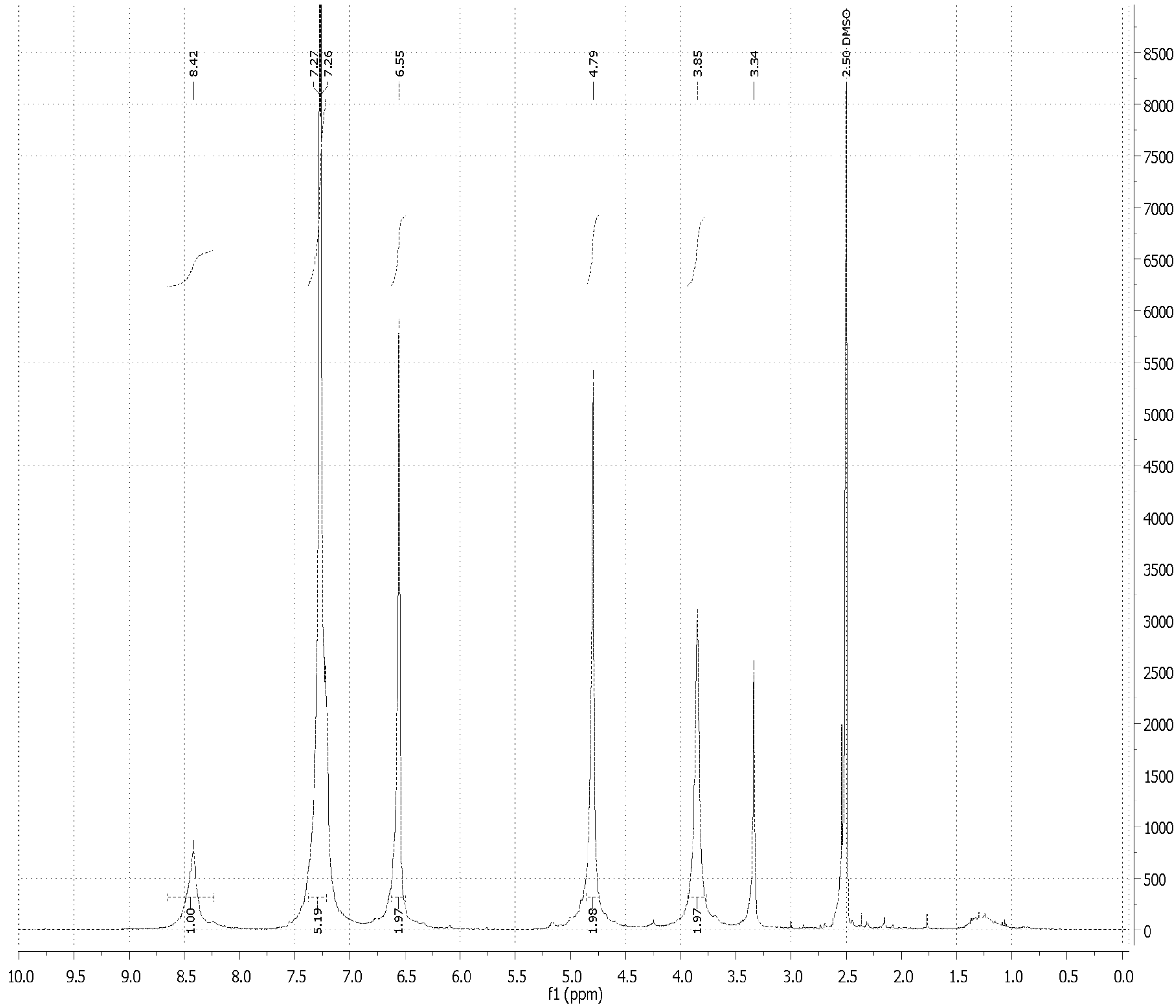

**Figure 6.5 : Fraction P4 (calix[16]arene)**

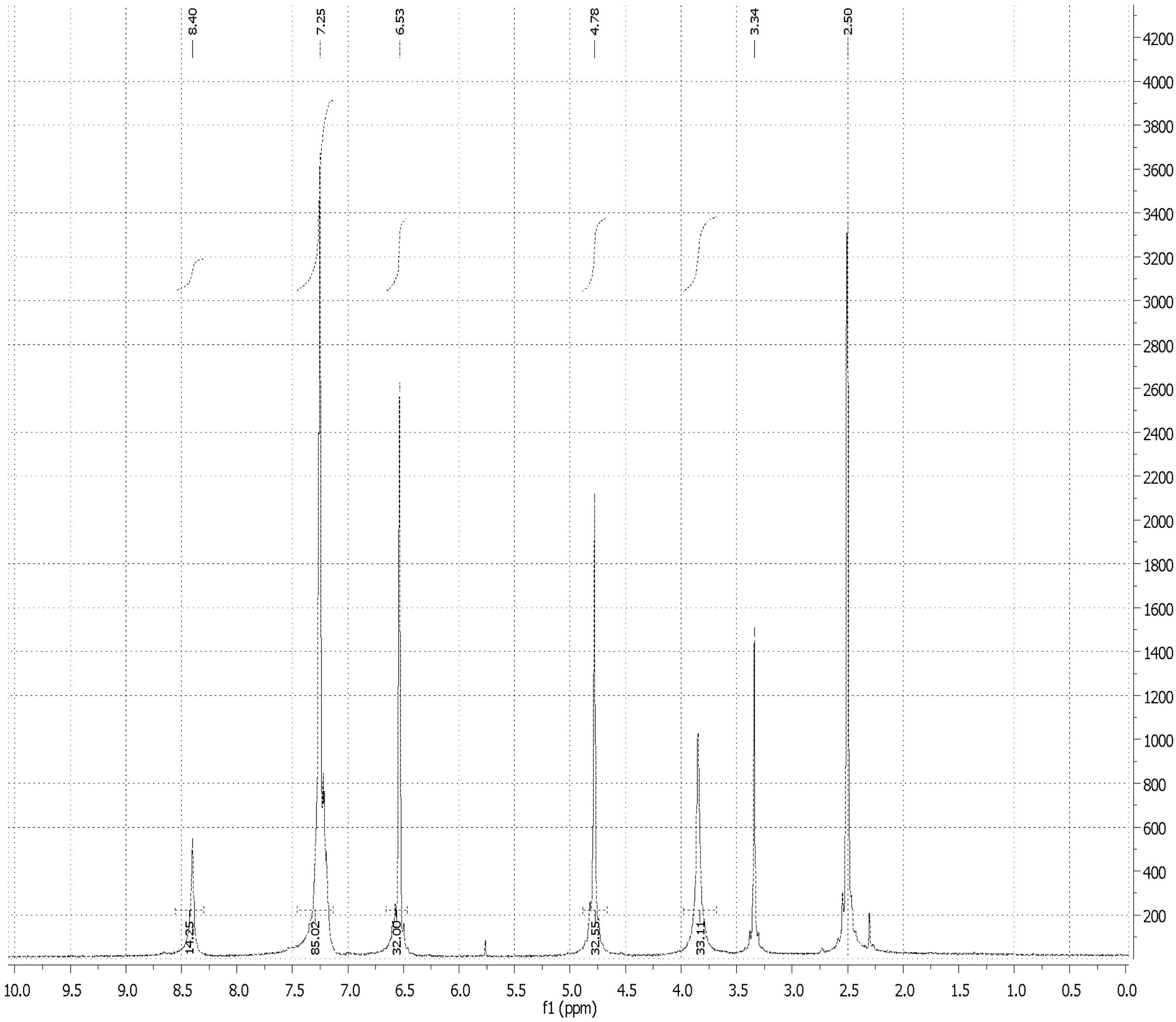

**FIGURE 7**

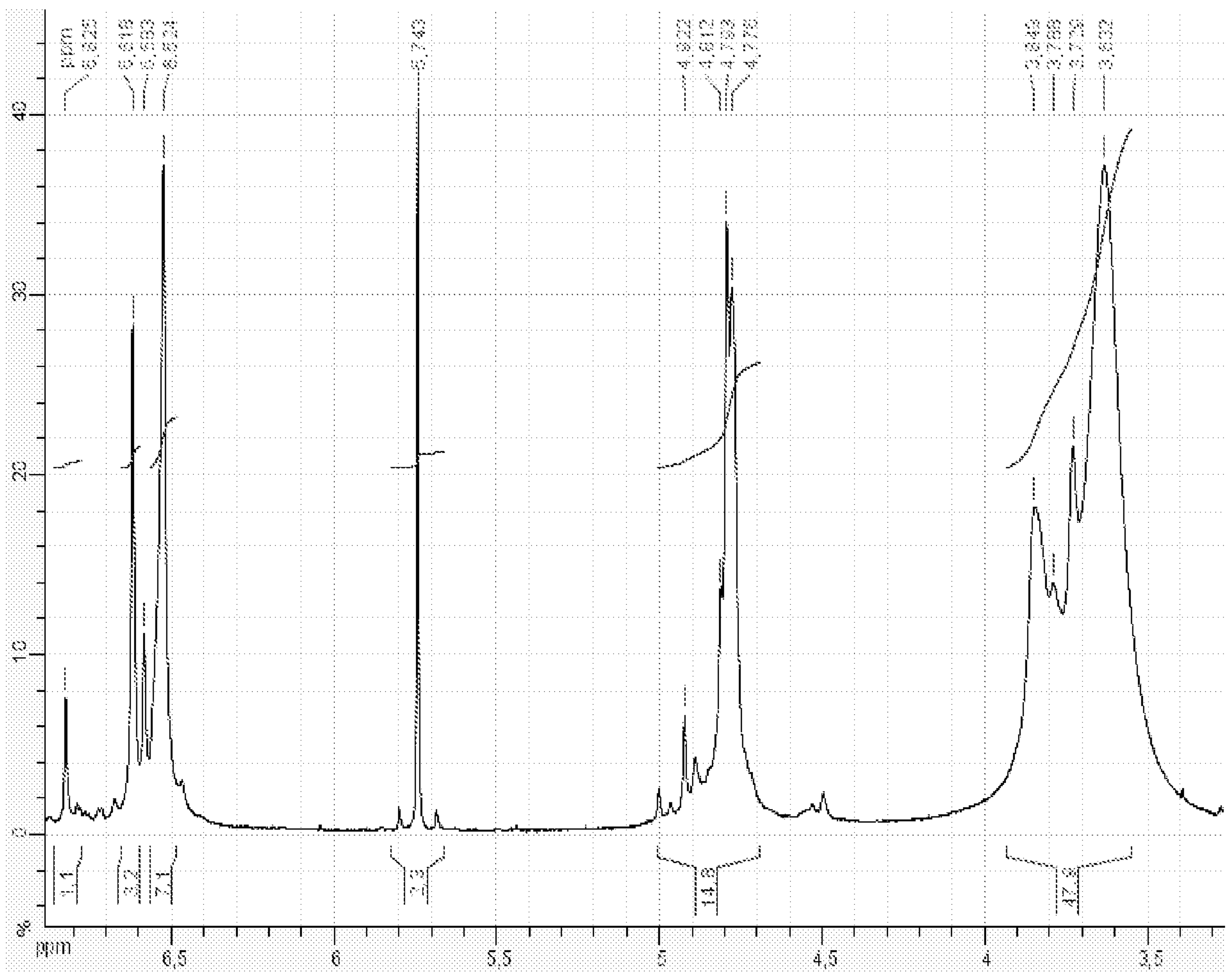

# FIGURE 8

## FIGURE 8.1

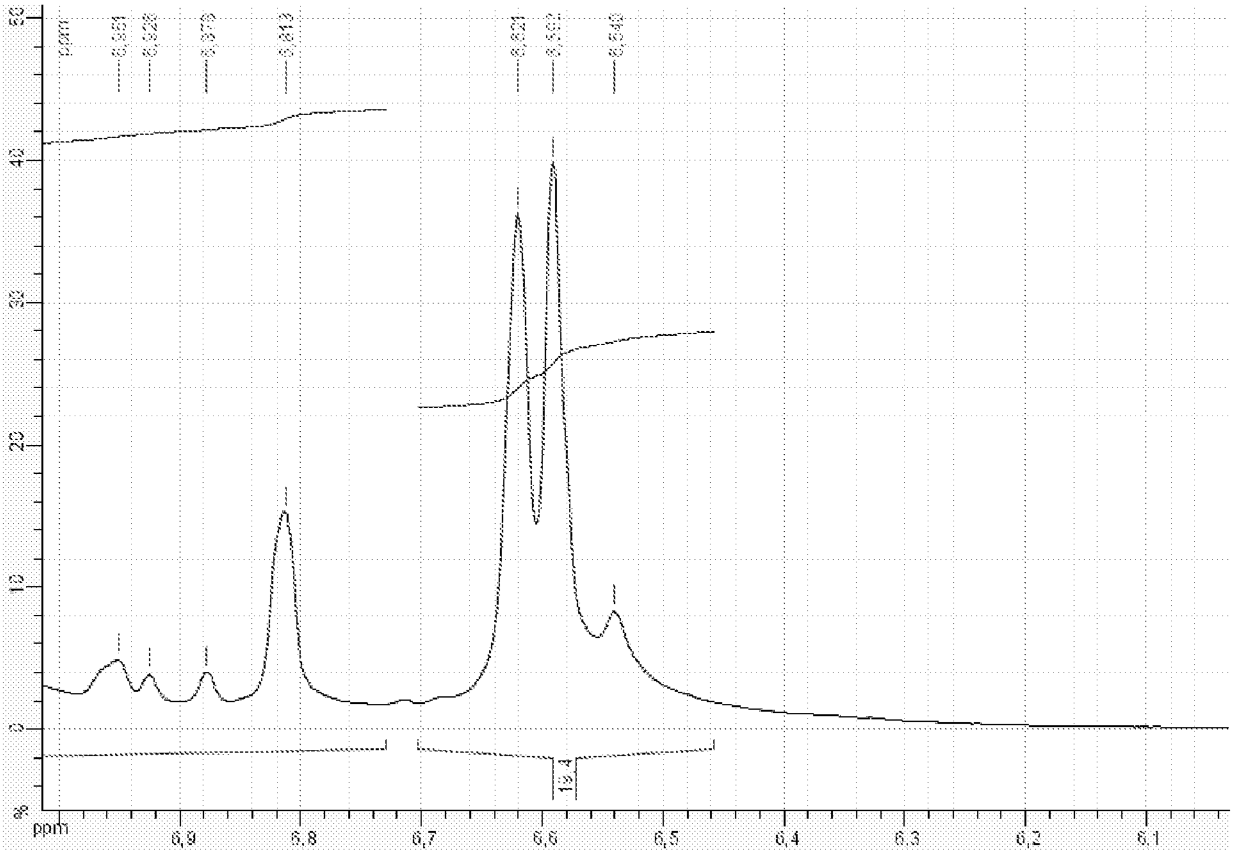

FIGURE 8.2

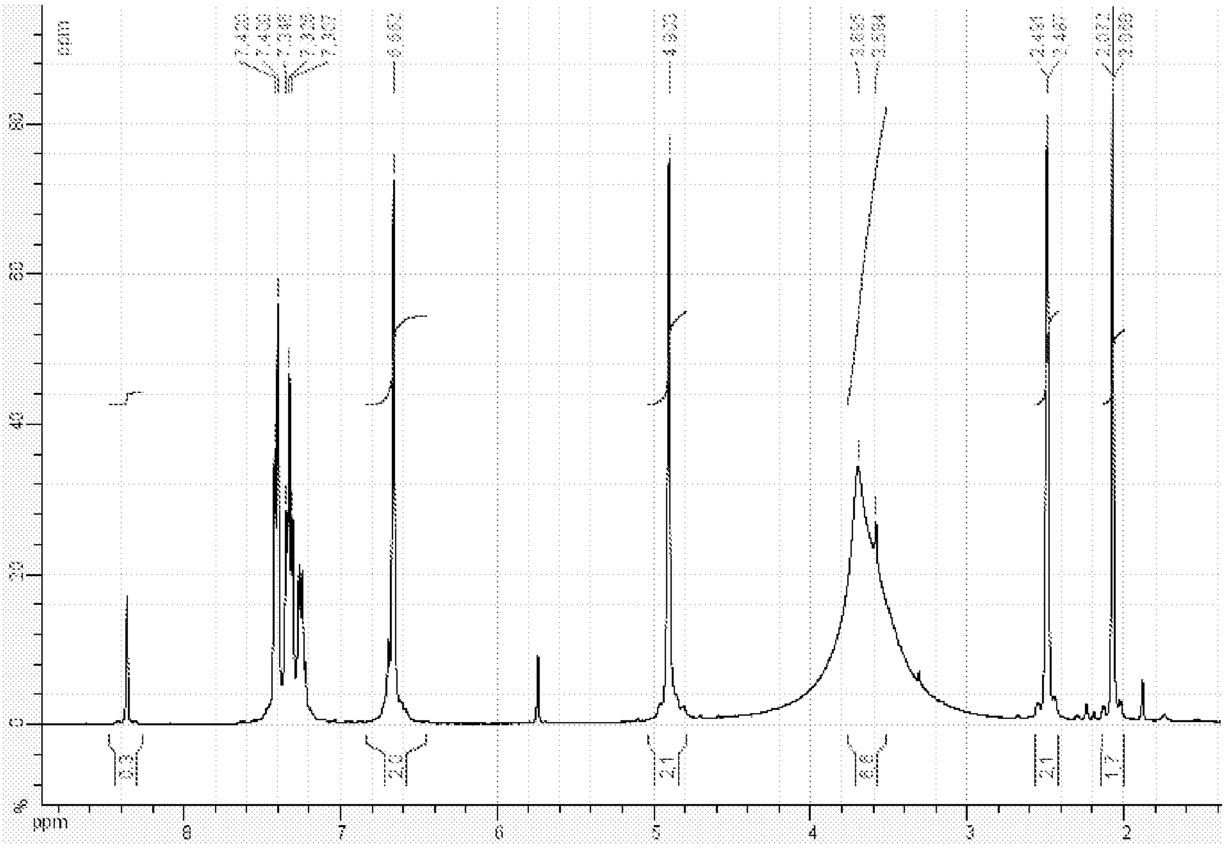

FIGURE 9

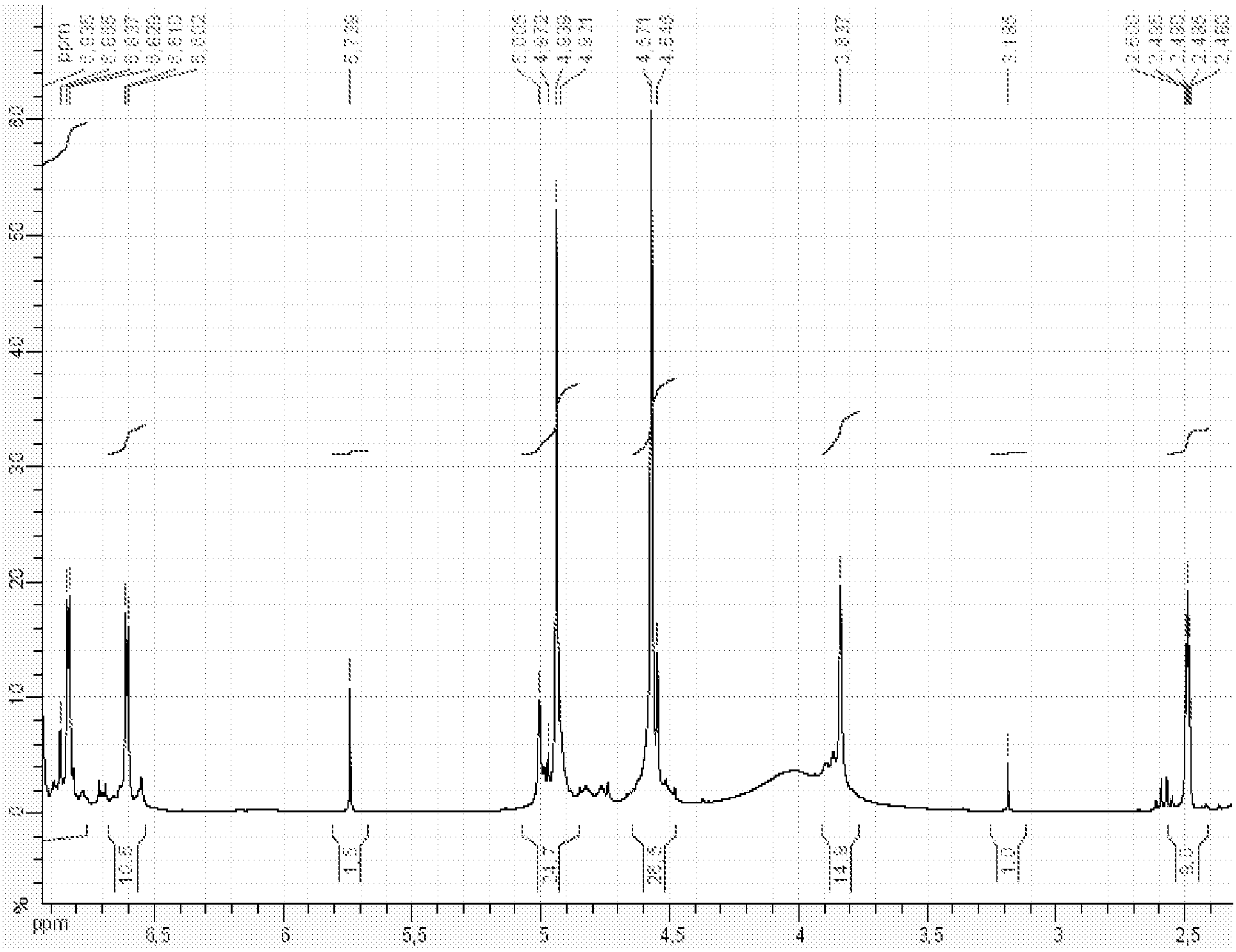

FIGURE 10

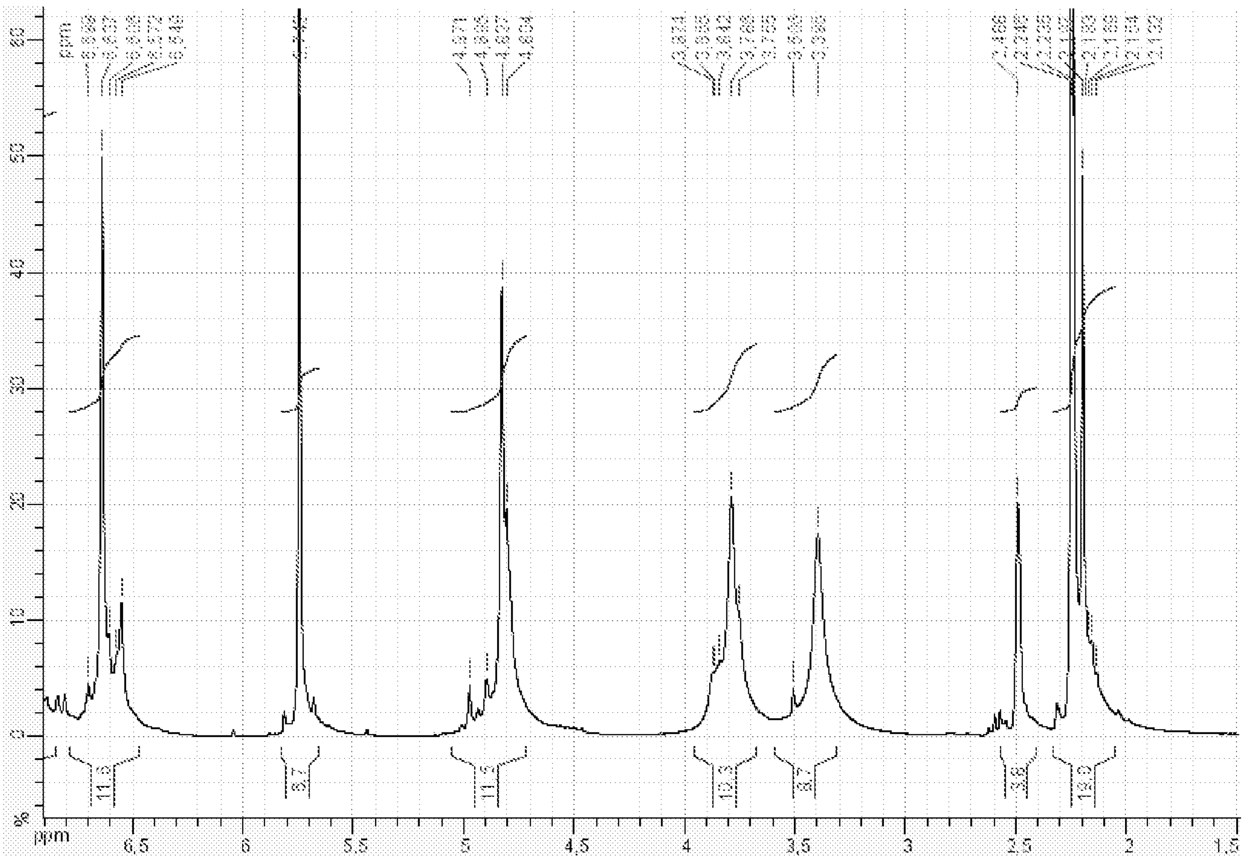

**FIGURE 11**

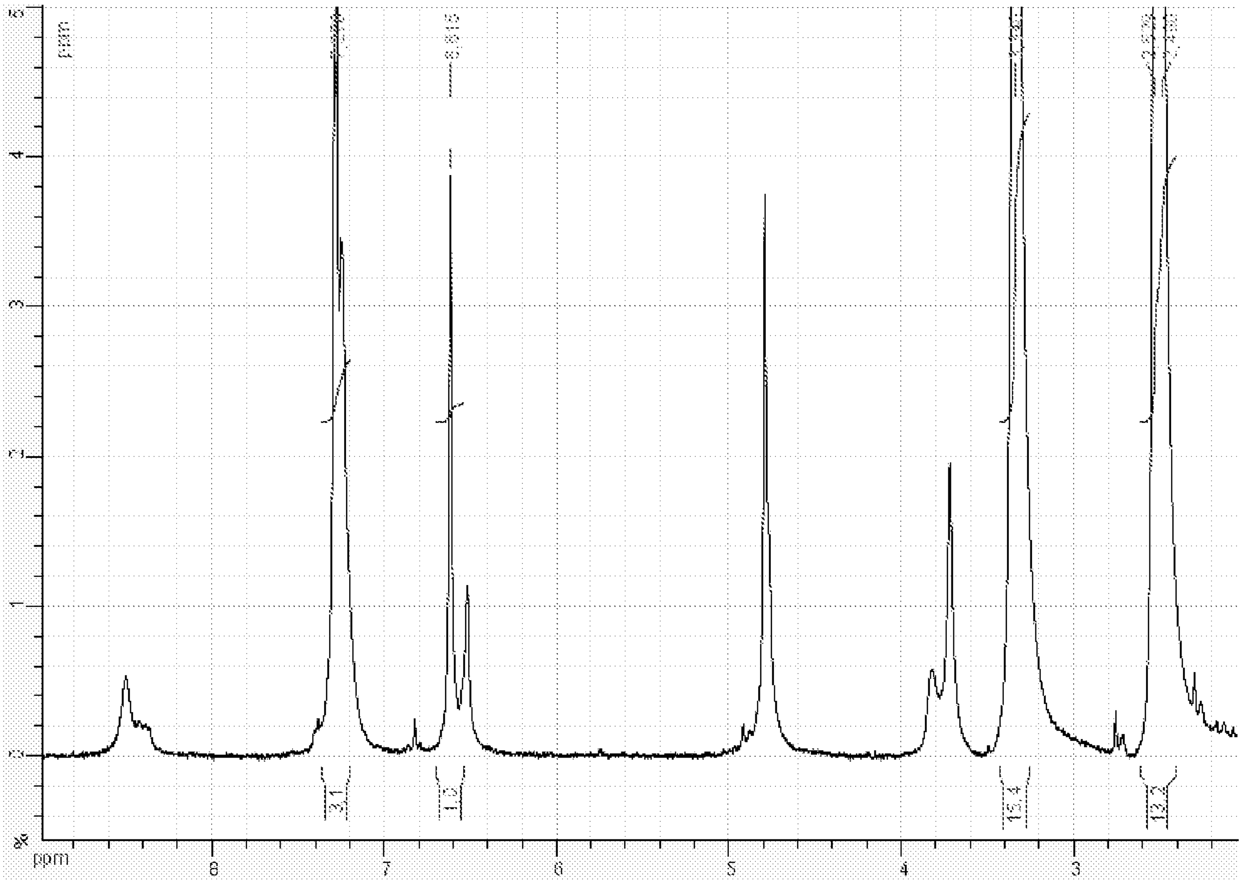

FIGURE 12

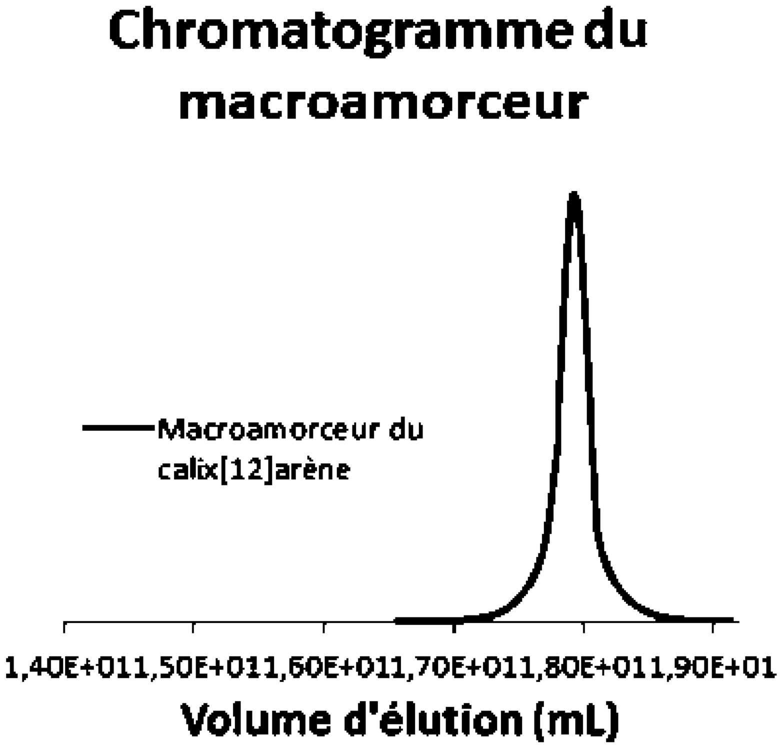


FIGURE 13

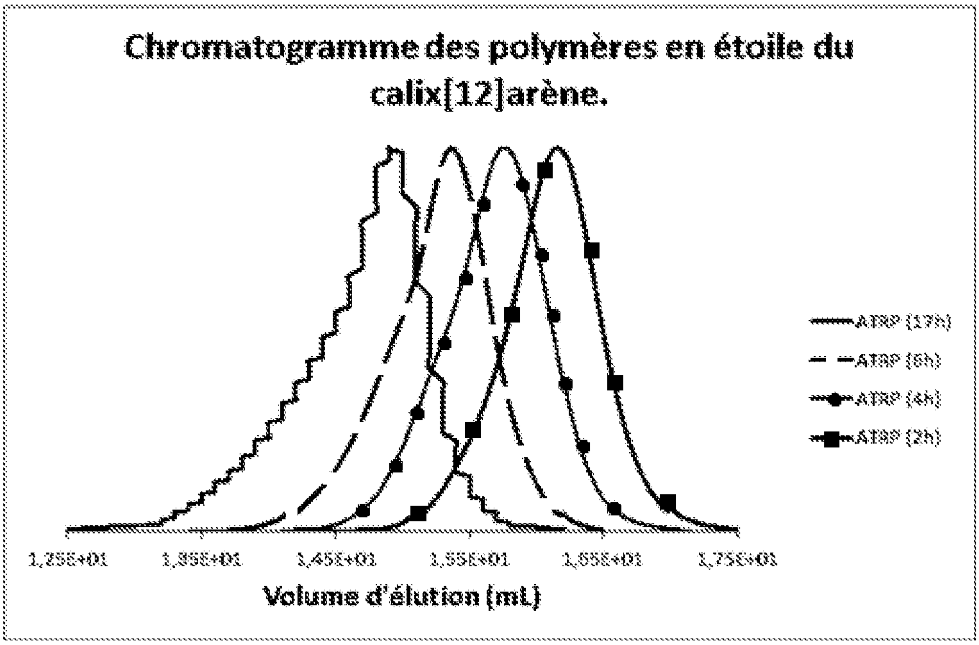

# FIGURE 14

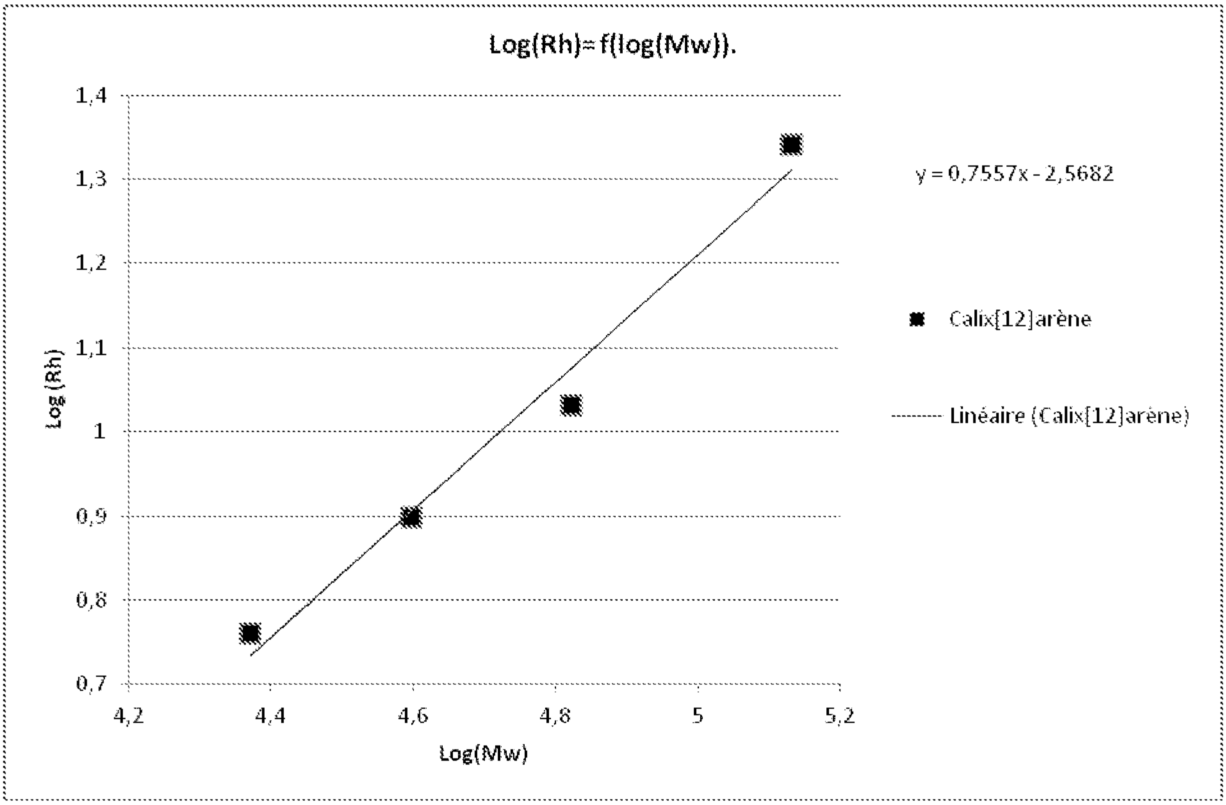
Log(Rh)= f(log(Mw)).
1,4
1,3
1,2
1,1
1
0,9
0,8
0,7
Log (Rh)
4,2
4,4
4,6
4,8
5
5,2
Log(Mw)
y = 0,7557x - 2,5682
Calix[12]arène
Linéaire (Calix[12]arène)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CA 2251070


### Littérature non-brevet citée dans la description


- **D. GUTSCHE.** Calixarenes: An Introduction. The Royal Society of Chemistry, 2008
- **Z. ASFARI ; V. BÖHMER ; J. HARROWFIELD ; J. VICENS.** Calixarenes 2001. Kluwer, 2001
- **T. PATRICK ; P. EGAN.** *J. Org. Chem.,* 1977, vol. 42, 382
- **T. PATRICK ; P. EGAN.** *J. Org. Chem.,* 1978, vol. 43, 4280
- **Z. ASFARI ; J. VICENS.** *Tetrahedron Lett.,* 1988, vol. 29, 2659
- **F. VOCANSON ; M. PERRIN ; R. LAMARTINE.** *J. Inclusion Phenom. Macrocyclic Chem.,* 2001, vol. 39, 127
- **JERRY L. ATWOOD et al.** *Org. Lett.,* 1999, vol. 1, 1523
- **B. DAHWAN et al.** *Macromolec. Chem.,* 1987, vol. 188, 921
- **C. D. GUTSCHE et al.** *Org. Synth.,* 1990, vol. 68, 234
- **C. D. GUTSCHE et al.** *Org. Synth.,* 1990, vol. 68, 238
- **A. CASNATI et al.** *J. Am. Chem. Soc,* 2001, vol. 123, 12182
- **V. HUC et al.** *Eur. J. Org. Chem,* 2010, 6186
- **V. HUC et al.** *Eur. J. Org. Chem.,* 2010, 6186
- **A. NINEGAWA et al.** *Macromol. Chem. Rapid. Chem.,* 1982, vol. 3, 65
- **Y. NAKAMOTO et al.** *Macromol. Chem. Rapid. Chem.,* 1982, vol. 3, 705
- **F. VOCANSON et al.** *New. J. Chem.,* 1995, vol. 19, 825
- **J. L. ATWOOD et al.** *Org. Lett.,* 1999, vol. 1, 1523
- **A CASNATI et al.** *J. Org. Chem.,* 1997, vol. 62, 6236
- **GUTSCHE et al.** *J. Am. Chem. Soc.,* 1999, vol. 121, 4136